# EUROPEAN PATENT SPECIFICATION

(11) **EP 3 399 986 B1**
(45) Date of publication and mention of the grant of the patent: **22.02.2023**
(21) Application number: 17704333.8
(22) Date of filing: 06.01.2017
(51) Int. Cl.: A61K 35/19, A61M 1/36, A61M 1/38, A61P 7/04, A61M 1/02

(54) **SYSTEMS AND METHODS FOR PREPARATION OF PLATELETS**
SYSTEME UND VERFAHREN ZUR HERSTELLUNG VON PLÄTTCHEN
SYSTÈMES ET MÉTHODES POUR LA PRÉPARATION DE PLAQUETTES

(30) Priority: 07.01.2016 US 201662276223 P; 21.03.2016 US 201662311373 P
(43) Date of publication of application: 14.11.2018
(73) Proprietor: Cerus Corporation, Concord, CA 94520 (US)
(72) Inventor: WEINER, Elan, Concord, CA 94520 (US); GREENMAN, William, Concord, CA 94520 (US)
(74) Representative: Mewburn Ellis LLP
(86) International application number: PCT/US2017/012633
(87) International publication number: WO 2017/120545

(56) References cited:
- WO-A1-98/30327
- WO-A1-99/34914
- WO-A1-2012/005934
- WO-A1-2012/061317
- WO-A1-2014/051537
- WO-A1-2016/014854
- WO-A1-2016/210374
- WO-A2-2006/118614
- US-A1- 2002 192 632
- US-A1- 2005 256 443
- US-A1- 2006 093 999
- US-A1- 2009 217 202
- US-A1- 2015 056 602
- US-B1- 6 544 727
- GRACINDA DE SOUSA ET AL: "Highlights of PBTI Coimbra Conference on PRT of Plasma & Current Opinions on Pathogen Reduction Treatment of Blood Components", TRANSFUSION AND APHERESIS SCIENCE, vol. 52, no. 2, April 2015 (2015-04), pages 228-232, XP055382999, GB ISSN: 1473-0502, DOI: 10.1016/j.transci.2015.02.010

## Description

### TECHNICAL FIELD

The methods described herein generally relate to the preparation of platelets. More particularly the present disclosure relates to improved methods for preparing apheresis derived platelet units suitable for infusion and compositions comprising such platelet units.

### BACKGROUND

Blood component collection and processing serves a critical role in healthcare worldwide, and millions of units of donated blood components are collected by blood banks each year. While some units of whole blood are collected from donors and used directly for transfusion, most donations are generally separated into the blood components red blood cells, platelets and plasma for more specific therapeutic use. Separation may be either following collection of whole blood donations or at the point of collection if using a suitable separation device system, such as an apheresis collection device. Individual blood components are then used in treating different medical needs and conditions based on therapeutic need.

Platelets play a key role in hemostasis, clot stability and retraction, as well as in vascular repair and anti-microbial host defense. A variety of methods are used to collect and store platelet blood products for clinical use. Collection of platelets from donated whole blood donation is generally in the form of platelet concentrates (PC), obtained using processing methods such as a buffy coat or platelet rich plasma method, and such PC may be pooled to generate a platelet unit of sufficient therapeutic dosage for transfusion. In general, PC from four to six individual donors of compatible blood types must be combined to produce a single platelet unit of sufficient therapeutic dosage for transfusion.

Apheresis collection provides a method to obtain platelet units of sufficient therapeutic dosage from a single donor, without the need for pooling and any related risk from the infusion recipient's exposure to platelets from multiple donors. Apheresis collection of platelets utilizes an automated device that separates platelets from the donor blood and returns remaining blood components (*e.g.,* red blood cells, plasma) to the donor during the donation process. Apheresis platelet donations are generally based on certain donor parameters, such as gender, physical size (*e.g.,* weight), hemoglobin level, platelet count on the day of donation and donation frequency, in part to ensure only a safe amount is collected. From these parameters, apheresis platelet donations generally are collected from an individual donor as a volume to yield one, two or three platelet units each containing a specified minimum number (*e.g.,* at least a minimum number) of platelets per unit to meet the therapeutic dose requirement, with such per unit or therapeutic dose criteria generally determined by governmental, regulatory, institution or accrediting organization (*e.g.,* industry) standards. Blood centers may further determine their own apheresis platelet collection specifications for singles, doubles and triples collections (*e.g.,* split points), including for example, the amount (*e.g.,* number) of excess platelets over that required to meet governmental, regulatory, institution or accrediting organization standards. Generally platelet collections to achieve the specified threshold for one, two or three platelet units typically factor in a number of variables that result in a distribution of platelet yields (*e.g.,* bell curve distribution), but do not need to be collected in excess from a donor in apheresis procedures, other than to account for anticipated donor and/or process variability, collection time limitations (*e.g.,* based on donor, based on blood center operations) splitting and testing (*e.g.,* Bac-T testing), as excess platelets would be retained in the one, two or three units, but without providing additional value from a platelet unit production standpoint. Thus, even if a donor could safely donate an amount of platelets by apheresis corresponding to, for example, 1.6 platelet units or 2.8 platelet units, there is generally no benefit or incentive for a blood center to collect such an amount, as the platelet units ultimately produced and provided to a doctor or hospital would be simply 1 platelet unit or 2 platelet units, respectively.

There remains a need for improved systems and methods that provide greater efficiency for collection and preparation of apheresis platelet units, including pathogen-inactivated platelet units, in order to more fully capture the overall potential for production of platelet units, such as in blood centers using apheresis collection systems for platelets.

### SUMMARY

The methods described herein are useful for improved collection and preparation of apheresis-derived platelet units suitable for infusion (*e.g.,* into a subject), and compositions comprising such platelet units, such as therapeutic dosage units of platelets.

In one aspect, the present disclosure provides a method of preparing a plurality of platelet units each comprising a therapeutic dose of platelets suitable for infusion into a human subject), comprising: a) collecting a first platelet donation by apheresis from a first donor, b) collecting a second platelet donation by apheresis from a second donor, c) combining the first platelet donation and the second platelet donation to yield a pooled platelet preparation, and d) separating the pooled platelet preparation into a plurality of platelet units each in an individual container, wherein the plurality of platelet units comprises a greater number of platelet units than the total number of platelet units that can be prepared from the first platelet donation and second platelet donation without combining the first and second platelet donations; wherein each platelet unit comprises a therapeutic dose of platelets.

In some embodiments, the first platelet donation comprises less than the number (*e.g.,* less than the minimum number, less than the minimum quantity) of platelets required to prepare one platelet unit. In some embodiments, the platelet donation comprises an amount (*e.g.,* number) of platelets that is less than about 99%, 98%, 95%, 90%, 85%, 80%, 75%, 70%, 65%, 60%, 55%, 50%, 45%, 40%, 35%, 30% or about 25% of the number of platelets required to prepare one platelet unit. In some embodiments, the platelet donation comprises an amount of platelets that is at least 10% of the number of platelets required to prepare one platelet unit. In some embodiments, the platelet donation comprises an amount of platelets that is about 10% to about 98%, about 15% to about 98%, about 20% to about 98%, about 25% to about 98%, about 30% to about 98%, about 35% to about 98%, about 40% to about 98% or about 50% to about 98% of the number of platelets required to prepare one platelet unit. In some embodiments, the platelet donation comprises an amount of platelets that is about 98%, 95%, 90%, 85%, 80%, 75%, 70%, 65%, 60%, 55%, 50%, 45%, 40%, 35%, 30% or about 25% of the number of platelets required to prepare one platelet unit. In some embodiments, collecting the first platelet donation from the first donor comprises targeting the platelet collection to yield a first platelet donation comprising less than about 99%, 98%, 95%, 90%, 85%, 80%, 75%, 70%, 65%, 60%, 55%, 50%, 45%, 40%, 35%, 30% or less than about 25% of the number of platelets required to prepare one platelet unit.

In some embodiments, the first platelet donation comprises greater than the number (*e.g.,* greater than the minimum number, greater than the minimum quantity) of platelets required to prepare one platelet unit, but less than the number (*e.g.,* less than the minimum number, less than the minimum quantity) of platelets required to prepare two platelet units. In some embodiments, greater than the number of platelets required to prepare one platelet unit, but less than the number of platelets required to prepare two platelet units is about 1.1-fold, 1.2-fold, 1.3-fold, 1.4-fold, 1.5-fold, 1.6-fold, 1.7-fold, 1.8-fold, 1.9-fold, 1.95-fold or about 1.98-fold the number of platelets required to prepare one platelet unit. In some embodiments, greater than the number of platelets required to prepare one platelet unit, but less than the number of platelets required to prepare two platelet units is about 1.1-fold to about 1.98-fold, about 1.15-fold to about 1.98-fold, about 1.2-fold to about 1.98-fold, about 1.25-fold to about 1.98-fold, 1.3-fold to about 1.98-fold, about 1.35-fold to about 1.98-fold, about 1.4-fold to about 1.98-fold or about 1.5-fold to about 1.98-fold the number of platelets required to prepare one platelet unit. In some embodiments, greater than the number of platelets required to prepare one platelet unit, but less than the number of platelets required to prepare two platelet units is about 105%, 110%, 115%, 120%, 125%, 130%, 135%, 140%, 145%, 150%, 155%, 160%, 165%, 170%, 175%, 180%, 185%, 190% or about 195% the number of platelets required to prepare one platelet unit. In some embodiments, greater than the number of platelets required to prepare one platelet unit, but less than the number of platelets required to prepare two platelet units is about 110% to about 198%, about 115% to about 198%, about 120% to about 198%, about 125% to about 198%, about 130% to about 198%, about 135% to about 198%, about 140% to about 198% or about 150% to about 198% the number of platelets required to prepare one platelet unit. In some embodiments, collecting the first platelet donation from the first donor comprises targeting the platelet collection to yield a first platelet donation comprising at least about 125%, 130%, 135%, 140%, 145%, 150%, 155%, 160%, 165%, 170%, 175%, 180%, 185%, 190% or about 195% of the number of platelets required to prepare one platelet unit, but less than the number of platelets required to prepare two platelet units.

In some embodiments, the first platelet donation comprises greater than the number of platelets required to prepare two platelet units, but less than the number of platelets required to prepare three platelet units. In some embodiments, greater than the number of platelets required to prepare two platelet units, but less than the number of platelets required to prepare three platelet units is about 1. 1-fold, 1.2-fold, 1.3-fold, 1.4-fold, 1.5-fold, 1.6-fold, 1.7-fold, 1.8-fold, 1.9-fold, 1.95-fold or about 1.98-fold the number of platelets required to prepare two platelet units. In some embodiments, greater than the number of platelets required to prepare two platelet units, but less than the number of platelets required to prepare three platelet units is about 1. 1-fold to about 1.98-fold, about 1.15-fold to about 1.98-fold, about 1.2-fold to about 1.98-fold, about 1.25-fold to about 1.98-fold, about 1.3-fold to about 1.98-fold, about 1.35-fold to about 1.98-fold, about 1.4-fold to about 1.98-fold or about 1.5-fold to about 1.98-fold the number of platelets required to prepare two platelet units. In some embodiments, greater than the number of platelets required to prepare two platelet units, but less than the number of platelets required to prepare three platelet units is about 105%, 110%, 115%, 120%, 125%, 130%, 135%, 140%, 145%, 150%, 155%, 160%, 165%, 170%, 175%, 180%, 185%, 190% or about 195% the number of platelets required to prepare two platelet units. In some embodiments, greater than the number of platelets required to prepare two platelet units, but less than the number of platelets required to prepare three platelet units is about 110% to about 198%, about 115% to about 198%, about 120% to about 198%, about 125% to about 198%, about 130% to about 198%, about 135% to about 198%, about 140% to about 198% or about 150% to about 198% the number of platelets required to prepare two platelet units. In some embodiments, collecting the first platelet donation from the first donor comprises targeting the platelet collection to yield a first platelet donation comprising at least about 225%, 230%, 235%, 240%, 245%, 250%, 255%, 260%, 265%, 270%, 275%, 280%, 285%, 290% or at least about 295% of the number of platelets required to prepare one platelet unit, but less than the number of platelets required to prepare three platelet units. In some embodiments, the first platelet donation comprises about 210% to about 298%, about 215% to about 298%, about 220% to about 298%, about 225% to about 298%, about 230% to about 298%, about 235% to about 298%, about 240% to about 298%, about 245% to about 298%, or about 250% to about 298% of the number of platelets required to prepare one platelet unit.

In some embodiments, the first platelet donation comprises greater than the number of platelets required to prepare three platelet units, but less than the number of platelets required to prepare four platelet units. In some embodiments, greater than the number of platelets required to prepare three platelet units, but less than the number of platelets required to prepare four platelet units is about 1. 1-fold, 1.2-fold, 1.3-fold, 1.4-fold, 1.5-fold, 1.6-fold, 1.7-fold, 1.8-fold, 1.9-fold, 1.95-fold or about 1.98-fold the number of platelets required to prepare three platelet units. In some embodiments, greater than the number of platelets required to prepare three platelet units, but less than the number of platelets required to prepare four platelet units is about 1. 1-fold to about 1.98-fold, about 1.15-fold to about 1.98-fold, about 1.2-fold to about 1.98-fold, about 1.25-fold to about 1.98-fold, about 1.3-fold to about 1.98-fold, about 1.35-fold to about 1.98-fold, about 1.4-fold to about 1.98-fold or about 1.5-fold to about 1.98-fold the number of platelets required to prepare three platelet units. In some embodiments, greater than the number of platelets required to prepare three platelet units, but less than the number of platelets required to prepare four platelet units is about 105%, 110%, 115%, 120%, 125%, 130%, 135%, 140%, 145%, 150%, 155%, 160%, 165%, 170%, 175%, 180%, 185%, 190% or about 195% the number of platelets required to prepare three platelet units. In some embodiments, greater than the number of platelets required to prepare three platelet units, but less than the number of platelets required to prepare four platelet units is about 110% to about 198%, about 115% to about 198%, about 120% to about 198%, about 125% to about 198%, about 130% to about 198%, about 135% to about 198%, about 140% to about 198% or about 150% to about 198% the number of platelets required to prepare three platelet units. In some embodiments, collecting the first platelet donation from the first donor comprises targeting the platelet collection to yield a first platelet donation comprising at least about 325%, 330%, 335%, 340%, 345%, 350%, 355%, 360%, 365%, 370%, 375%, 380%, 385%, 390% or at least about 395% of the number of platelets required to prepare one platelet unit, but less than the number of platelets required to prepare three platelet units. In some embodiments, the first platelet donation comprises about 310% to about 398%, about 315% to about 398%, about 320% to about 398%, about 325% to about 398%, about 330% to about 398%, about 335% to about 398%, about 340% to about 398%, about 345% to about 398%, or about 350% to about 398% of the number of platelets required to prepare one platelet unit.

In some embodiments, the second platelet donation comprises greater than the number of platelets required to prepare one platelet unit, but less than the number of platelets required to prepare two platelet units. In some embodiments, greater than the number of platelets required to prepare one platelet unit, but less than the number of platelets required to prepare two platelet units is about 1.1-fold, 1.2-fold, 1.3-fold, 1.4-fold, 1.5-fold, 1.6-fold, 1.7-fold, 1.8-fold, 1.9-fold, 1.95-fold or about 1.98-fold the number of platelets required to prepare one platelet unit. In some embodiments, greater than the number of platelets required to prepare one platelet unit, but less than the number of platelets required to prepare two platelet units is about 1.1-fold to about 1.98-fold, about 1.15-fold to about 1.98-fold, about 1.2-fold to about 1.98-fold, about 1.25-fold to about 1.98-fold, about 1.3-fold to about 1.98-fold, about 1.35-fold to about 1.98-fold, about 1.4-fold to about 1.98-fold or about 1.5-fold to about 1.98-fold the number of platelets required to prepare one platelet unit. In some embodiments, greater than the number of platelets required to prepare one platelet unit, but less than the number of platelets required to prepare two platelet units is about 105%, 110%, 115%, 120%, 125%, 130%, 135%, 140%, 145%, 150%, 155%, 160%, 165%, 170%, 175%, 180%, 185%, 190% or about 195% the number of platelets required to prepare one platelet unit. In some embodiments, greater than the number of platelets required to prepare one platelet unit, but less than the number of platelets required to prepare two platelet units is about 110% to about 198%, about 115% to about 198%, about 120% to about 198%, about 125% to about 198%, about 130% to about 198%, about 135% to about 198%, about 140% to about 198% or about 150% to about 198% the number of platelets required to prepare one platelet unit. In some embodiments, collecting the second platelet donation from the second donor comprises targeting the platelet collection to yield a second platelet donation comprising at least about 125%, 130%, 135%, 140%, 145%, 150%, 155%, 160%, 165%, 170%, 175%, 180%, 185%, 190% or about 195% of the number of platelets required to prepare one platelet unit, but less than the number of platelets required to prepare two platelet units. In some embodiments, collecting the second platelet donation from the second donor comprises targeting the platelet collection to yield a second platelet donation comprising a sufficient number of platelets to prepare two platelet units, to prepare three platelet units, to prepare four platelet units, or to prepare five platelet units after combining the first and second platelet donations and separating the resulting pooled platelet preparation into a plurality of platelet units. In some embodiments, the second platelet donation comprises a sufficient number of platelets that, when combined with the first platelet donation, yields a pooled platelet preparation comprising a sufficient number of platelets to separate into two, into three, into four, or into five platelet units. In some embodiments, collecting the second platelet donation from the second donor comprises collecting a sufficient number of platelets to prepare two platelet units, to prepare three platelet units, to prepare four platelet units, or to prepare five platelet units from the pooled preparation.

In some embodiments, the second platelet donation comprises greater than the number of platelets required to prepare two platelet units, but less than the number of platelets required to prepare three platelet units. In some embodiments, greater than the number of platelets required to prepare two platelet units, but less than the number of platelets required to prepare three platelet units is about 1.1-fold, 1.2-fold, 1.3-fold, 1.4-fold, 1.5-fold, 1.6-fold, 1.7-fold, 1.8-fold, 1.9-fold, 1.95-fold or about 1.98-fold the number of platelets required to prepare two platelet units. In some embodiments, greater than the number of platelets required to prepare two platelet units, but less than the number of platelets required to prepare three platelet units is about 1. 1-fold to about 1.98-fold, about 1.15-fold to about 1.98-fold, about 1.2-fold to about 1.98-fold, about 1.25-fold to about 1.98-fold, about 1.3-fold to about 1.98-fold, about 1.35-fold to about 1.98-fold, about 1.4-fold to about 1.98-fold or about 1.5-fold to about 1.98-fold the number of platelets required to prepare two platelet units. In some embodiments, greater than the number of platelets required to prepare two platelet units, but less than the number of platelets required to prepare three platelet units is about 105%, 110%, 115%, 120%, 125%, 130%, 135%, 140%, 145%, 150%, 155%, 160%, 165%, 170%, 175%, 180%, 185%, 190% or about 195% the number of platelets required to prepare two platelet units. In some embodiments, greater than the number of platelets required to prepare two platelet units, but less than the number of platelets required to prepare three platelet units is about 110% to about 198%, about 115% to about 198%, about 120% to about 198%, about 125% to about 198%, about 130% to about 198%, about 135% to about 198%, about 140% to about 198% or about 150% to about 198% the number of platelets required to prepare two platelet units. In some embodiments, greater than the number of platelets required to prepare two platelet units, but less than the number of platelets required to prepare three platelet units is about 210% to about 298%, about 215% to about 298%, about 220% to about 298%, about 225% to about 298%, about 230% to about 298%, about 235% to about 298%, about 240% to about 298% or about 250% to about 298% the number of platelets required to prepare one platelet unit. In some embodiments, collecting the second platelet donation from the second donor comprises targeting the platelet collection to yield a second platelet donation comprising at least about 225%, 230%, 235%, 240%, 245%, 250%, 255%, 260%, 265%, 270%, 275%, 280%, 285%, 290% or about 295% of the number of platelets required to prepare one platelet unit, but less than the number of platelets required to prepare three platelet units. In some embodiments, collecting the second platelet donation from the second donor comprises targeting the platelet collection to yield a second platelet donation comprising a sufficient number of platelets to prepare three platelet units, to prepare four platelet units, to prepare five platelet units, or to prepare six platelet units after combining the first and second platelet donations and separating the resulting pooled platelet preparation into a plurality of platelet units. In some embodiments, the second platelet donation comprises a sufficient number of platelets that, when combined with the first platelet donation, yields a pooled platelet preparation comprising a sufficient number of platelets to separate into three, into four, into five, or into six platelet units. In some embodiments, collecting the second platelet donation from the second donor comprises collecting a sufficient number of platelets to prepare three platelet units, to prepare four platelet units, to prepare five platelet units, or to prepare six platelet units from the pooled preparation.

In some embodiments, the second platelet donation comprises greater than the number of platelets required to prepare three platelet units, but less than the number of platelets required to prepare four platelet units. In some embodiments, greater than the number of platelets required to prepare three platelet units, but less than the number of platelets required to prepare four platelet units is about 1. 1-fold, 1.2-fold, 1.3-fold, 1.4-fold, 1.5-fold, 1.6-fold, 1.7-fold, 1.8-fold, 1.9-fold, 1.95-fold or about 1.98-fold the number of platelets required to prepare three platelet units. In some embodiments, greater than the number of platelets required to prepare three platelet units, but less than the number of platelets required to prepare four platelet units is about 1. 1-fold to about 1.98-fold, about 1.15-fold to about 1.98-fold, about 1.2-fold to about 1.98-fold, about 1.25-fold to about 1.98-fold, about 1.3-fold to about 1.98-fold, about 1.35-fold to about 1.98-fold, about 1.4-fold to about 1.98-fold or about 1.5-fold to about 1.98-fold the number of platelets required to prepare three platelet units. In some embodiments, greater than the number of platelets required to prepare three platelet units, but less than the number of platelets required to prepare four platelet units is about 105%, 110%, 115%, 120%, 125%, 130%, 135%, 140%, 145%, 150%, 155%, 160%, 165%, 170%, 175%, 180%, 185%, 190% or about 195% the number of platelets required to prepare three platelet units. In some embodiments, greater than the number of platelets required to prepare three platelet units, but less than the number of platelets required to prepare four platelet units is about 110% to about 198%, about 115% to about 198%, about 120% to about 198%, about 125% to about 198%, about 130% to about 198%, about 135% to about 198%, about 140% to about 198% or about 150% to about 198% the number of platelets required to prepare three platelet units. In some embodiments, greater than the number of platelets required to prepare two platelet units, but less than the number of platelets required to prepare three platelet units is about 310% to about 398%, about 315% to about 398%, about 320% to about 398%, about 325% to about 398%, about 330% to about 398%, about 335% to about 398%, about 340% to about 398% or about 350% to about 398% the number of platelets required to prepare one platelet unit. In some embodiments, collecting the second platelet donation from the second donor comprises targeting the platelet collection to yield a second platelet donation comprising at least about 325%, 330%, 335%, 340%, 345%, 350%, 355%, 360%, 365%, 370%, 375%, 380%, 385%, 390% or about 395% of the number of platelets required to prepare one platelet unit, but less than the number of platelets required to prepare four platelet units. In some embodiments, collecting the second platelet donation from the second donor comprises targeting the platelet collection to yield a second platelet donation comprising a sufficient number of platelets to prepare four platelet units, to prepare five platelet units, to prepare six platelet units, or to prepare seven platelet units after combining the first and second platelet donations and separating the resulting pooled platelet preparation into a plurality of platelet units. In some embodiments, the second platelet donation comprises a sufficient number of platelets that, when combined with the first platelet donation, yields a pooled platelet preparation comprising a sufficient number of platelets to separate into four, into five, into six, or into seven platelet units. In some embodiments, collecting the second platelet donation from the second donor comprises collecting a sufficient number of platelets to prepare four platelet units, to prepare five platelet units, to prepare six platelet units, or to prepare seven platelet units from the pooled preparation.

In some embodiments, the first platelet donation comprises less than the number of platelets required to prepare one platelet unit and the second platelet donation comprises greater than the number of platelets required to prepare one platelet unit, but less than the number of platelets required to prepare two platelet units. In some embodiments, the first platelet donation comprises less than the number of platelets required to prepare one platelet unit and the second platelet donation comprises greater than the number of platelets required to prepare two platelet units, but less than the number of platelets required to prepare three platelet units. In some embodiments, the first platelet donation comprises less than the number of platelets required to prepare one platelet unit and the second platelet donation comprises greater than the number of platelets required to prepare three platelet units, but less than the number of platelets required to prepare four platelet units. In some embodiments, the pooled platelet preparation comprises a sufficient number of platelets to prepare two platelet units, to prepare three platelet units, or to prepare four platelet units. In some embodiments, the plurality of platelet units comprises 2 platelet units, 3 platelet units or 4 platelet units.

In some embodiments, the first platelet donation comprises greater than the number of platelets required to prepare one platelet unit, but less than the number of platelets required to prepare two platelet units, and the second platelet donation comprises greater than the number of platelets required to prepare one platelet unit, but less than the number of platelets required to prepare two platelet units. In some embodiments, the first platelet donation comprises greater than the number of platelets required to prepare one platelet unit, but less than the number of platelets required to prepare two platelet units, and the second platelet donation comprises greater than the number of platelets required to prepare two platelet units, but less than the number of platelets required to prepare three platelet units. In some embodiments, the first platelet donation comprises greater than the number of platelets required to prepare one platelet unit, but less than the number of platelets required to prepare two platelet units, and the second platelet donation comprises greater than the number of platelets required to prepare three platelet units, but less than the number of platelets required to prepare four platelet units. In some embodiments, the pooled platelet preparation comprises a sufficient number of platelets to prepare three platelet units, to prepare four platelet units, or to prepare five platelet units. In some embodiments, the plurality of platelet units comprises 3 platelet units, 4 platelet units or 5 platelet units.

In some embodiments, the first platelet donation comprises greater than the number of platelets required to prepare two platelet units, but less than the number of platelets required to prepare three platelet units, and the second platelet donation comprises greater than the number of platelets required to prepare two platelet units, but less than the number of platelets required to prepare three platelet units. In some embodiments, the first platelet donation comprises greater than the number of platelets required to prepare two platelet units, but less than the number of platelets required to prepare three platelet units, and the second platelet donation comprises greater than the number of platelets required to prepare three platelet units, but less than the number of platelets required to prepare four platelet units. In some embodiments, the first platelet donation comprises greater than the number of platelets required to prepare three platelet units, but less than the number of platelets required to prepare four platelet units, and the second platelet donation comprises greater than the number of platelets required to prepare three platelet units, but less than the number of platelets required to prepare four platelet units. In some embodiments, the pooled platelet preparation comprises a sufficient number of platelets to prepare five platelet units, to prepare six platelet units, or to prepare seven platelet units. In some embodiments, the plurality of platelet units comprises 5 platelet units, 6 platelet units or 7 platelet units.

In some of the aforementioned embodiments, the method further comprises collecting a third platelet donation by apheresis from a third donor, and combining the third platelet donation with the first and the second platelet donations to yield a pooled platelet preparation, and separating the pooled platelet preparation into a plurality of platelet units each in an individual container, wherein the plurality of platelet units comprises a greater number of platelet units than the total number of platelet units that can be prepared from the first platelet donation, the second platelet donation and the third platelet donation without combining the first, second and third platelet donations. In some embodiments, the third platelet donation comprises greater than the number of platelets required to prepare one platelet unit, but less than the number of platelets required to prepare two platelet units. In some embodiments, the third platelet donation comprises greater than the number of platelets required to prepare two platelet units, but less than the number of platelets required to prepare three platelet units. In some embodiments, the third platelet donation comprises greater than the number of platelets required to prepare three platelet units, but less than the number of platelets required to prepare four platelet units.

In some of the aforementioned embodiments, the second platelet donation is collected less than about (e.g., within) 24 hours, 22 hours, 20 hours, 16 hours, 12 hours, 10 hours, 8 hours, 6 hours, 5 hours, 4 hours, 3 hours, 2 hours or less than about 1 hour from the time the first platelet donation is collected. In some embodiments, the second platelet donation is collected about the same time (e.g., overlapping collection time) as the first platelet donation. In some embodiments, the second platelet donation is collected within about 7 days, within about 6 days, within about 5 days, within about 4 days, within about 3 days, within about 2 days, or within about 1 day from the time the first platelet donation is collected. In some embodiments, the second platelet donation is collected within 20 hours from the time the first platelet donation is collected. In some embodiments, the second platelet donation is collected within 12 hours from the time the first platelet donation is collected. In some embodiments, the second platelet donation is collected within 6 hours from the time the first platelet donation is collected. In some of the aforementioned embodiments, the third platelet donation is collected less than about (e.g., within) 24 hours, 22 hours, 20 hours, 16 hours, 12 hours, 10 hours, 8 hours, 6 hours, 5 hours, 4 hours, 3 hours, 2 hours or less than about 1 hour from the time the first platelet donation is collected. In some embodiments, the third platelet donation is collected about the same time (e.g., overlapping collection time) as the first platelet donation. In some embodiments, the third platelet donation is collected within about 7 days, within about 6 days, within about 5 days, within about 4 days, within about 3 days, within about 2 days, or within about 1 day from the time the first platelet donation is collected. In some embodiments, the third platelet donation is collected within 20 hours from the time the first platelet donation is collected. In some embodiments, the third platelet donation is collected within 12 hours from the time the first platelet donation is collected. In some embodiments, the third platelet donation is collected within 6 hours from the time the first platelet donation is collected. In some embodiments, the first platelet donation, the second platelet donation and the third platelet donation are collected within a period *(e.g.,* the same period) of about 24 hours, 22 hours, 20 hours, 16 hours, 12 hours, 10 hours, 8 hours, 6 hours, 5 hours, 4 hours, 3 hours, 2 hours or about 1 hour. In some embodiments, the first platelet donation, the second platelet donation and the third platelet donation are collected at about the same time (e.g., overlapping collection times). In some embodiments, the first platelet donation, the second platelet donation and the third platelet donation are collected within about 7 days, within about 6 days, within about 5 days, within about 4 days, within about 3 days, within about 2 days, within about 1 day, within about 72 hours, within about 48 hours, within about 36 hours, within about 24 hours, within about 20 hours, within about 12 hours or within about 6 hours of each other. In some of the aforementioned embodiments, the pooled platelet preparation comprises platelets only from donors (*e.g.,* first donor, second donor, third donor) of the same ABO blood type.

In some of the aforementioned embodiments, the method further comprises subjecting the first platelet donation, the second platelet donation, and/or the third platelet donation to a pathogen inactivation process. In some embodiments, the method further comprises subjecting each of the first platelet donation and the second platelet donation to a pathogen inactivation process. In some embodiments, the method further comprises subjecting each of the first platelet donation, the second platelet donation and the third platelet donation to a pathogen inactivation process. In some embodiments, the method further comprises subjecting the pooled platelet preparation to a pathogen inactivation process. In some embodiments, subjecting the platelet donation or pooled platelet preparation to a pathogen inactivation process comprises treating the platelet donation or pooled platelet preparation with a pathogen inactivating compound. In some embodiments, the pathogen inactivating compound is a photoactive pathogen inactivating compound selected from the group consisting of a psoralen, an isoalloxazine, an alloxazine, a phthalocyanine, a phenothiazine, a porphyrin, and merocyanine 540. In some embodiments, the pathogen inactivating compound is a psoralen. In some embodiments, the pathogen inactivating compound is amotosalen.

In some of the aforementioned embodiments, the platelet units (*e.g.,* each of the platelet units) in the plurality of platelet units comprise at least about 2.0×10¹¹ platelets, at least about 2.2×10¹¹ platelets, at least about 2.4×10¹¹ platelets, at least about 2.5×10¹¹ platelets, at least about 2.6×10¹¹ platelets, at least about 2.7×10¹¹ platelets, at least about 2.8×10¹¹ platelets, at least about 2.9×10¹¹ platelets or at least about 3.0×10¹¹ platelets. In some embodiments, the platelet units each comprise at least about 3.0×10¹¹ platelets, and the pooled platelet preparation comprises at least about 6.0×10¹¹, at least about 6.5×10¹¹, at least about 7.0×10¹¹, at least about 7.5×10¹¹, at least about 8.0×10¹¹, at least about 8.5×10¹¹, at least about 9.0×10¹¹, at least about 9.5×10¹¹, at least about 10.0×10¹¹, at least about 10.5×10¹¹, at least about 11.0×10¹¹, at least about 11.5×10¹¹, at least about 12.0×10¹¹, at least about 12.5×10¹¹, at least about 13.0×10¹¹, at least about 13.5×10¹¹, at least about 14.0×10¹¹, at least about 14.5×10¹¹, or at least about 15.0×10¹¹ platelets. In some embodiments, the platelet units each comprise at least about 3.0×10¹¹ platelets, and the pooled platelet preparation comprises at least about 6.1×10¹¹, at least about 6.2×10¹¹, at least about 6.3×10¹¹, at least about 6.4×10¹¹, or at least about 6.5×10¹¹ platelets. In some embodiments, the platelet units each comprise at least about 3.0×10¹¹ platelets, and the pooled platelet preparation comprises at least about 9.2×10¹¹, at least about 9.3×10¹¹, at least about 9.4×10¹¹, at least about 9.5×10¹¹, or at least about 9.6×10¹¹ platelets. In some embodiments, the platelet units each comprise at least about 3.0×10¹¹ platelets, and the pooled platelet preparation comprises at least about 12.3×10¹¹, at least about 12.4×10¹¹, at least about 12.5×10¹¹, at least about 12.6×10¹¹, at least about 12.7×10¹¹ platelets. In some embodiments, the platelet units each comprise at least about 3.0×10¹¹ platelets, and the pooled platelet preparation comprises at least about 15.4×10¹¹, at least about 15.5×10¹¹, at least about 15.6×10¹¹, at least about 15.7×10¹¹, at least about 15.8×10¹¹, at least about 16.0×10¹¹ platelets. In some embodiments, the platelet units each comprise at least about 3.0×10¹¹ platelets, and the pooled platelet preparation comprises less than about 16.0×10¹¹, less than about 14.0×10¹¹, or less than about 12.0×10¹¹ platelets. In some embodiments, the platelet units each comprise at least about 3.0×10¹¹ platelets, and the pooled platelet preparation comprises about 6.0×10¹¹, about 6.5×10¹¹, about 7.0×10¹¹, about 7.5×10¹¹, about 8.0×10¹¹, about 8.5×10¹¹, about 9.0×10¹¹, about 9.5×10¹¹, about 10.0×10¹¹, about 10.5×10¹¹, about 11.0×10¹¹, about 11.5×10¹¹, about 12.0×10¹¹, about 12.5×10¹¹, about 13.0×10¹¹, about 13.5×10¹¹, about 14.0×10¹¹, about 14.5×10¹¹, or about 15.0×10¹¹ platelets.

In some embodiments, the platelet units each comprise at least about 2.0×10¹¹ platelets, and the pooled platelet preparation comprises at least about 5.0×10¹¹, at least about 5.5×10¹¹, at least about 6.0×10¹¹, at least about 6.5×10¹¹, at least about 7.0×10¹¹, at least about 7.5×10¹¹, at least about 8.0×10¹¹, at least about 8.5×10¹¹, at least about 9.0×10¹¹, at least about 9.5×10¹¹, at least about 10.0×10¹¹, at least about 10.5×10¹¹, at least about 11.0×10¹¹, at least about 11.5×10¹¹, at least about 12.0×10¹¹, at least about 12.5×10¹¹, at least about 13.0×10¹¹, at least about 13.5×10¹¹, at least about 14.0×10¹¹, at least about 14.5×10¹¹, or at least about 15.0×10¹¹ platelets. In some embodiments, the platelet units each comprise at least about 2.0×10¹¹ platelets, and the pooled platelet preparation comprises less than about 16.0×10¹¹, less than about 14.0×10¹¹, or less than about 12.0×10¹¹ platelets. In some embodiments, the platelet units each comprise at least about 2.0×10¹¹ platelets, and the pooled platelet preparation comprises about 5.0×10¹¹, about 5.5×10¹¹, about 6.0×10¹¹, about 6.5×10¹¹, about 7.0×10¹¹, about 7.5×10¹¹, about 8.0×10¹¹, about 8.5×10¹¹, about 9.0×10¹¹, about 9.5×10¹¹, about 10.0×10¹¹, about 10.5×10¹¹, about 11.0×10¹¹, about 11.5×10¹¹, about 12.0×10¹¹, about 12.5×10¹¹, about 13.0×10¹¹, about 13.5×10¹¹, about 14.0×10¹¹, about 14.5×10¹¹, or about 15.0×10¹¹ platelets.

In some embodiments, the platelet units each comprise at least about 2.2×10¹¹ platelets, and the pooled platelet preparation comprises at least about 5.0×10¹¹, at least about 5.5×10¹¹, at least about 6.0×10¹¹, at least about 6.5×10¹¹, at least about 7.0×10¹¹, at least about 7.5×10¹¹, at least about 8.0×10¹¹, at least about 8.5×10¹¹, at least about 9.0×10¹¹, at least about 9.5×10¹¹, at least about 10.0×10¹¹, at least about 10.5×10¹¹, at least about 11.0×10¹¹, at least about 11.5×10¹¹, at least about 12.0×10¹¹, at least about 12.5×10¹¹, at least about 13.0×10¹¹, at least about 13.5×10¹¹, at least about 14.0×10¹¹, at least about 14.5×10¹¹, or at least about 15.0×10¹¹ platelets. In some embodiments, the platelet units each comprise at least about 2.2×10¹¹ platelets, and the pooled platelet preparation comprises less than about 16.0×10¹¹, less than about 14.0×10¹¹, or less than about 12.0×10¹¹ platelets. In some embodiments, the platelet units each comprise at least about 2.2×10¹¹ platelets, and the pooled platelet preparation comprises about 5.0×10¹¹, about 5.5×10¹¹, about 6.0×10¹¹, about 6.5×10¹¹, about 7.0×10¹¹, about 7.5×10¹¹, about 8.0×10¹¹, about 8.5×10¹¹, about 9.0×10¹¹, about 9.5×10¹¹, about 10.0×10¹¹, about 10.5×10¹¹, about 11.0×10¹¹, about 11.5×10¹¹, about 12.0×10¹¹, about 12.5×10¹¹, about 13.0×10¹¹, about 13.5×10¹¹, about 14.0×10¹¹, about 14.5×10¹¹, or about 15.0×10¹¹ platelets.

In some embodiments, the platelet units each comprise at least about 2.4×10¹¹ platelets, and the pooled platelet preparation comprises at least about 5.0×10¹¹, at least about 5.5×10¹¹, at least about 6.0×10¹¹, at least about 6.5×10¹¹, at least about 7.0×10¹¹, at least about 7.5×10¹¹, at least about 8.0×10¹¹, at least about 8.5×10¹¹, at least about 9.0×10¹¹, at least about 9.5×10¹¹, at least about 10.0×10¹¹, at least about 10.5×10¹¹, at least about 11.0×10¹¹, at least about 11.5×10¹¹, at least about 12.0×10¹¹, at least about 12.5×10¹¹, at least about 13.0×10¹¹, at least about 13.5×10¹¹, at least about 14.0×10¹¹, at least about 14.5×10¹¹, or at least about 15.0×10¹¹ platelets. In some embodiments, the platelet units each comprise at least about 2.4×10¹¹ platelets, and the pooled platelet preparation comprises less than about 16.0×10¹¹, less than about 14.0×10¹¹, or less than about 12.0×10¹¹ platelets. In some embodiments, the platelet units each comprise at least about 2.4×10¹¹ platelets, and the pooled platelet preparation comprises about 5.0×10¹¹, about 5.5×10¹¹, about 6.0×10¹¹, about 6.5×10¹¹, about 7.0×10¹¹, about 7.5×10¹¹, about 8.0×10¹¹, about 8.5×10¹¹, about 9.0×10¹¹, about 9.5×10¹¹, about 10.0×10¹¹, about 10.5×10¹¹, about 11.0×10¹¹, about 11.5×10¹¹, about 12.0×10¹¹, about 12.5×10¹¹, about 13.0×10¹¹, about 13.5×10¹¹, about 14.0×10¹¹, about 14.5×10¹¹, or about 15.0×10¹¹ platelets.

In some embodiments, the platelet units each comprise at least about 2.6×10¹¹ platelets, and the pooled platelet preparation comprises at least about 5.0×10¹¹, at least about 5.5×10¹¹, at least about 6.0×10¹¹, at least about 6.5×10¹¹, at least about 7.0×10¹¹, at least about 7.5×10¹¹, at least about 8.0×10¹¹, at least about 8.5×10¹¹, at least about 9.0×10¹¹, at least about 9.5×10¹¹, at least about 10.0×10¹¹, at least about 10.5×10¹¹, at least about 11.0×10¹¹, at least about 11.5×10¹¹, at least about 12.0×10¹¹, at least about 12.5×10¹¹, at least about 13.0×10¹¹, at least about 13.5×10¹¹, at least about 14.0×10¹¹, at least about 14.5×10¹¹, or at least about 15.0×10¹¹ platelets. In some embodiments, the platelet units each comprise at least about 2.6×10¹¹ platelets, and the pooled platelet preparation comprises less than about 16.0×10¹¹, less than about 14.0×10¹¹, or less than about 12.0×10¹¹ platelets. In some embodiments, the platelet units each comprise at least about 2.6×10¹¹ platelets, and the pooled platelet preparation comprises about 5.0×10¹¹, about 5.5×10¹¹, about 6.0×10¹¹, about 6.5×10¹¹, about 7.0×10¹¹, about 7.5×10¹¹, about 8.0×10¹¹, about 8.5×10¹¹, about 9.0×10¹¹, about 9.5×10¹¹, about 10.0×10¹¹, about 10.5×10¹¹, about 11.0×10¹¹, about 11.5×10¹¹, about 12.0×10¹¹, about 12.5×10¹¹, about 13.0×10¹¹, about 13.5×10¹¹, about 14.0×10¹¹, about 14.5×10¹¹, or about 15.0×10¹¹ platelets.

In some embodiments, the platelet units each comprise at least about 2.8×10¹¹ platelets, and the pooled platelet preparation comprises at least about 5.0×10¹¹, at least about 5.5×10¹¹, at least about 6.0×10¹¹, at least about 6.5×10¹¹, at least about 7.0×10¹¹, at least about 7.5×10¹¹, at least about 8.0×10¹¹, at least about 8.5×10¹¹, at least about 9.0×10¹¹, at least about 9.5×10¹¹, at least about 10.0×10¹¹, at least about 10.5×10¹¹, at least about 11.0×10¹¹, at least about 11.5×10¹¹, at least about 12.0×10¹¹, at least about 12.5×10¹¹, at least about 13.0×10¹¹, at least about 13.5×10¹¹, at least about 14.0×10¹¹, at least about 14.5×10¹¹, or at least about 15.0×10¹¹ platelets. In some embodiments, the platelet units each comprise at least about 2.8×10¹¹ platelets, and the pooled platelet preparation comprises less than about 16.0×10¹¹, less than about 14.0×10¹¹, or less than about 12.0×10¹¹ platelets. In some embodiments, the platelet units each comprise at least about 2.8×10¹¹ platelets, and the pooled platelet preparation comprises about 5.0×10¹¹, about 5.5×10¹¹, about 6.0×10¹¹, about 6.5×10¹¹, about 7.0×10¹¹, about 7.5×10¹¹, about 8.0×10¹¹, about 8.5×10¹¹, about 9.0×10¹¹, about 9.5×10¹¹, about 10.0×10¹¹, about 10.5×10¹¹, about 11.0×10¹¹, about 11.5×10¹¹, about 12.0×10¹¹, about 12.5×10¹¹, about 13.0×10¹¹, about 13.5×10¹¹, about 14.0×10¹¹, about 14.5×10¹¹, or about 15.0×10¹¹ platelets.

In the aforementioned embodiments, the platelet units (*e.g.,* each platelet unit) in the plurality of platelet units comprise a therapeutic dose (*e.g.,* therapeutic dosage unit) of platelets suitable for infusion into a human subject (*e.g.,* subject in need of a platelet infusion). In some embodiments, the therapeutic dose comprises a minimum number (*e.g.,* at least a minimum number) of platelets as defined by criteria (*e.g.,* acceptance criteria) of a governmental agency, regulatory agency, institution and/or accrediting organization (*e.g.,* governmental agency, regulatory agency, institution and/or accrediting organization responsible for donated blood products (*e.g.,* donated platelets)). In some embodiments, the platelet units are prepared in the country of the governmental agency, regulatory agency, institution and/or accrediting organization defining the criteria of a therapeutic dose of platelets. In some embodiments, at least about 75%, at least about 80%, at least about 85%, at least about 90%, at least about 95%, at least about 98%, at least about 99% or 100% of the platelet units in the plurality of platelet units comprise the minimum number of platelets of a therapeutic dose. In some embodiments, each of the platelet units in the plurality of platelet units comprises the minimum number of platelets of a therapeutic dose. In some embodiments, the minimum number of platelets in a therapeutic dose is at least about at least about 2.0×10¹¹ platelets, at least about 2.2×10¹¹ platelets, 2.4×10¹¹ platelets, at least about 2.5×10¹¹ platelets, at least about 2.6×10¹¹ platelets, at least about 2.7×10¹¹ platelets, at least about 2.8×10¹¹ platelets, at least about 2.9×10¹¹ platelets or at least about 3.0×10¹¹ platelets.

In some of the aforementioned embodiments, targeting the platelet collection comprises determining an optimal platelet collection. In some embodiments, an optimal platelet collection is a maximum platelet collection (*e.g.,* maximum that can be safely collected, maximum according to regulatory and/or industry standards or regulations) that can be collected from a donor. In some embodiments, an optimal platelet collection is less than a maximum platelet collection that can be collected from a donor, but greater than the platelet collection required for a particular number (*e.g.,* targeted number, desired number) of platelet units. In some embodiments, targeting the platelet collection comprises providing an input (*e.g.,* programming) to an apheresis system. In some embodiments, targeting the platelet collection comprises providing a donation volume to an apheresis system. In some embodiments, targeting the platelet collection comprises providing a duration time for the platelet collection to an apheresis system. In some embodiments, targeting the platelet collection comprises providing a donation volume and/or duration time for the platelet collection that factors in (*e.g.,* accounts for) variation (*e.g.,* distribution) in actual platelet collection yields as compared to targeted collection yields. In some embodiments, the first platelet donation and the second platelet donation are combined in a single container of suitable size for the volume of the pooled platelet preparation. In some embodiments, the first platelet donation and the second platelet donation are of suitable volume for treating with a pathogen inactivating compound. In some embodiments, the first platelet donation and the second platelet donation are of suitable volume and platelet number (*e.g.,* platelet quantity) for treating with a pathogen inactivating compound. In some embodiments, the pooled platelet preparation is of suitable volume for treating with a pathogen inactivating compound. In some embodiments, the pooled platelet preparation is of suitable volume and platelet number for treating with a pathogen inactivating compound.

In another aspect, the present disclosure provides a method of preparing a plurality of platelet units suitable for infusion into a human subject), comprising: a) maintaining an inventory log of an inventory of apheresis-collected platelet preparations, the inventory log comprising for each platelet preparation a unique identifier, a donor blood type, a donation time (*e.g.,* date, time, date and time) and a quantity (*e.g.,* total platelet number, platelet count, platelet concentration and volume) of platelets in the preparation, b) identifying an available donor suitable for donating platelets by apheresis, c) selecting from the inventory log at least one (*e.g.,* one or more) platelet preparation in the inventory from a first donor for combining with platelets collected from the available donor, wherein the first donor and the available donor are different, d) collecting a new platelet donation by apheresis from the available donor, e) combining the at least one selected platelet preparation from the inventory of apheresis-collected platelet preparations and the new platelet donation to yield a pooled platelet preparation, and f) separating the pooled platelet preparation into a plurality of platelet units (*e.g.,* therapeutic doses, therapeutic dosage units) each in an individual container, wherein the plurality of platelet units comprises a greater number of platelet units than the total number of platelet units that can be prepared from the at least one selected platelet preparation from the inventory of apheresis-collected platelet preparations and the new platelet donation from the available donor without combining the at least one selected platelet preparation and new platelet donation. In some embodiments, the inventory log further comprises for each platelet preparation a donor gender and/or a platelet preparation volume. In some embodiments, the inventory of apheresis-collected platelet preparations comprises existing platelet preparations (*e.g.,* existing inventory) that have already been collected.

In some embodiments, the method further comprises determining for the available donor one or more donor parameters selected from the group consisting of gender, physical size (*e.g.,* weight, height), hemoglobin level and platelet count (*e.g.,* platelet count on day of donation, pre-donation platelet count). In some embodiments, the method further comprises determining a maximum amount (*e.g.,* maximum safe amount, maximum volume, maximum number) of platelets that can be collected from the available donor by apheresis.

In some embodiments, the at least one selected platelet preparation comprises less than the number (*e.g.,* less than the minimum number, less than the minimum quantity) of platelets required to prepare one platelet unit. In some embodiments, the at least one selected platelet preparation comprises an amount (*e.g.,* number) of platelets that is less than about 99%, 98%, 95%, 90%, 85%, 80%, 75%, 70%, 65%, 60%, 55%, 50%, 45%, 40%, 35%, 30% or less than about 25% of the number of platelets required to prepare one platelet unit. In some embodiments, the at least one selected platelet preparation comprises an amount of platelets that is at least 10% of the number of platelets required to prepare one platelet unit. In some embodiments, the at least one selected platelet preparation comprises an amount of platelets that is about 10% to about 98%, about 15% to about 98%, about 20% to about 98%, about 25% to about 98%, about 30% to about 98%, about 35% to about 98%, about 40% to about 98% or about 50% to about 98% of the number of platelets required to prepare one platelet unit. In some embodiments, the at least one selected platelet preparation comprises an amount of platelets that is about 98%, 95%, 90%, 85%, 80%, 75%, 70%, 65%, 60%, 55%, 50%, 45%, 40%, 35%, 30% or about 25% of the number of platelets required to prepare one platelet unit.

In some embodiments, the at least one selected platelet preparation from the inventory of apheresis-collected platelet preparations comprises greater than the number (*e.g.,* greater than the minimum number, greater than the minimum quantity) of platelets required to prepare one platelet unit, but less than the number (*e.g.,* less than the minimum number, less than the minimum quantity) of platelets required to prepare two platelet units. In some embodiments, greater than the number of platelets required to prepare one platelet unit, but less than the number of platelets required to prepare two platelet units is about 1.1-fold, 1.2-fold, 1.3-fold, 1.4-fold, 1.5-fold, 1.6-fold, 1.7-fold, 1.8-fold, 1.9-fold, 1.95-fold or about 1.98-fold the number of platelets required to prepare one platelet unit. In some embodiments, greater than the number of platelets required to prepare one platelet unit, but less than the number of platelets required to prepare two platelet units is about 1. 1-fold to about 1.98-fold, about 1.15-fold to about 1.98-fold, about 1.2-fold to about 1.98-fold, about 1.25-fold to about 1.98-fold, about 1.3-fold to about 1.98-fold, about 1.35-fold to about 1.98-fold, about 1.4-fold to about 1.98-fold or about 1.5-fold to about 1.98-fold the number of platelets required to prepare one platelet unit. In some embodiments, greater than the number of platelets required to prepare one platelet unit, but less than the number of platelets required to prepare two platelet units is about 105%, 110%, 115%, 120%, 125%, 130%, 135%, 140%, 145%, 150%, 155%, 160%, 165%, 170%, 175%, 180%, 185%, 190% or about 195% the number of platelets required to prepare one platelet unit. In some embodiments, greater than the number of platelets required to prepare one platelet unit, but less than the number of platelets required to prepare two platelet units is about 110% to about 198%, about 115% to about 198%, about 120% to about 198%, about 125% to about 198%, about 130% to about 198%, about 135% to about 198%, about 140% to about 198% or about 150% to about 198% the number of platelets required to prepare one platelet unit.

In some embodiments, the at least one selected platelet preparation from the inventory of apheresis-collected platelet preparations comprises greater than the number of platelets required to prepare two platelet units, but less than the number of platelets required to prepare three platelet units. In some embodiments, greater than the number of platelets required to prepare two platelet units, but less than the number of platelets required to prepare three platelet units is about 1.1-fold, 1.2-fold, 1.3-fold, 1.4-fold, 1.5-fold, 1.6-fold, 1.7-fold, 1.8-fold, 1.9-fold, 1.95-fold or about 1.98-fold the number of platelets required to prepare two platelet units. In some embodiments, greater than the number of platelets required to prepare two platelet units, but less than the number of platelets required to prepare three platelet units is about 1. 1-fold to about 1.98-fold, about 1.15-fold to about 1.98-fold, about 1.2-fold to about 1.98-fold, about 1.25-fold to about 1.98-fold, about 1.3-fold to about 1.98-fold, about 1.35-fold to about 1.98-fold, about 1.4-fold to about 1.98-fold or about 1.5-fold to about 1.98-fold the number of platelets required to prepare two platelet units. In some embodiments, greater than the number of platelets required to prepare two platelet units, but less than the number of platelets required to prepare three platelet units is about 105%, 110%, 115%, 120%, 125%, 130%, 135%, 140%, 145%, 150%, 155%, 160%, 165%, 170%, 175%, 180%, 185%, 190% or about 195% the number of platelets required to prepare two platelet units. In some embodiments, greater than the number of platelets required to prepare two platelet units, but less than the number of platelets required to prepare three platelet units is about 110% to about 198%, about 115% to about 198%, about 120% to about 198%, about 125% to about 198%, about 130% to about 198%, about 135% to about 198%, about 140% to about 198% or about 150% to about 198% the number of platelets required to prepare two platelet units. In some embodiments, the at least one selected platelet preparation comprises at least about 225%, 230%, 235%, 240%, 245%, 250%, 255%, 260%, 265%, 270%, 275%, 280%, 285%, 290% or about 295% of the number of platelets required to prepare one platelet unit, but less than the number of platelets required to prepare three platelet units. In some embodiments, the at least one selected platelet preparation comprises about 225% to about 298%, about 230% to about 298%, about 235% to about 298%, about 240% to about 298%, about 245% to about 298%, or about 250% to about 298% of the number of platelets required to prepare one platelet unit.

In some embodiments, the at least one selected platelet preparation from the inventory of apheresis-collected platelet preparations comprises greater than the number of platelets required to prepare three platelet units, but less than the number of platelets required to prepare four platelet units. In some embodiments, greater than the number of platelets required to prepare three platelet units, but less than the number of platelets required to prepare four platelet units is about 1.1-fold, 1.2-fold, 1.3-fold, 1.4-fold, 1.5-fold, 1.6-fold, 1.7-fold, 1.8-fold, 1.9-fold, 1.95-fold or about 1.98-fold the number of platelets required to prepare three platelet units. In some embodiments, greater than the number of platelets required to prepare three platelet units, but less than the number of platelets required to prepare four platelet units is about 1.1-fold to about 1.98-fold, about 1.15-fold to about 1.98-fold, about 1.2-fold to about 1.98-fold, about 1.25-fold to about 1.98-fold, about 1.3-fold to about 1.98-fold, about 1.35-fold to about 1.98-fold, about 1.4-fold to about 1.98-fold or about 1.5-fold to about 1.98-fold the number of platelets required to prepare three platelet units. In some embodiments, greater than the number of platelets required to prepare three platelet units, but less than the number of platelets required to prepare four platelet units is about 105%, 110%, 115%, 120%, 125%, 130%, 135%, 140%, 145%, 150%, 155%, 160%, 165%, 170%, 175%, 180%, 185%, 190% or about 195% the number of platelets required to prepare three platelet units. In some embodiments, greater than the number of platelets required to prepare three platelet units, but less than the number of platelets required to prepare four platelet units is about 110% to about 198%, about 115% to about 198%, about 120% to about 198%, about 125% to about 198%, about 130% to about 198%, about 135% to about 198%, about 140% to about 198% or about 150% to about 198% the number of platelets required to prepare three platelet units. In some embodiments, the at least one selected platelet preparation comprises at least about 325%, 330%, 335%, 340%, 345%, 350%, 355%, 360%, 365%, 370%, 375%, 380%, 385%, 390% or about 395% of the number of platelets required to prepare one platelet unit, but less than the number of platelets required to prepare four platelet units. In some embodiments, the at least one selected platelet preparation comprises about 325% to about 398%, about 330% to about 398%, about 335% to about 398%, about 340% to about 398%, about 345% to about 398%, or about 350% to about 398% of the number of platelets required to prepare one platelet unit

In some embodiments, the new platelet donation from the available donor comprises less than the number (*e.g.,* less than the minimum number, less than the minimum quantity) of platelets required to prepare one platelet unit. In some embodiments, the new platelet donation from the available donor comprises an amount (*e.g.,* number) of platelets that is less than about 99%, 98%, 95%, 90%, 85%, 80%, 75%, 70%, 65%, 60%, 55%, 50%, 45%, 40%, 35%, 30% or less than about 25% of the number of platelets required to prepare one platelet unit. In some embodiments, the new platelet donation from the available donor comprises an amount of platelets that is at least 10% of the number of platelets required to prepare one platelet unit. In some embodiments, the new platelet donation from the available donor comprises an amount of platelets that is about 10% to about 98%, about 15% to about 98%, about 20% to about 98%, about 25% to about 98%, about 30% to about 98%, about 35% to about 98%, about 40% to about 98% or about 50% to about 98% of the number of platelets required to prepare one platelet unit. In some embodiments, the new platelet donation from the available donor comprises an amount of platelets that is about 98%, 95%, 90%, 85%, 80%, 75%, 70%, 65%, 60%, 55%, 50%, 45%, 40%, 35%, 30% or about 25% of the number of platelets required to prepare one platelet unit. In some embodiments, collecting the new platelet donation comprises targeting the platelet collection to yield a donation comprising less than the number of platelets required to prepare one platelet unit. In some embodiments, collecting the new platelet donation comprises targeting the platelet collection to yield a donation comprising less than about 99%, 98%, 95%, 90%, 85%, 80%, 75%, 70%, 65%, 60%, 55%, 50%, 45%, 40%, 35%, 30% or less than about 25% number of platelets required to prepare one platelet unit. In some embodiments, collecting the new platelet donation from the available donor comprises targeting the platelet collection to yield a donation comprising about 10% to about 98%, about 15% to about 98%, about 20% to about 98%, about 25% to about 98%, about 30% to about 98%, about 35% to about 98%, about 40% to about 98% or about 50% to about 98% of the number of platelets required to prepare one platelet unit. In some embodiments, collecting the new platelet donation from the available donor comprises targeting the platelet collection to yield a donation comprising a sufficient number of platelets to prepare one, to prepare two, to prepare three, or to prepare four platelet units after combining the at least one selected platelet preparation and the new platelet donation and separating the resulting pooled platelet preparation into a plurality of platelet units. In some embodiments, the new platelet donation from the available donor comprises a sufficient number of platelets that, when combined with the at least one selected platelet preparation, yields a pooled platelet preparation comprising a sufficient number of platelets to separate into one, into two, into three, or into four platelet units. In some embodiments, collecting the new platelet donation from the available donor comprises collecting a sufficient number of platelets to prepare one, to prepare two, to prepare three, or to prepare four platelet units from the pooled preparation.

In some embodiments, the new platelet donation from the available donor comprises greater than the number of platelets required to prepare one platelet unit, but less than the number of platelets required to prepare two platelet units. In some embodiments, greater than the number of platelets required to prepare one platelet unit, but less than the number of platelets required to prepare two platelet units is about 1.1-fold, 1.2-fold, 1.3-fold, 1.4-fold, 1.5-fold, 1.6-fold, 1.7-fold, 1.8-fold, 1.9-fold, 1.95-fold or about 1.98-fold the number of platelets required to prepare one platelet unit. In some embodiments, greater than the number of platelets required to prepare one platelet unit, but less than the number of platelets required to prepare two platelet units is about 1.1-fold to about 1.98-fold, about 1.15-fold to about 1.98-fold, about 1.2-fold to about 1.98-fold, about 1.25-fold to about 1.98-fold, about 1.3-fold to about 1.98-fold, about 1.35-fold to about 1.98-fold, about 1.4-fold to about 1.98-fold or about 1.5-fold to about 1.98-fold the number of platelets required to prepare one platelet unit. In some embodiments, greater than the number of platelets required to prepare one platelet unit, but less than the number of platelets required to prepare two platelet units is about 105%, 110%, 115%, 120%, 125%, 130%, 135%, 140%, 145%, 150%, 155%, 160%, 165%, 170%, 175%, 180%, 185%, 190% or about 195% the number of platelets required to prepare one platelet unit. In some embodiments, greater than the number of platelets required to prepare one platelet unit, but less than the number of platelets required to prepare two platelet units is about 110% to about 198%, about 115% to about 198%, about 120% to about 198%, about 125% to about 198%, about 130% to about 198%, about 135% to about 198%, about 140% to about 198% or about 150% to about 198% the number of platelets required to prepare one platelet unit. In some embodiments, collecting the new platelet donation from the available donor comprises targeting the platelet collection to yield a donation comprising at least about 125%, 130%, 135%, 140%, 145%, 150%, 155%, 160%, 165%, 170%, 175%, 180%, 185%, 190% or about 195% of the number of platelets required to prepare one platelet unit, but less than the number of platelets required to prepare two platelet units. In some embodiments, collecting the new platelet donation from the available comprises targeting the platelet collection to yield a donation comprising about 110% to about 198%, about 115% to about 198%, about 120% to about 198%, about 125% to about 198%, about 130% to about 198%, about 135% to about 198%, about 140% to about 198%, about 145% to about 198%, or about 150% to about 198% the number of platelets required to prepare one platelet unit. In some embodiments, collecting the new platelet donation from the available donor comprises targeting the platelet collection to yield a donation comprising a sufficient number of platelets to prepare two, to prepare three, to prepare four, or to prepare five platelet units after combining the at least one selected platelet preparation and the new platelet donation and separating the resulting pooled platelet preparation into a plurality of platelet units. In some embodiments, the new platelet donation from the available donor comprises a sufficient number of platelets that, when combined with the at least one selected platelet preparation, yields a pooled platelet preparation comprising a sufficient number of platelets to separate into two, into three, into four, or into five platelet units. In some embodiments, collecting the new platelet donation from the available donor comprises collecting a sufficient number of platelets to prepare two, to prepare three, to prepare four, or to prepare five platelet units from the pooled preparation.

In some embodiments, the new platelet donation from the available donor comprises greater than the number of platelets required to prepare two platelet units, but less than the number of platelets required to prepare three platelet units. In some embodiments, greater than the number of platelets required to prepare two platelet units, but less than the number of platelets required to prepare three platelet units is about 1. 1-fold, 1.2-fold, 1.3-fold, 1.4-fold, 1.5-fold, 1.6-fold, 1.7-fold, 1.8-fold, 1.9-fold, 1.95-fold or about 1.98-fold the number of platelets required to prepare two platelet units. In some embodiments, greater than the number of platelets required to prepare two platelet units, but less than the number of platelets required to prepare three platelet units is about 1.1-fold to about 1.98-fold, about 1.15-fold to about 1.98-fold, about 1.2-fold to about 1.98-fold, about 1.25-fold to about 1.98-fold, about 1.3-fold to about 1.98-fold, about 1.35-fold to about 1.98-fold, about 1.4-fold to about 1.98-fold or about 1.5-fold to about 1.98-fold the number of platelets required to prepare two platelet units. In some embodiments, greater than the number of platelets required to prepare two platelet units, but less than the number of platelets required to prepare three platelet units is about 105%, 110%, 115%, 120%, 125%, 130%, 135%, 140%, 145%, 150%, 155%, 160%, 165%, 170%, 175%, 180%, 185%, 190% or about 195% the number of platelets required to prepare two platelet units. In some embodiments, greater than the number of platelets required to prepare two platelet units, but less than the number of platelets required to prepare three platelet units is about 110% to about 198%, about 115% to about 198%, about 120% to about 198%, about 125% to about 198%, about 130% to about 198%, about 135% to about 198%, about 140% to about 198% or about 150% to about 198% the number of platelets required to prepare two platelet units. In some embodiments, the new platelet donation comprises at least about 225%, 230%, 235%, 240%, 245%, 250%, 255%, 260%, 265%, 270%, 275%, 280%, 285%, 290% or about 295% of the number of platelets required to prepare one platelet unit, but less than the number of platelets required to prepare three platelet units. In some embodiments, the new platelet donation comprises about 210% to about 298%, about 215% to about 298%, about 220% to about 298%, about 225% to about 298%, about 230% to about 298%, about 235% to about 298%, about 240% to about 298%, about 245% to about 298%, or about 250% to about 298% of the number of platelets required to prepare one platelet unit. In some embodiments, collecting the new platelet donation from the available donor comprises targeting the platelet collection to yield a donation comprising at least about 225%, 230%, 235%, 240%, 245%, 250%, 255%, 260%, 265%, 270%, 275%, 280%, 285%, 290% or about 295% of the number of platelets required to prepare one platelet unit, but less than the number of platelets required to prepare three platelet units. In some embodiments, collecting the new platelet donation from the available donor comprises targeting the platelet collection to yield a donation comprising about 210% to about 298%, about 215% to about 298%, about 220% to about 298%, about 225% to about 298%, about 230% to about 298%, about 235% to about 298%, about 240% to about 298%, about 245% to about 298%, or about 250% to about 298% the number of platelets required to prepare one platelet unit. In some embodiments, collecting the new platelet donation from the available donor comprises targeting the platelet collection to yield a donation comprising a sufficient number of platelets to prepare three, to prepare four, to prepare five, or to prepare six platelet units after combining the at least one selected platelet preparation and the new platelet donation and separating the resulting pooled platelet preparation into a plurality of platelet units. In some embodiments, the new platelet donation from the available donor comprises a sufficient number of platelets that, when combined with the at least one selected platelet preparation, yields a pooled platelet preparation comprising a sufficient number of platelets to separate into three, into four, into five, or into six platelet units. In some embodiments, collecting the new platelet donation from the available donor comprises collecting a sufficient number of platelets to prepare three, to prepare four, to prepare five, or to prepare six platelet units from the pooled preparation.

In some embodiments, the new platelet donation from the available donor comprises greater than the number of platelets required to prepare three platelet units, but less than the number of platelets required to prepare four platelet units. In some embodiments, greater than the number of platelets required to prepare three platelet units, but less than the number of platelets required to prepare four platelet units is about 1. 1-fold, 1.2-fold, 1.3-fold, 1.4-fold, 1.5-fold, 1.6-fold, 1.7-fold, 1.8-fold, 1.9-fold, 1.95-fold or about 1.98-fold the number of platelets required to prepare three platelet units. In some embodiments, greater than the number of platelets required to prepare three platelet units, but less than the number of platelets required to prepare four platelet units is about 1. 1-fold to about 1.98-fold, about 1.15-fold to about 1.98-fold, about 1.2-fold to about 1.98-fold, about 1.25-fold to about 1.98-fold, about 1.3-fold to about 1.98-fold, about 1.35-fold to about 1.98-fold, about 1.4-fold to about 1.98-fold or about 1.5-fold to about 1.98-fold the number of platelets required to prepare three platelet units. In some embodiments, greater than the number of platelets required to prepare three platelet units, but less than the number of platelets required to prepare four platelet units is about 105%, 110%, 115%, 120%, 125%, 130%, 135%, 140%, 145%, 150%, 155%, 160%, 165%, 170%, 175%, 180%, 185%, 190% or about 195% the number of platelets required to prepare three platelet units. In some embodiments, greater than the number of platelets required to prepare three platelet units, but less than the number of platelets required to prepare four platelet units is about 110% to about 198%, about 115% to about 198%, about 120% to about 198%, about 125% to about 198%, about 130% to about 198%, about 135% to about 198%, about 140% to about 198% or about 150% to about 198% the number of platelets required to prepare three platelet units. In some embodiments, the new platelet donation from the available donor comprises at least about 325%, 330%, 335%, 340%, 345%, 350%, 355%, 360%, 365%, 370%, 375%, 380%, 385%, 390% or about 395% of the number of platelets required to prepare one platelet unit, but less than the number of platelets required to prepare three platelet units. In some embodiments, the new platelet donation from the available donor comprises about 310% to about 398%, about 315% to about 398%, about 320% to about 398%, about 325% to about 398%, about 330% to about 398%, about 335% to about 398%, about 340% to about 398%, about 345% to about 398%, or about 350% to about 398% of the number of platelets required to prepare one platelet unit. In some embodiments, collecting the new platelet donation from the available donor comprises targeting the platelet collection to yield a donation comprising at least about 325%, 330%, 335%, 340%, 345%, 350%, 355%, 360%, 365%, 370%, 375%, 380%, 385%, 390% or about 395% of the number of platelets required to prepare one platelet unit, but less than the number of platelets required to prepare three platelet units. In some embodiments, collecting the new platelet donation comprises targeting the platelet collection to yield a donation comprising about 310% to about 398%, about 315% to about 398%, about 320% to about 398%, about 325% to about 398%, about 330% to about 398%, about 335% to about 398%, about 340% to about 398%, about 345% to about 398%, or about 350% to about 398% the number of platelets required to prepare one platelet unit. In some embodiments, collecting the new platelet donation from the available donor comprises targeting the platelet collection to yield a donation comprising a sufficient number of platelets to prepare four, to prepare five, to prepare six, or to prepare seven platelet units after combining the at least one selected platelet preparation and the new platelet donation and separating the resulting pooled platelet preparation into a plurality of platelet units. In some embodiments, the new platelet donation from the available donor comprises a sufficient number of platelets that, when combined with the at least one selected platelet preparation, yields a pooled platelet preparation comprising a sufficient number of platelets to separate into four, into five, into six, or into seven platelet units. In some embodiments, collecting the new platelet donation from the available donor comprises collecting a sufficient number of platelets to prepare four, to prepare five, to prepare six, or to prepare seven platelet units from the pooled preparation.

In some embodiments, the at least one selected platelet preparation from the inventory of apheresis-collected platelet preparations comprises less than the number of platelets required to prepare one platelet unit and the new platelet donation from the available donor comprises greater than the number of platelets required to prepare one platelet unit, but less than the number of platelets required to prepare two platelet units. In some embodiments, the at least one selected platelet preparation from a first donor comprises less than the number of platelets required to prepare one platelet unit and the new platelet donation from the available donor comprises greater than the number of platelets required to prepare two platelet units, but less than the number of platelets required to prepare three platelet units. In some embodiments, the at least one selected platelet preparation from the inventory of apheresis-collected platelet preparations comprises less than the number of platelets required to prepare one platelet unit and the new platelet donation from the available donor comprises greater than the number of platelets required to prepare three platelet units, but less than the number of platelets required to prepare four platelet units. In some embodiments, the pooled platelet preparation comprises a sufficient number of platelets to prepare two, to prepare three, or to prepare four platelet units. In some embodiments, the plurality of platelet units comprises two platelet units, three platelet units, or four platelet units.

In some embodiments, the new platelet donation from the available donor comprises less than the number of platelets required to prepare one platelet unit and the at least one selected platelet preparation from the inventory of apheresis-collected platelet preparations comprises greater than the number of platelets required to prepare one platelet unit, but less than the number of platelets required to prepare two platelet units. In some embodiments, the new platelet donation from the available donor comprises less than the number of platelets required to prepare one platelet unit and the at least one selected platelet preparation from the inventory of apheresis-collected platelet preparations comprises greater than the number of platelets required to prepare two platelet units, but less than the number of platelets required to prepare three platelet units. In some embodiments, the new platelet donation from the available donor comprises less than the number of platelets required to prepare one platelet unit and the at least one selected platelet preparation from the inventory of apheresis-collected platelet preparations comprises greater than the number of platelets required to prepare three platelet units, but less than the number of platelets required to prepare four platelet units. In some embodiments, the pooled platelet preparation comprises a sufficient number of platelets to prepare two, to prepare three, or to prepare four platelet units. In some embodiments, the plurality of platelet units comprises two platelet units, three platelet units, or four platelet units.

In some embodiments, the at least one selected platelet preparation from the inventory of apheresis-collected platelet preparations comprises greater than the number of platelets required to prepare one platelet unit, but less than the number of platelets required to prepare two platelet units, and the new platelet donation from the available donor comprises greater than the number of platelets required to prepare one platelet unit, but less than the number of platelets required to prepare two platelet units. In some embodiments, the at least one selected platelet preparation from the inventory of apheresis-collected platelet preparations comprises greater than the number of platelets required to prepare one platelet unit, but less than the number of platelets required to prepare two platelet units, and the new platelet donation comprises greater than the number of platelets required to prepare two platelet units, but less than the number of platelets required to prepare three platelet units. In some embodiments, the at least one selected platelet preparation from the inventory of apheresis-collected platelet preparations comprises greater than the number of platelets required to prepare one platelet unit, but less than the number of platelets required to prepare two platelet units, and the new platelet donation comprises greater than the number of platelets required to prepare three platelet units, but less than the number of platelets required to prepare four platelet units. In some embodiments, the pooled platelet preparation comprises a sufficient number of platelets to prepare three, to prepare four, or to prepare five platelet units. In some embodiments, the plurality of platelet units comprises three platelet units, four platelet units, or five platelet units.

In some embodiments, the at least one selected platelet preparation from the inventory of apheresis-collected platelet preparations comprises greater than the number of platelets required to prepare two platelet units, but less than the number of platelets required to prepare three platelet units, and the new platelet donation from the available donor comprises greater than the number of platelets required to prepare two platelet units, but less than the number of platelets required to prepare three platelet units. In some embodiments, the at least one selected platelet preparation from the inventory of apheresis-collected platelet preparations comprises greater than the number of platelets required to prepare two platelet units, but less than the number of platelets required to prepare three platelet units, and the new platelet donation comprises greater than the number of platelets required to prepare three platelet units, but less than the number of platelets required to prepare four platelet units. In some embodiments, the at least one selected platelet preparation from the inventory of apheresis-collected platelet preparations comprises greater than the number of platelets required to prepare three platelet units, but less than the number of platelets required to prepare four platelet units, and the new platelet donation from the available donor comprises greater than the number of platelets required to prepare three platelet units, but less than the number of platelets required to prepare four platelet units. In some embodiments, the pooled platelet preparation comprises a sufficient number of platelets to prepare five, to prepare six, or to prepare seven platelet units. In some embodiments, the plurality of platelet units comprises five platelet units, six platelet units, or seven platelet units.

In some embodiments, the method further comprises identifying a second available donor suitable for donating platelets by apheresis, collecting a second new platelet donation by apheresis from the second available donor, combining the second new platelet donation with the at least one selected platelet preparation from the inventory of apheresis-collected platelet preparations and the new platelet donation from the available donor to yield the pooled platelet preparation, and separating the pooled platelet preparation into a plurality of platelet units each in an individual container, wherein the plurality of platelet units comprises a greater number of platelet units than the total number of platelet units that can be prepared from the at least one selected platelet preparation, the new platelet donation and the second new platelet donation without combining the at least one selected platelet preparation, the new platelet donation and the second new platelet donation. In some embodiments, the method further comprises subjecting the at least one selected platelet preparation from the inventory of apheresis-collected platelet preparations, the new platelet donation from the available donor, and/or the second new platelet donation from the second available donor to a pathogen inactivation process. In some embodiments, the method further comprises subjecting each of the at least one selected platelet preparation and the new platelet donation a pathogen inactivation process. In some embodiments, the method further comprises subjecting each of the at least one selected platelet preparation, the new platelet donation and the second new platelet donation a pathogen inactivation process. In some embodiments, the method further comprises subjecting the pooled platelet preparation a pathogen inactivation process. In some embodiments, subjecting the at least one selected platelet preparation, new platelet donation, second new platelet donation and/or pooled platelet preparation to a pathogen inactivation process comprises treating the at least one selected platelet preparation, new platelet donation, second new platelet donation and/or pooled platelet preparation with a pathogen inactivating compound. In some embodiments, the pathogen inactivating compound is a photoactive pathogen inactivating compound selected from the group consisting of a psoralen, an isoalloxazine, an alloxazine, a phthalocyanine, a phenothiazine, a porphyrin, and merocyanine 540. In some embodiments, the pathogen inactivating compound is a psoralen. In some embodiments, the pathogen inactivating compound is amotosalen.

In some embodiments, the method further comprises collecting a third platelet donation by apheresis from a third available donor, combining the third platelet donation with the at least one selected platelet preparation from the inventory of apheresis-collected platelet preparations and the new platelet donation to yield a pooled platelet preparation, and separating the pooled platelet preparation into a plurality of platelet units each in an individual container, wherein the plurality of platelet units comprises a greater number of platelet units than the total number of platelet units that can be prepared from the at least one selected platelet preparation, the new platelet donation and the third platelet donation without combining the at least one selected platelet preparation, the new platelet donation and the third platelet donation. In some embodiments, the method further comprises subjecting the at least one selected platelet preparation from the inventory of apheresis-collected platelet preparations, the new platelet donation, and/or the third platelet donation to a pathogen inactivation process. In some embodiments, the method further comprises subjecting each of the at least one selected platelet preparation from the inventory of apheresis-collected platelet preparationsand the new platelet donation to a pathogen inactivation process. In some embodiments, the method further comprises subjecting each of the at least one selected platelet preparation, the new platelet donation and the third platelet donation to a pathogen inactivation process. In some embodiments, the method further comprises subjecting the pooled platelet preparation to a pathogen inactivation process. In some embodiments, subjecting the at least one selected platelet preparation, new platelet donation, third platelet donation and/or pooled platelet preparation to a pathogen inactivation process comprises treating the at least one selected platelet preparation, new platelet donation, third platelet donation and/or pooled platelet preparation with a pathogen inactivating compound. In some embodiments, the pathogen inactivating compound is a photoactive pathogen inactivating compound selected from the group consisting of a psoralen, an isoalloxazine, an alloxazine, a phthalocyanine, a phenothiazine, a porphyrin, and merocyanine 540. In some embodiments, the pathogen inactivating compound is a psoralen. In some embodiments, the pathogen inactivating compound is amotosalen.

In some of the aforementioned embodiments, the new platelet donation from the available donor is collected less than about (*e.g.,* within) 24 hours, 22 hours, 20 hours, 16 hours, 12 hours, 10 hours, 8 hours, 6 hours, 5 hours, 4 hours, 3 hours, 2 hours or less than about 1 hour from the time the at least one selected platelet preparation is collected. In some embodiments, the new platelet donation from the available donor is collected within about 7 days, within about 6 days, within about 5 days, within about 4 days, within about 3 days, within about 2 days, or within about 1 day from the time the at least one selected platelet preparation is collected. In some embodiments, the new platelet donation from the available donor is collected about the same time (*e.g.,* overlapping collection time) as the at least one selected platelet preparation from the inventory of apheresis-collected platelet preparations. In some embodiments, the new platelet donation from the available donor is collected within 20 hours from the time the at least one selected platelet preparation is collected. In some embodiments, the new platelet donation from the available donor is collected within 12 hours from the time the at least one selected platelet preparation is collected. In some embodiments, the new platelet donation from the available donor is collected within 6 hours from the time the at least one selected platelet preparation is collected. In some of the aforementioned embodiments, the second new platelet donation (or third platelet donation) is collected less than about (*e.g.,* within) 24 hours, 22 hours, 20 hours, 16 hours, 12 hours, 10 hours, 8 hours, 6 hours, 5 hours, 4 hours, 3 hours, 2 hours or less than about 1 hour from the time the at least one selected platelet preparation is collected. In some embodiments, the second new platelet donation is collected within about 7 days, within about 6 days, within about 5 days, within about 4 days, within about 3 days, within about 2 days, or within about 1 day from the time the at least one selected platelet preparation from the inventory of apheresis-collected platelet preparations is collected. In some embodiments, the second new platelet donation (or third platelet donation) is collected about the same time (*e.g.,* overlapping collection time) as the at least one selected platelet preparation from the inventory of apheresis-collected platelet preparations. In some embodiments, the second new platelet donation (third platelet donation) is collected within 20 hours from the time the at least one selected platelet preparation is collected. In some embodiments, the second new platelet donation (or third platelet donation) is collected within 12 hours from the time the at least one selected platelet preparation is collected. In some embodiments, the second new platelet donation (or third platelet donation) is collected within 6 hours from the time the at least one selected platelet preparation from the inventory of apheresis-collected platelet preparationsis collected. In some embodiments, the second new platelet donation is collected within about 24 hours, 22 hours, 20 hours, 16 hours, 12 hours, 10 hours, 8 hours, 6 hours, 5 hours, 4 hours, 3 hours, 2 hours or about 1 hour from the time the at least one selected platelet preparation from the inventory of apheresis-collected platelet preparationsand the new platelet donation are collected. In some embodiments, the at least one selected platelet preparation for the inventory of apheresis-collected platelet preparations, the new platelet donation and the second new platelet donation (or third platelet donation) are collected within a period (*e.g.,* the same period) of about 24 hours, 22 hours, 20 hours, 16 hours, 12 hours, 10 hours, 8 hours, 6 hours, 5 hours, 4 hours, 3 hours, 2 hours or about 1 hour. In some embodiments, the at least one selected platelet preparation for the inventory of apheresis-collected platelet preparations, the new platelet donation and the second new platelet donation (or third platelet donation) are collected within about 7 days, within about 6 days, within about 5 days, within about 4 days, within about 3 days, within about 2 days, within about 1 day, within about 72 hours, within about 48 hours, within about 36 hours, within about 24 hours, within about 20 hours, within about 12 hours or within about 6 hours of each other. In some embodiments, the at least one selected platelet preparation for the inventory of apheresis-collected platelet preparations, the new platelet donation and the second new platelet donation (or third platelet donation) are collected at about the same time (*e.g.,* overlapping collection times).

In some of the aforementioned embodiments, the pooled platelet preparation comprises platelets (*e.g.,* the at least one selected platelet preparation from the inventory of apheresis-collected platelet preparations, the new platelet donation from an available donor, the second new platelet donation from an available donor, the third platelet donation from an available donor) only from donors of the same ABO blood type.

In some of the aforementioned embodiments, the platelet units (*e.g.,* each of the platelet units) in the plurality of platelet units comprise at least about 2.0×10¹¹ platelets, at least about 2.2×10¹¹ platelets, at least about 2.4×10¹¹ platelets, at least about 2.5×10¹¹ platelets, at least about 2.6×10¹¹ platelets, at least about 2.7×10¹¹ platelets, at least about 2.8×10¹¹ platelets, at least about 2.9×10¹¹ platelets or at least about 3.0×10¹¹ platelets. In some embodiments, the platelet units each comprise at least about 3.0×10¹¹ platelets, and the pooled platelet preparation comprises at least about 6.2×10¹¹ platelets. In some embodiments, the platelet units each comprise at least about 3.0×10¹¹ platelets, and the pooled platelet preparation comprises at least about 9.3×10¹¹ platelets. In some embodiments, the platelet units each comprise at least about 3.0×10¹¹ platelets, and the pooled platelet preparation comprises at least about 12.4×10¹¹ platelets. In some embodiments, the platelet units each comprise at least about 3.0×10¹¹ platelets, and the pooled platelet preparation comprises at least about 15.5×10¹¹ platelets.

In the aforementioned embodiments, the platelet units *(e.g.,* each platelet unit) in the plurality of platelet units comprise a therapeutic dose (*e.g.,* therapeutic dosage unit) of platelets suitable for infusion into a human subject (*e.g.,* subject in need of a platelet infusion). In some embodiments, the therapeutic dose comprises a minimum number (*e.g.,* at least a minimum number) of platelets as defined by criteria (*e.g.,* acceptance criteria) of a governmental agency, regulatory agency, institution and/or accrediting organization (*e.g.,* governmental agency, regulatory agency, institution and/or accrediting organization for donated blood products (e.g., donated platelets)). In some embodiments, the platelet units are prepared in the country of the governmental agency, regulatory agency, institution and/or accrediting organization defining the criteria of a therapeutic dose of platelets. In some embodiments, at least about 75%, at least about 80%, at least about 85%, at least about 90%, at least about 95%, at least about 98%, at least about 99% or more of the platelet units in the plurality of platelet units comprise the minimum number of platelets of a therapeutic dose. In some embodiments, each of the platelet units in the plurality of platelet units comprises the minimum number of platelets of a therapeutic dose. In some embodiments, the minimum number of platelets in a therapeutic dose is at least about 2.0×10¹¹ platelets, at least about 2.2×10¹¹, platelets, at least about 2.4×10¹¹ platelets, at least about 2.5×10¹¹ platelets, at least about 2.6×10¹¹ platelets, at least about 2.7×10¹¹ platelets, at least about 2.8×10¹¹ platelets, at least about 2.9×10¹¹ platelets or at least about 3.0×10¹¹ platelets.

In some of the aforementioned embodiments, targeting the platelet collection comprises determining an optimal platelet collection. In some embodiments, an optimal platelet collection is a maximum platelet collection (*e.g.,* maximum that can be safely collected, maximum according to regulatory and/or industry standards or regulations) that can be collected from a donor. In some embodiments, targeting the platelet collection comprises providing an input (*e.g.,* programming) to an apheresis system. In some embodiments, targeting the platelet collection comprises providing a donation volume to an apheresis system. In some embodiments, targeting the platelet collection comprises providing a duration time for the platelet collection to an apheresis system. In some embodiments, targeting the platelet collection comprises providing a donation volume and/or duration time for the platelet collection that factors in (*e.g.,* accounts for) variation (*e.g.,* distribution) in actual platelet collection yields as compared to targeted collection yields. In some embodiments, the at least one selected platelet preparation from the inventory of apheresis-collected platelet preparations and the new platelet donation from an available donor are combined in a single container of suitable size for the volume of the pooled platelet preparation. In some embodiments, the at least one selected platelet preparation and the new platelet donation are of suitable volume for treating with a pathogen inactivating compound. In some embodiments, the at least one selected platelet preparation and the new platelet donation are of suitable volume and platelet number (*e.g.,* platelet quantity) for treating with a pathogen inactivating compound. In some embodiments, the pooled platelet preparation is of suitable volume for treating with a pathogen inactivating compound. In some embodiments, the pooled platelet preparation is of suitable volume and platelet number for treating with a pathogen inactivating compound.

In some embodiments, the method for collecting a platelet donation by apheresis from a donor further comprises a) determining a target volume of the new platelet donation from the available donor, wherein the target volume optimizes the number of platelet units that can be prepared from a combination comprising the new platelet donation and the one or more selected platelet preparations from the inventory of apheresis-collected platelet preparations; and b) collecting the target volume of the new platelet donation by apheresis from the available donor, c) combining the one or more selected platelet preparations from the inventory of apheresis-collected platelet preparations and the new platelet donation from the available donor to yield a pooled platelet preparation; and d) separating the pooled platelet preparation into a plurality of platelet units (*e.g.,* therapeutic dosage units) each in an individual container, wherein each of the platelet units in the plurality of platelet units comprises a therapeutic dose (*e.g.,* therapeutic dose of platelets) suitable for infusion into a human subject in need thereof, and wherein the plurality of platelet units comprises a greater number of platelet units than the total number of platelet units that can be prepared from the one or more selected platelet preparations from the inventory of apheresis-collected platelet preparations and the new platelet donation from the available donor without combining the one or more selected platelet preparations and the new platelet donation.

In some embodiments, each of the one or more selected platelet preparations comprises less than the number of platelets required to prepare one therapeutic dose. In some embodiments, each of the one or more selected platelet preparations comprises greater than the number of platelets required to prepare one therapeutic dose, but less than the number of platelets required to prepare two therapeutic doses. In some embodiments, each of the one or more selected platelet preparations comprises greater than the number of platelets required to prepare two therapeutic doses, but less than the number of platelets required to prepare three therapeutic doses. In some embodiments, each of the one or more selected platelet preparations comprises greater than the number of platelets required to prepare three therapeutic doses, but less than the number of platelets required to prepare four therapeutic doses.

In some embodiments, the new platelet donation comprises less than the number of platelets required to prepare one therapeutic dose. In some embodiments, the new platelet donation comprises greater than the number of platelets required to prepare one therapeutic dose, but less than the number of platelets required to prepare two therapeutic doses. In some embodiments, the new platelet donation comprises greater than the number of platelets required to prepare two therapeutic doses, but less than the number of platelets required to prepare three therapeutic doses. In some embodiments, the new platelet donation comprises greater than the number of platelets required to prepare three therapeutic doses, but less than the number of platelets required to prepare four therapeutic doses.

In some embodiments, the one or more selected platelet preparations comprises less than the number of platelets required to prepare one platelet unit. In some embodiments, the target volume of the new platelet donation is a volume comprising a sufficient number of platelets to prepare two, to prepare three, or to prepare four platelet units after combining the one or more selected platelet preparations and the new platelet donation and separating the resulting pooled platelet preparation into a plurality of platelet units. In some embodiments, the new platelet donation comprises a sufficient number of platelets that, when combined with the one or more selected platelet preparation, yields a pooled platelet preparation comprising a sufficient number of platelets to separate into two, into three, or into four platelet units. In some embodiments, collecting the new platelet donation from the donor comprises collecting a sufficient number of platelets to prepare two, to prepare three, or to prepare four platelet units from the pooled preparation.

In some embodiments, the one or more selected platelet preparations comprises greater than the number of platelets required to prepare one platelet unit, but less than the number of platelets required to prepare two platelet units. In some embodiments, the one or more selected platelet preparations comprises at least about 125%, at least about 130%, at least about 135%, at least about 140%, at least about 145%, or at least about at least about 150% of the number of platelets required to prepare one platelet unit, but less than the number of platelets required to prepare two platelet units. In some embodiments, the target volume of the new platelet donation is a volume comprising a sufficient number of platelets to prepare two, to prepare three, to prepare four, or to prepare five platelet units after combining the one or more selected platelet preparations and the new platelet donation and separating the resulting pooled platelet preparation into a plurality of platelet units. In some embodiments, the new platelet donation comprises a sufficient number of platelets that, when combined with the one or more selected platelet preparation, yields a pooled platelet preparation comprising a sufficient number of platelets to separate into two, into three, into four, or into five platelet units. In some embodiments, collecting the new platelet donation from the donor comprises collecting a sufficient number of platelets to prepare two, to prepare three, to prepare four, or to prepare five platelet units from the pooled preparation.

In some embodiments, the one or more selected platelet preparations comprises greater than the number of platelets required to prepare two platelet units, but less than the number of platelets required to prepare three platelet units. In some embodiments, the one or more selected platelet preparations comprises at least about 225%, at least about 230%, at least about 235%, at least about 240%, at least about 245%, or at least about at least about 250% of the number of platelets required to prepare one platelet unit, but less than the number of platelets required to prepare three platelet units. In some embodiments, the target volume of the new platelet donation is a volume comprising a sufficient number of platelets to prepare three, to prepare four, to prepare five, or to prepare six platelet units after combining the one or more selected platelet preparations and the new platelet donation and separating the resulting pooled platelet preparation into a plurality of platelet units. In some embodiments, the new platelet donation comprises a sufficient number of platelets that, when combined with the one or more selected platelet preparation, yields a pooled platelet preparation comprising a sufficient number of platelets to separate into three, into four, into five, or into six platelet units. In some embodiments, collecting the new platelet donation from the donor comprises collecting a sufficient number of platelets to prepare three, to prepare four, to prepare five, or to prepare six platelet units from the pooled preparation.

In some embodiments, the one or more selected platelet preparations comprises greater than the number of platelets required to prepare three platelet units, but less than the number of platelets required to prepare four platelet units. In some embodiments, the one or more selected platelet preparations comprises at least about 325%, at least about 330%, at least about 335%, at least about 340%, at least about 345%, or at least about at least about 350% of the number of platelets required to prepare one platelet unit, but less than the number of platelets required to prepare four platelet units. In some embodiments, the target volume of the new platelet donation is a volume comprising a sufficient number of platelets to prepare to prepare four, to prepare five, to prepare six, or to prepare seven platelet units after combining the one or more selected platelet preparations and the new platelet donation and separating the resulting pooled platelet preparation into a plurality of platelet units. In some embodiments, the new platelet donation comprises a sufficient number of platelets that, when combined with the one or more selected platelet preparation, yields a pooled platelet preparation comprising a sufficient number of platelets to separate into four, into five, into six, or into seven platelet units. In some embodiments, collecting the new platelet donation from the donor comprises collecting a sufficient number of platelets to prepare four, to prepare five, to prepare six, or to prepare seven platelet units from the pooled preparation.

In some embodiments, each of the one or more selected platelet preparations and the new platelet donation comprises greater than the number of platelets required to prepare one therapeutic dose, but less than the number of platelets required to prepare two therapeutic doses. In some embodiments, each of the one or more selected platelet preparations and the new platelet donation comprises greater than the number of platelets required to prepare two therapeutic dosage units of platelets, but less than the number of platelets required to prepare three therapeutic dosage units of platelets. In some embodiments, the one or more selected platelet preparations comprise greater than the number of platelets required to prepare one therapeutic dose, but less than the number of platelets required to prepare two therapeutic doses and the new platelet donation comprises less than the number of platelets required to prepare one therapeutic doses. In some embodiments, the one or more selected platelet preparations comprise greater than the number of platelets required to prepare one therapeutic dose, but less than the number of platelets required to prepare two therapeutic doses and the new platelet donation comprises greater than the number of platelets required to prepare two therapeutic doses, but less than the number of platelets required to prepare three therapeutic doses. In some embodiments, the one or more selected platelet preparations comprise greater than the number of platelets required to prepare one therapeutic dose, but less than the number of platelets required to prepare two therapeutic doses and the new platelet donation comprises greater than the number of platelets required to prepare three therapeutic doses, but less than the number of platelets required to prepare four therapeutic doses. In some embodiments, the one or more selected platelet preparations comprise greater than the number of platelets required to prepare two therapeutic doses, but less than the number of platelets required to prepare three therapeutic doses and the new platelet donation comprises less than the number of platelets required to prepare one therapeutic dose. In some embodiments, the one or more selected platelet preparations comprise greater than the number of platelets required to prepare two therapeutic doses, but less than the number of platelets required to prepare three therapeutic doses and the new platelet donation comprises greater than the number of platelets required to prepare one therapeutic dose, but less than the number of platelets required to prepare two therapeutic doses. In some embodiments, the one or more selected platelet preparations comprise greater than the number of platelets required to prepare two therapeutic doses, but less than the number of platelets required to prepare three therapeutic doses and the new platelet donation comprises greater than the number of platelets required to prepare three therapeutic doses, but less than the number of platelets required to prepare four therapeutic doses.

In some embodiments, the one or more selected platelet preparations and the new platelet donation are combined within about 7 days, within about 6 days, within about 5 days, within about 4 days, within about 3 days, within about 2 days, or within about 1 day from the time of collection. In some embodiments, the one or more selected platelet preparations and the new platelet donation are combined within 20 hours from the time of collection. In some embodiments, the one or more selected platelet preparations and the new platelet donation are combined within 12 hours from the time of collection. In some embodiments, the one or more selected platelet preparations and the new platelet donation are combined within 6 hours from the time of collection. In some embodiments, the one or more selected platelet preparations and the new platelet donation are from donors of the same ABO blood type.

In some embodiments, the platelet units (*e.g.,* each platelet unit) in the plurality of platelet units comprise at least about 2.0×10¹¹ platelets, at least about 2.2×10¹¹ platelets, at least about 2.4×10¹¹ platelets, at least about 2.5×10¹¹ platelets, at least about 2.6×10¹¹ platelets, at least about 2.7×10¹¹ platelets, at least about 2.8×10¹¹ platelets, at least about 2.9×10¹¹ platelets or at least about 3.0×10¹¹ platelets. In some embodiments, each platelet unit comprises at least 2.4×10¹¹ platelets. In some embodiments, each platelet unit comprises at least 2.6×10¹¹ platelets. In some embodiments, each platelet unit comprises at least 3.0×10¹¹ platelets. In some embodiments, the platelet units each comprise at least about 3.0×10¹¹ platelets, and the pooled platelet preparation comprises at least about 6.2×10¹¹ platelets. In some embodiments, the platelet units each comprise at least about 3.0×10¹¹ platelets, and the pooled platelet preparation comprises at least about 9.3×10¹¹ platelets. In some embodiments, the platelet units each comprise at least about 3.0×10¹¹ platelets, and the pooled platelet preparation comprises at least about 12.4×10¹¹ platelets. In some embodiments, the platelet units each comprise at least about 3.0×10¹¹ platelets, and the pooled platelet preparation comprises at least about 15.5×10¹¹ platelets.

In some embodiments, the method further comprises subjecting the pooled platelet preparation to a pathogen inactivation process. In some embodiments, subjecting the pooled platelet preparation to a pathogen inactivation process comprises treating the pooled platelet preparation with a pathogen inactivating compound. In some embodiments, the method further comprises subjecting each of the one or more selected platelet preparations and the new platelet donation to a pathogen inactivation process. In some embodiments, subjecting the one or more selected platelet preparations and the new platelet donation to a pathogen inactivation process comprises treating the one or more selected platelet preparations and the new platelet donation with a pathogen inactivating compound, In some embodiments, the pathogen inactivating compound is a photoactive pathogen inactivating compound selected from the group consisting of a psoralen, an isoalloxazine, an alloxazine, a phthalocyanine, a phenothiazine, a porphyrin, and merocyanine 540. In some embodiments, the pathogen inactivating compound is a psoralen. In some embodiments, the pathogen inactivating compound is amotosalen.

In some embodiments, the platelet units comprise a minimum number of platelets as defined by criteria (*e.g.,* acceptance criteria) of a governmental agency, regulatory agency, institution and/or accrediting organization (*e.g.,* governmental agency, regulatory agency, institution and/or accrediting organization for donated blood products (*e.g.,* donated platelets)). In some embodiments, the platelet units of platelets are prepared in the country of the governmental agency, regulatory agency, institution and/or accrediting organization defining the criteria of a platelet unit. In some embodiments, at least about 75%, at least about 80%, at least about 85%, at least about 90%, at least about 95%, at least about 98%, at least about 99% or more of the platelets units comprise the minimum number of platelets of a platelet unit. In some embodiments, each of the platelet units comprises the minimum number of platelets of a therapeutic dosage unit. In some embodiments, the minimum number of platelets in a therapeutic dosage unit is at least about 2.0×10¹¹ platelets, at least about 2.2×10¹¹ platelets, at least about 2.4×10¹¹ platelets, at least about 2.5×10¹¹ platelets, at least about 2.6×10¹¹ platelets, at least about 2.7×10¹¹ platelets, at least about 2.8×10¹¹ platelets, at least about 2.9×10¹¹ platelets or at least about 3.0×10¹¹ platelets.

It is to be understood that one, some, or all of the properties of the various embodiments described herein may be combined to form other embodiments. These and other aspects will become apparent to one of skill in the art. These and other embodiments are further described by the detailed description that follows.

### DESCRIPTION OF THE DRAWINGS

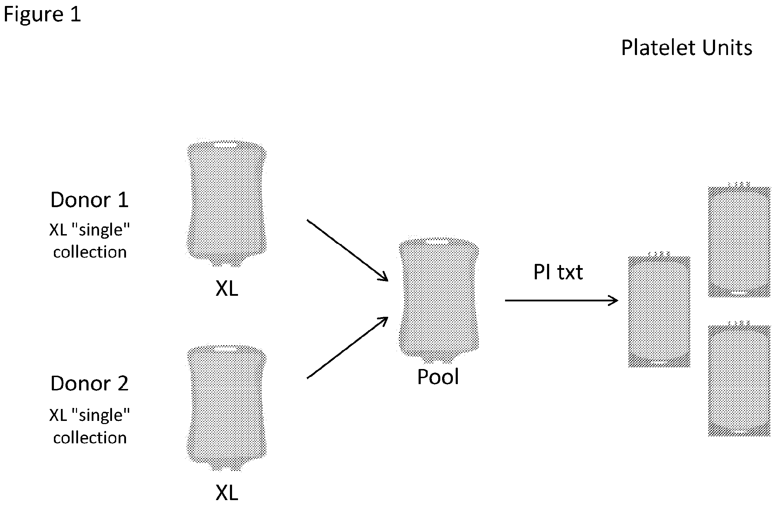

**Figure 1** shows a method of preparing three therapeutic units of platelets from two extra-large "single" apheresis platelet donations from a first donor and second donor. Abbreviations: XL, extra-large single; PI txt, pathogen inactivation treatment.

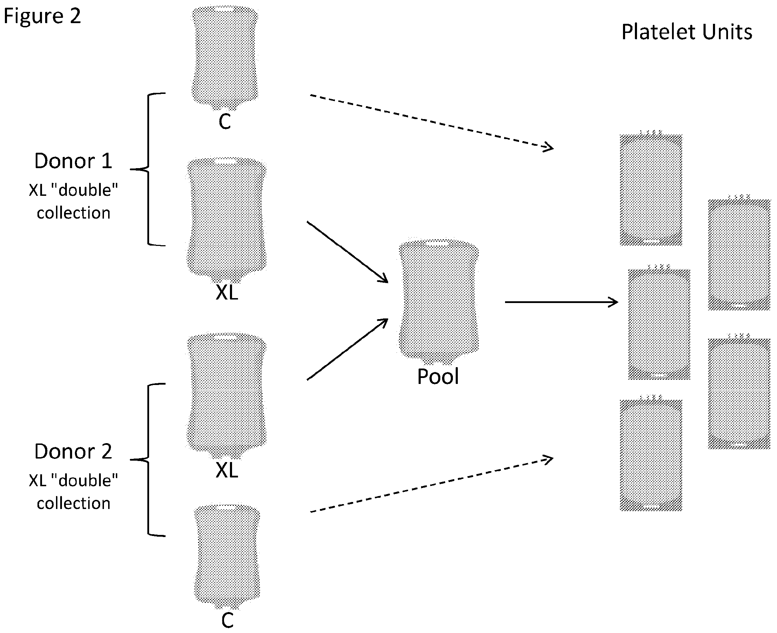

**Figure 2** shows a method of preparing five therapeutic units of platelets from two extra-large "double" apheresis platelet donations collected from a first donor and second donor. Abbreviations: XL, extra-large single; C, conventional single.

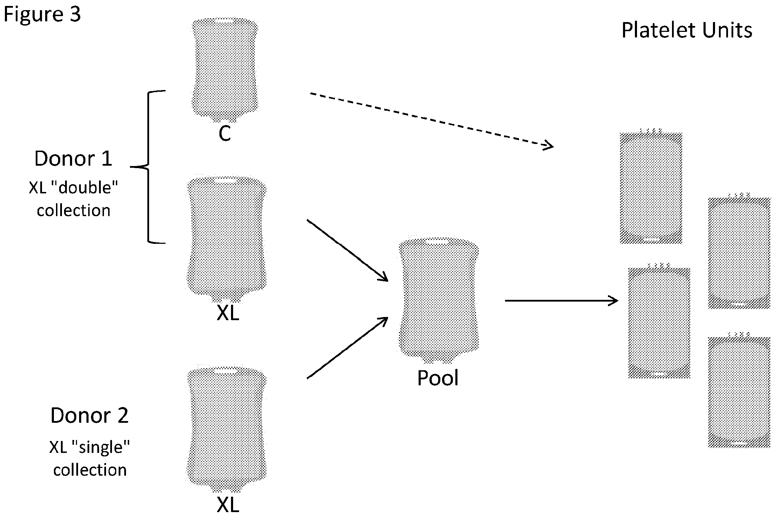

**Figure 3** shows a method of preparing four therapeutic units of platelets from one extra-large "double" apheresis platelet donation collected from a first donor and one extra-large "single" apheresis platelet donation collected from second donor. Abbreviations: XL, extra-large single; C, conventional single.

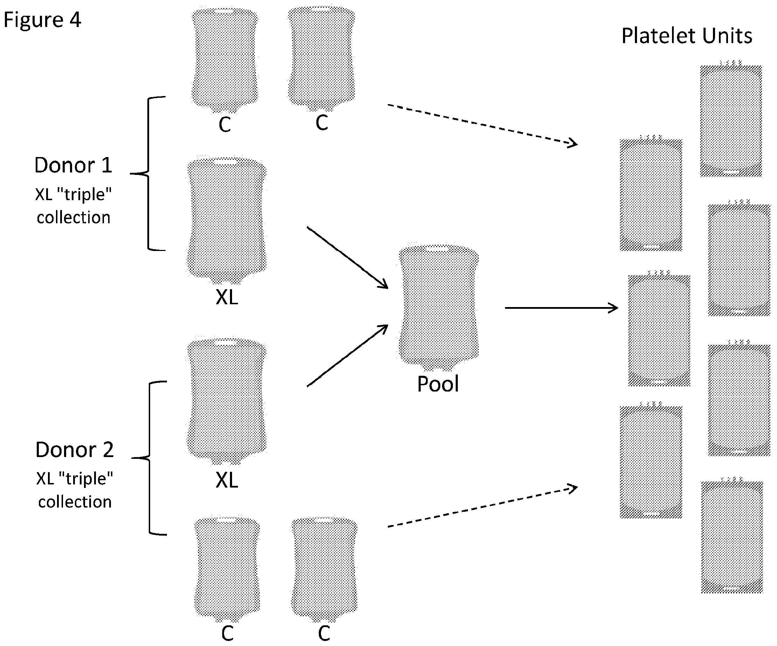

**Figure 4** shows a method of preparing seven therapeutic units of platelets from two extra-large "triple" apheresis platelet donations collected from a first donor and second donor. Abbreviations: XL, extra-large single; C, conventional single.

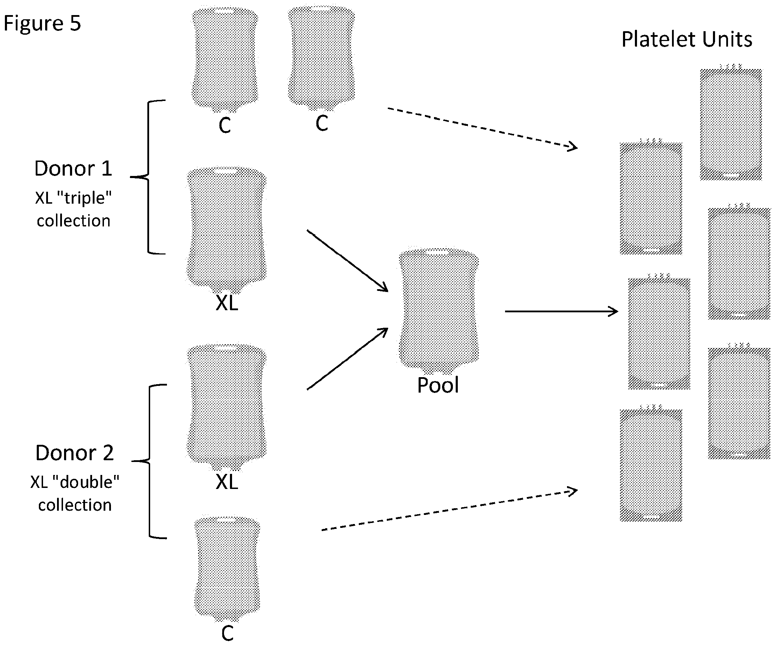

**Figure 5** shows a method of preparing six therapeutic units of platelets from one extra-large "triple" apheresis platelet donation collected from a first donor and one extra-large "double" apheresis platelet donation collected from second donor. Abbreviations: XL, extra-large single; C, conventional single.

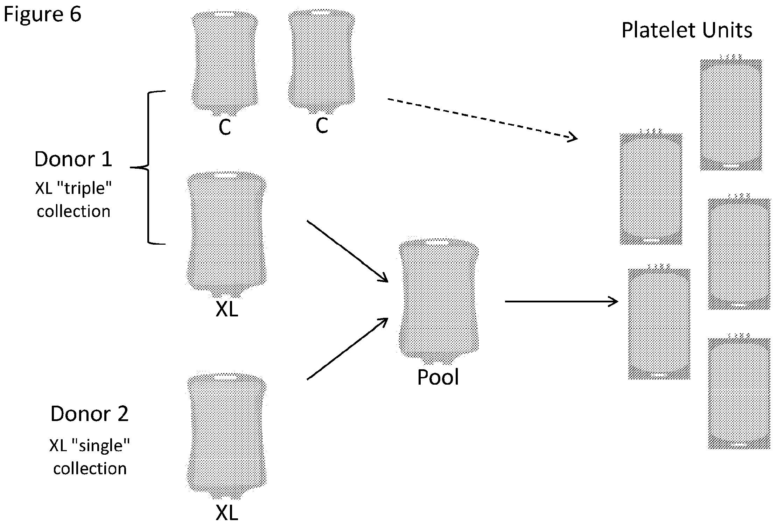

**Figure 6** shows a method of preparing five therapeutic units of platelets from one extra-large "triple" apheresis platelet donation collected from a first donor and one extra-large "single" apheresis platelet donation collected from second donor. Abbreviations: XL, extra-large single; C, conventional single.

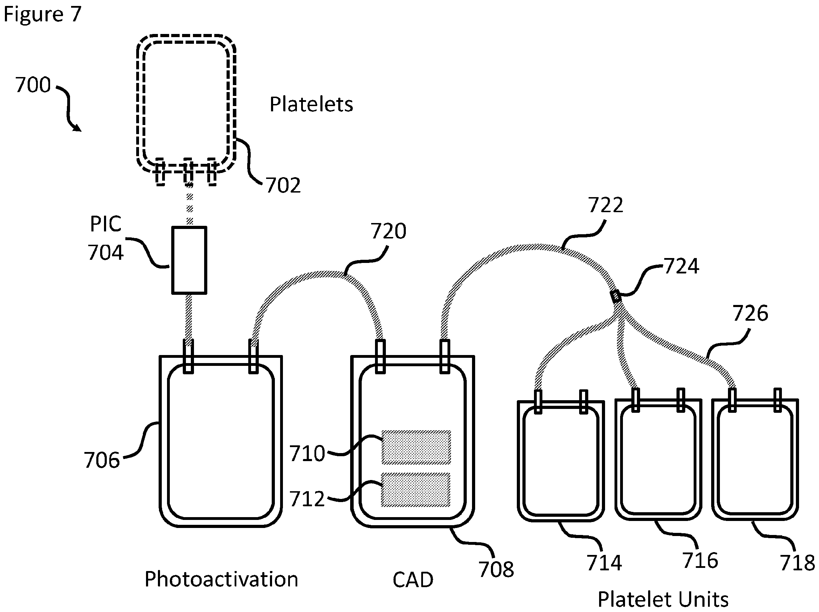

**Figure 7** shows an exemplary processing kit for use in preparing pathogen inactivated platelet units in accordance with some embodiments. Dotted components depict one container with platelets (*e.g*., donor platelets) sterile connected to the processing kit. Abbreviations: PIC, pathogen inactivating compound; CAD, compound adsorption device.

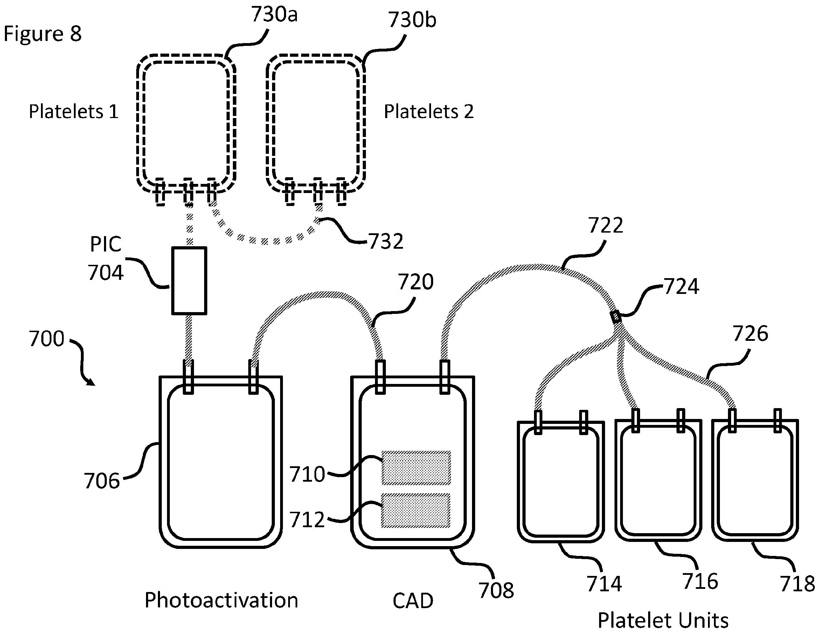

**Figure 8** shows an exemplary processing kit for use in preparing pathogen inactivated platelet units in accordance with some embodiments. Dotted components depict two containers with platelets (*e.g.,* first donor platelets, second donor platelets) sterile connected to each other (*e.g*., for pooling) and to the processing kit. Abbreviations: PIC, pathogen inactivating compound; CAD, compound adsorption device.

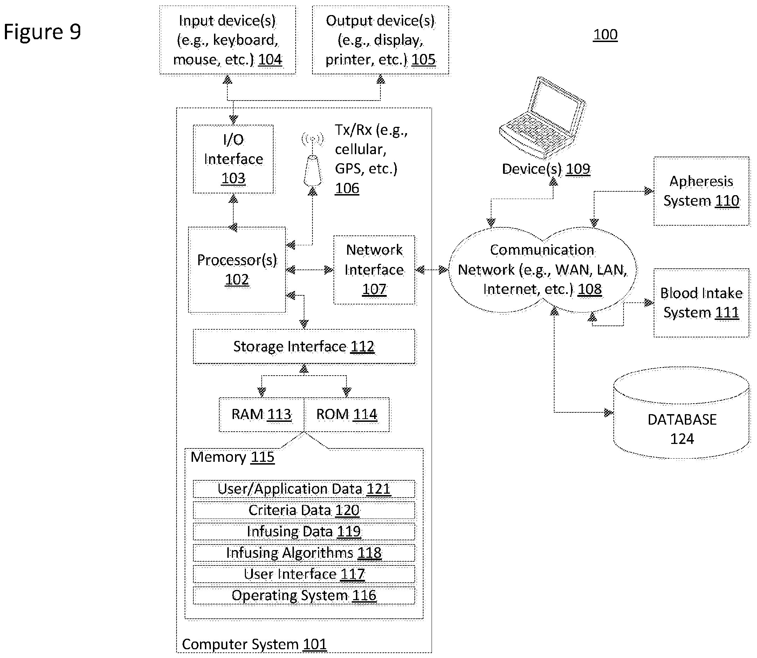

**Figure 9** illustrates a system for optimization of platelet units for infusion.

### DETAILED DESCRIPTION

The term "pooled platelet preparation" refers to a preparation of platelets comprising platelets obtained from more than one donation, such as an apheresis platelet donation, and subsequently combined (*e.g.,* in a single container). Generally, the platelet donations are obtained from different donors. Platelets may be pooled at any stage after donation and prior to therapeutic use, including but not limited to pooling before or after any addition of additive solution, before or after any storage period, and before or after any pathogen inactivation treatment or process. The platelets may be combined into any container suitable for platelet preparations and of sufficient size to accommodate the platelet volume, such as for example, by sterile connecting the containers of two platelet preparations (*e.g.,* with connecting tubing) and transferring the platelets from one container into the other, or by sterile connecting the containers of two platelet preparations to a third container (*e.g.,* with connecting tubing) and transferring the platelets into the third container.

The term "pathogen inactivation process" means a process useful to inactivate pathogens that may be present in a preparation of platelets, such as a platelet donation, where it is understood that the process does not necessarily inactivate completely all pathogens that may be present, but substantially reduces the amount of pathogens to significantly reduce the risk of a transfusion associated disease. The inactivation of a pathogen may be assayed by measuring the number of infective pathogen (*e.g.,* viral or bacterial particles) in a certain volume, and the level of inactivation is typically represented in the log reduction in the infectivity of the pathogen, or log reduction in titer. Methods of assaying log reduction in titer, and measurements thereof for pathogen inactivation are known in the art. When the inactivation process is tested against a variety of pathogens, the reduction in a particular active pathogen is at least about 1 log, at least about 2 log, at least about 3 log, at least about 4 log, or at least about 5 log reduction in titer. A variety of pathogen inactivation processes are known in the art and may be used in the methods of the present disclosure, including for example, commercially available pathogen inactivation processes, such as INTERCEPT Blood System (Cerus Corp), Mirasol (TerumoBCT) and Theraflex (MacoPharma). In certain embodiments, a pathogen inactivation process may comprise treating with a pathogen inactivating compound.

The term "pathogen inactivating compound" means any suitable compound, such as a small organic compound, that can be used to inactivate a pathogen that may be present in a platelet-containing blood product. A "photoactivated pathogen inactivation compound" is a suitable compound that requires some level of light in order to sufficiently inactivate a pathogen. Such compounds are preferred in the inactivation of pathogens in platelet products as they provide control over the inactivation process. Such photoactivated pathogen inactivation compounds described herein include psoralens, isoalloxazines, alloxazines, phthalocyanines, phenothiazines, and porphyrins, where these terms are understood to encompass a general class of compounds, i.e. the core compound and suitable derivatives thereof. For example psoralens or a psoralen generally describes the psoralen core compound and any derivative thereof (e.g. amotosalen), isoalloxazines or an isoalloxazine generally describes the isoalloxazine core and any derivative thereof (e.g. riboflavin), and so forth. Such derivatives comprise the core compound structure as well as additional substituents on the core. Descriptions of such compounds include any salts thereof.

The term "amotosalen" means the compound 3-(2-aminoethoxymethyl)-2,5,9-trimethylfuro[3,2-g]chromen-7-one and any salts thereof. The compound may also be referred to as 4'-(4-amino-2-oxa)butyl-4,5',8-trimethyl psoralen. The compound may also be referred to as 3-[(2-aminoethoxy) methyl]-2.5.9-trimethyl-7H-furo [3, 2-g] [1] benzopyran-7-one. Where the inactivation of platelets includes adding amotosalen HCl (the HCl salt of amotosalen) to a unit of platelets, the removal of this compound from the unit of platelets is not limited to the removal of amotosalen HCl, as the amotosalen can be present in solution as other salts or as the free base. As used in the methods described herein, removal of amotosalen means removal of the compound in any form, e.g. as the free base or as any salt, as measured by the assays described herein.

### Apheresis Collected Platelets

Certain aspects of the present disclosure provide methods related to improvements (*e.g.,* providing greater efficiency) in the collection and preparation of platelet units (*e.g.,* apheresis-derived platelet units), including pathogen-inactivated platelet units, such as for example, in blood centers using apheresis collection systems for platelet donations. A particular benefit, among others, provided by the improvements disclosed herein is the opportunity to produce an increased number of platelet units (e.g., platelet units suitable for infusion) without increased the number of platelet donations or required donors. Apheresis generally refers to automated blood collection device that uses a centrifugal or filtration separation to automatically withdraw whole blood from a donor, separate the whole blood into blood components, collects certain of the components (*e.g.,* platelets), and return to the donor the remainder of the whole blood and remaining uncollected blood components. Plateletpheresis is the collection of platelets using such an automated blood cell separator device, which results in obtaining a high yield of platelets (*e.g.,* apheresis platelets) from a single donor. Some automated blood cell separator devices are capable of collection procedures not only for single platelet units, but also double and triple platelet units. Apheresis collection devices are well known in the art, with several such devices commercially available, including for example, the Amicus^{™} system (Fenwal, Inc), the Trima Accel^{®} system (Terumo BCT) and the MCS^{®}+ 9000 mobile system (Haemonetics, Inc).

Apheresis platelet donations are based on certain donor parameters, such as for example, gender, physical size (*e.g.,* weight), hemoglobin level, platelet count on the day of donation, prior donation history and donation frequency, in part to ensure only a safe amount of platelets is collected. Any or all of these parameters may be entered into a computer system and/or the apheresis collection device. From these parameters, apheresis platelet donations generally are collected from an individual donor as a volume to yield one, two or three platelet units (e.g., therapeutic dosage units) each containing a specified minimum number (*e.g.,* at least a specified minimum number) of platelets per unit to meet the therapeutic dose requirement, with such per unit or therapeutic dose criteria generally determined by governmental, regulatory or accrediting organization (*e.g.,* industry) standards. Non-limiting examples of such standards include, for example, those set forth by FDA, EDQM, AABB, PMDA, TGA and SFDA. The specified minimum, for example, may vary by country. In some embodiments, platelet units may comprise at least about 2.0×10¹¹, at least about 2.2×10¹¹, at least about 2.4×10¹¹ platelets, at least about 2.5×10¹¹ platelets, at least about 2.6×10¹¹ platelets, at least about 2.7×10¹¹ platelets, at least about 2.8×10¹¹ platelets, at least about 2.9×10¹¹ platelets or at least about 3.0×10¹¹ or more platelets. Generally, a platelet number may be determined for each platelet unit, for example, based on a pre-donation platelet count and information about the volume collected, or alternatively by post-collection testing of units. Preferably, each platelet unit will comprise the specified minimum platelet number; however, determination of the platelet number for each unit may not be an absolute requirement and some platelet units in a plurality of platelet units may have less than a specified number.

The present disclosure may refer in various ways to platelets collected from a donor (*e.g.,* platelet donations, platelet preparations), such as for example as platelet donations or platelet preparations already collected (*e.g.,* collected and stored), with or without further processing (e.g., leukofiltration, pathogen inactivation), platelet donations or platelet preparations currently being collected from a donor, and/or platelet donations or platelet preparations to be collected from a donor (*e.g.,* a donor that has been identified, a present donor). Generally, reference to platelet donations or platelet preparations collected from a donor is prior to any pooling or combining step with additional platelets (*e.g.,* from a different donor, such as a second donor or third donor) that may provide pooled platelet preparation.

In some embodiments of the present disclosure, platelets collected from a donor by apheresis may comprise less than the number (*e.g.,* less than the minimum number, less than the minimum quantity, less than 100%) of platelets required to prepare one platelet unit (*e.g.,* therapeutic dosage unit). For example, a platelet donation may comprise platelets in an amount that is less than about 99%, less than about 98%, less than about 97%, less than about 95%, less than about 90%, less than about 85%, less than about 80%, less than about 75%, less than about 70%, less than about 65%, less than about 60%, less than about 55%, less than about 50%, less than about 45%, less than about 40%, less than about 35%, less than about 30% or less than about 25% of the number of platelets required to prepare one platelet unit. Such platelet donations may comprise a minimum amount of platelets, such as for example, at least about 10%, at least about 20%, at least about 30%, at least about 40% or at least about 50% or more of the number of platelets required to prepare one platelet unit. Alternatively or in addition, the platelet donation may comprise an amount of platelets that is about 10% to about 98%, about 10% to about 95%, about 10% to about 90%, 15% to about 98%, about 15% to about 95%, about 15% to about 90%, 20% to about 98%, about 20% to about 95%, about 20% to about 90%, 25% to about 98%, about 25% to about 95%, about 25% to about 90%, 30% to about 98%, about 30% to about 95%, about 30% to about 90%, 35% to about 98%, about 35% to about 95%, about 35% to about 90%, 40% to about 98%, about 40% to about 95%, about 40% to about 90%, 50% to about 98%, about 50% to about 95% or about 50% to about 90% of the number of platelets required to prepare one platelet unit. In some embodiments, the platelet donation comprises an amount of platelets that is about 98%, about 95%, about 90%, about 85%, about 80%, about 75%, about 70%, about 65%, about 60%, about 55%, about 50%, about 45%, about 40%, about 35%, about 30% or about 25% of the number of platelets required to prepare one platelet unit.

In some embodiments, platelets collected from a donor by apheresis may comprise greater than the number (*e.g.,* greater than the minimum number, greater than the minimum quantity) of platelets required to prepare one platelet unit, but less than the number (*e.g.,* less than the minimum number, less than the minimum quantity) of platelets required to prepare two platelet units. For example, a platelet donation comprising greater than the number of platelets required to prepare one platelet unit, but less than the number of platelets required to prepare two platelet units may comprise platelets in an amount that is about 1.1-fold, about 1.15-fold, about 1.2-fold, about 1.25-fold, about 1.3-fold, about 1.35-fold, about 1.4-fold, about 1.45-fold, about 1.5-fold, about 1.6-fold, about 1.7-fold, about 1.8-fold, about 1.9-fold, about 1.95-fold or about 1.98-fold the number of platelets required to prepare one platelet unit. Alternatively or in addition, a platelet donation comprising greater than the number of platelets required to prepare one platelet unit, but less than the number of platelets required to prepare two platelet units may comprise platelets in an amount that is about 1.1-fold to about 1.98-fold, about 1.1-fold to about 1.95-fold, about 1.1-fold to about 1.9-fold, about 1.15-fold to about 1.98-fold, about 1.15-fold to about 1.95-fold, about 1.15-fold to about 1.9-fold, about 1.2-fold to about 1.98-fold, about 1.2-fold to about 1.95-fold, about 1.2-fold to about 1.9-fold, about 1.25-fold to about 1.98-fold, about 1.25-fold to about 1.95-fold, about 1.25-fold to about 1.9-fold, about 1.3-fold to about 1.98-fold, about 1.3-fold to about 1.95-fold, about 1.3-fold to about 1.9-fold, about 1.35-fold to about 1.98-fold, about 1.35-fold to about 1.95-fold, about 1.35-fold to about 1.9-fold, about 1.4-fold to about 1.98-fold, about 1.4-fold to about 1.95-fold, about 1.4-fold to about 1.9-fold, about 1.5-fold to about 1.98-fold, about 1.5-fold to about 1.95-fold, or about 1.5-fold to about 1.9-fold the number of platelets required to prepare one platelet unit. Alternatively or in addition, a platelet donation comprising greater than the number of platelets required to prepare one platelet unit, but less than the number of platelets required to prepare two platelet units may comprise platelets in an amount that is about 105%, about 110%, about 115%, about 120%, about 125%, about 130%, about 135%, about 140%, about 145%, about 150%, about 155%, about 160%, about 165%, about 170%, about 175%, about 180%, about 185%, about 190%, about 195% or about 198% the number of platelets required to prepare one platelet unit. Alternatively or in addition, a platelet donation comprising greater than the number of platelets required to prepare one platelet unit, but less than the number of platelets required to prepare two platelet units may comprise platelets in an amount that is about 110% to about 198%, about 110% to about 195%, about 110% to about 190%, about 115% to about 198%, about 115% to about 195%, about 115% to about 190%, about 120% to about 198%, about 120% to about 195%, about 120% to about 190%, about 125% to about 198%, about 125% to about 195%, about 125% to about 190%, about 130% to about 198%, about 130% to about 195%, about 130% to about 190%, about 135% to about 198%, about 135% to about 195%, about 135% to about 190%, about 140% to about 198%, about 140% to about 195%, about 140% to about 190%, about 150% to about 198%, about 150% to about 195%, or about 150% to about 190% the number of platelets required to prepare one platelet unit.

In some embodiments, platelets collected from a donor by apheresis may comprise greater than the number (*e.g.,* greater than the minimum number, greater than the minimum quantity) of platelets required to prepare two platelet units, but less than the number (*e.g.,* less than the minimum number, less than the minimum quantity) of platelets required to prepare three platelet units. For example, a platelet donation comprising greater than the number of platelets required to prepare two platelet units, but less than the number of platelets required to prepare three platelet units may comprise platelets in an amount that is about 1.1-fold, about 1.15-fold, about 1.2-fold, about 1.25-fold, about 1.3-fold, about 1.35-fold, about 1.4-fold, about 1.45-fold, about 1.5-fold, about 1.6-fold, about 1.7-fold, about 1.8-fold, about 1.9-fold, about 1.95-fold or about 1.98-fold the number of platelets required to prepare two platelet units. Alternatively or in addition, a platelet donation comprising greater than the number of platelets required to prepare two platelet units, but less than the number of platelets required to prepare three platelet units may comprise platelets in an amount that is about 1.1-fold to about 1.98-fold, about 1.1-fold to about 1.95-fold, about 1.1-fold to about 1.9-fold, about 1.15-fold to about 1.98-fold, about 1.15-fold to about 1.95-fold, about 1.15-fold to about 1.9-fold, about 1.2-fold to about 1.98-fold, about 1.2-fold to about 1.95-fold, about 1.2-fold to about 1.9-fold, about 1.25-fold to about 1.98-fold, about 1.25-fold to about 1.95-fold, about 1.25-fold to about 1.9-fold, about 1.3-fold to about 1.98-fold, about 1.3-fold to about 1.95-fold, about 1.3-fold to about 1.9-fold, about 1.35-fold to about 1.98-fold, about 1.35-fold to about 1.95-fold, about 1.35-fold to about 1.9-fold, about 1.4-fold to about 1.98-fold, about 1.4-fold to about 1.95-fold, about 1.4-fold to about 1.9-fold, about 1.5-fold to about 1.98-fold, about 1.5-fold to about 1.95-fold, or about 1.5-fold to about 1.9-fold the number of platelets required to prepare two platelet units. Alternatively or in addition, a platelet donation comprising greater than the number of platelets required to prepare two platelet units, but less than the number of platelets required to prepare three platelet units may comprise platelets in an amount that is about 105%, about 110%, about 115%, about 120%, about 125%, about 130%, about 135%, about 140%, about 145%, about 150%, about 155%, about 160%, about 165%, about 170%, about 175%, about 180%, about 185%, about 190%, about 195% or about 198% the number of platelets required to prepare two platelet units. Alternatively or in addition, a platelet donation comprising greater than the number of platelets required to prepare two platelet units, but less than the number of platelets required to prepare three platelet units may comprise platelets in an amount that is about 110% to about 198%, about 110% to about 195%, about 110% to about 190%, about 115% to about 198%, about 115% to about 195%, about 115% to about 190%, about 120% to about 198%, about 120% to about 195%, about 120% to about 190%, about 125% to about 198%, about 125% to about 195%, about 125% to about 190%, about 130% to about 198%, about 130% to about 195%, about 130% to about 190%, about 135% to about 198%, about 135% to about 195%, about 135% to about 190%, about 140% to about 198%, about 140% to about 195%, about 140% to about 190%, about 150% to about 198%, about 150% to about 195%, or about 150% to about 190% the number of platelets required to prepare two platelet units.

In some embodiments, platelets collected from a donor by apheresis may comprise greater than the number (*e.g.,* greater than the minimum number, greater than the minimum quantity) of platelets required to prepare three platelet units, but less than the number (*e.g.,* less than the minimum number, less than the minimum quantity) of platelets required to prepare four platelet units. For example, a platelet donation comprising greater than the number of platelets required to prepare three platelet units, but less than the number of platelets required to prepare four platelet units may comprise platelets in an amount that is about 1.1-fold, about 1.15-fold, about 1.2-fold, about 1.25-fold, about 1.3-fold, about 1.35-fold, about 1.4-fold, about 1.45-fold, about 1.5-fold, about 1.6-fold, about 1.7-fold, about 1.8-fold, about 1.9-fold, about 1.95-fold or about 1.98-fold the number of platelets required to prepare three platelet units. Alternatively or in addition, a platelet donation comprising greater than the number of platelets required to prepare three platelet units, but less than the number of platelets required to prepare four platelet units may comprise platelets in an amount that is about 1.1-fold to about 1.98-fold, about 1.1-fold to about 1.95-fold, about 1.1-fold to about 1.9-fold, about 1.15-fold to about 1.98-fold, about 1.15-fold to about 1.95-fold, about 1.15-fold to about 1.9-fold, about 1.2-fold to about 1.98-fold, about 1.2-fold to about 1.95-fold, about 1.2-fold to about 1.9-fold, about 1.25-fold to about 1.98-fold, about 1.25-fold to about 1.95-fold, about 1.25-fold to about 1.9-fold, about 1.3-fold to about 1.98-fold, about 1.3-fold to about 1.95-fold, about 1.3-fold to about 1.9-fold, about 1.35-fold to about 1.98-fold, about 1.35-fold to about 1.95-fold, about 1.35-fold to about 1.9-fold, about 1.4-fold to about 1.98-fold, about 1.4-fold to about 1.95-fold, about 1.4-fold to about 1.9-fold, about 1.5-fold to about 1.98-fold, about 1.5-fold to about 1.95-fold, or about 1.5-fold to about 1.9-fold the number of platelets required to prepare three platelet units. Alternatively or in addition, a platelet donation comprising greater than the number of platelets required to prepare three platelet units, but less than the number of platelets required to prepare four platelet units may comprise platelets in an amount that is about 105%, about 110%, about 115%, about 120%, about 125%, about 130%, about 135%, about 140%, about 145%, about 150%, about 155%, about 160%, about 165%, about 170%, about 175%, about 180%, about 185%, about 190%, about 195% or about 198% the number of platelets required to prepare three platelet units. Alternatively or in addition, a platelet donation comprising greater than the number of platelets required to prepare three platelet units, but less than the number of platelets required to prepare four platelet units may comprise platelets in an amount that is about 110% to about 198%, about 110% to about 195%, about 110% to about 190%, about 115% to about 198%, about 115% to about 195%, about 115% to about 190%, about 120% to about 198%, about 120% to about 195%, about 120% to about 190%, about 125% to about 198%, about 125% to about 195%, about 125% to about 190%, about 130% to about 198%, about 130% to about 195%, about 130% to about 190%, about 135% to about 198%, about 135% to about 195%, about 135% to about 190%, about 140% to about 198%, about 140% to about 195%, about 140% to about 190%, about 150% to about 198%, about 150% to about 195%, or about 150% to about 190% the number of platelets required to prepare three platelet units.

In some or all of the aforementioned embodiments, when a donation comprises greater than the number of platelets required to prepare a particular (*e.g.,* targeted, desired) number of platelet units (*e.g.,* one platelet unit, two platelet units, three platelet units), greater than the number of platelets required to prepare the particular number of units refers to the donation comprising a number of platelets greater than the number required to prepare the particular number of units after factoring in any platelet losses from processing, sampling (*e.g.,* sampling for testing) and/or pathogen inactivation treatment. Likewise, in some or all of the aforementioned embodiments, when a donation comprises less than the number of platelets required to prepare a particular (*e.g.,* targeted, desired) number of platelet units (*e.g.,* one platelet unit, two platelet units, three platelet units), less than the number of platelets required to prepare the particular number of units refers to the donation comprising a number of platelets less than the number required to prepare the particular number of units after factoring in any platelet losses from processing, sampling (*e.g.,* sampling for testing) and/or pathogen inactivation treatment. In some embodiments, a donation may comprise more than the number of platelets required to prepare a particular number of platelet units (*e.g.,* one platelet unit, two platelet units, three platelet units) prior to any processing, sampling and/or pathogen inactivation treatment, but less than the number of platelets required to prepare the same number of platelet units after any processing, sampling and/or pathogen inactivation treatment.

### Preparation of Platelet Units

In certain aspects, the present disclosure provides a method for preparing a plurality of platelet units each comprising a therapeutic dose of platelets suitable for infusion into a human subject, comprising: a) collecting a first platelet donation by apheresis from a first donor, b) collecting a second platelet donation by apheresis from a second donor, c) combining the first platelet donation and the second platelet donation to yield a pooled platelet preparation, and d) separating the pooled platelet preparation into a plurality of platelet units each in an individual container, wherein the plurality of platelet units comprises a greater number of platelet units than the total number of platelet units that can be prepared from the first platelet donation and second platelet donation without combining the first and second platelet donations; wherein each platelet unit comprises a therapeutic dose of platelets.

In another aspect, the present disclosure also provides a method of preparing a plurality of platelet units suitable for infusion into a human subject, comprising: a) maintaining an inventory log of an inventory of apheresis-collected platelet preparations, the inventory log comprising for each platelet preparation a unique identifier, a donor blood type, a donation time and a quantity of platelets in the preparation, b) identifying an available donor suitable for donating platelets by apheresis, c) selecting from the inventory log at least one platelet preparation in the inventory from a first donor for combining with platelets collected from the available donor, wherein the first donor is different to the available donor, d) collecting a new platelet donation by apheresis from the available donor, e) combining the at least one selected platelet preparation from the inventory of apheresis-collected platelet preparations and the new platelet donation from the available donor to yield a pooled platelet preparation, and f) separating the pooled platelet preparation into a plurality of platelet units each in an individual container, wherein the plurality of platelet units comprises a greater number of platelet units than the total number of platelet units that can be prepared from the at least one selected platelet preparation from the inventory of apheresis-collected platelet preparations and the new platelet donation from the available donor without combining the at least one selected platelet preparation and new platelet donation.

In an embodiment, the method further comprises a) determining a target volume of the new platelet donation from the available donor, wherein the target volume optimizes the number of platelet units that can be prepared from a combination comprising the new platelet donation and the one or more selected platelet preparations from the inventory of apheresis-collected platelet preparations; and b) collecting the target volume of the new platelet donation from the available donor by apheresis from the donor. In some embodiments, the method still further comprises: c) combining the one or more selected platelet preparations and the new platelet donation from the available donor to yield a pooled platelet preparation; and d) separating the pooled platelet preparation into a plurality of platelet units (*e.g.,* therapeutic dosage units) each in an individual container, whereineach of platelet units in the plurality of platelet units comprises a therapeutic dose suitable for infusion into a human subject, and wherein the plurality of platelet units comprises a greater number of platelet units than the total number of platelet units that can be prepared from the one or more selected platelet preparations from the inventory of apheresis-collected platelet preparations and the new platelet donation from the available donor without combining the one or more selected platelet preparations and the new platelet donation.

In the aforementioned aspects, when two or more platelet donations/platelet preparations are combined to yield a pooled platelet preparation, each of the platelet donations/platelet preparations may comprise an amount (*e.g.,* number) of platelets that is similar or different from the other, such as set forth above. For example, one of the platelet donations (*e.g.,* a first platelet donation) may comprise less than the number of platelets required to prepare one platelet unit and the other platelet donation (*e.g.,* a second platelet donation) may comprise greater than the number of platelets required to prepare one platelet unit, but less than the number of platelets required to prepare two platelet units. Alternatively, one of the platelet donations may comprise less than the number of platelets required to prepare one platelet unit and the second platelet donation may comprise greater than the number of platelets required to prepare two platelet units, but less than the number of platelets required to prepare three platelet units. Alternatively, one of the platelet donations may comprise less than the number of platelets required to prepare one platelet unit and the other platelet donation may comprise greater than the number of platelets required to prepare three platelet units, but less than the number of platelets required to prepare four platelet units. Alternatively, one of the platelet donations may comprise greater than the number of platelets required to prepare one platelet unit, but less than the number of platelets required to prepare two platelet units, and the other platelet donation may comprise greater than the number of platelets required to prepare one platelet unit, but less than the number of platelets required to prepare two platelet units (see *e.g.,* Figure 1). Alternatively, one of the platelet donations may comprise greater than the number of platelets required to prepare one platelet unit, but less than the number of platelets required to prepare two platelet units, and the other platelet donation may comprise greater than the number of platelets required to prepare two platelet units, but less than the number of platelets required to prepare three platelet units (see *e.g.,* Figure 3). Alternatively, one of the platelet donations may comprise greater than the number of platelets required to prepare one platelet unit, but less than the number of platelets required to prepare two platelet units, and the other platelet donation may comprise greater than the number of platelets required to prepare three platelet units, but less than the number of platelets required to prepare four platelet units (see *e.g.,* Figure 6). Alternatively, one of the platelet donations may comprise greater than the number of platelets required to prepare two platelet units, but less than the number of platelets required to prepare three platelet units, and the other platelet donation may comprise greater than the number of platelets required to prepare two platelet units, but less than the number of platelets required to prepare three platelet units (see *e.g.,* Figure 2). Alternatively, one of the platelet donations may comprise greater than the number of platelets required to prepare two platelet units, but less than the number of platelets required to prepare three platelet units, and the other platelet donation may comprise greater than the number of platelets required to prepare three platelet units, but less than the number of platelets required to prepare four platelet units (see *e.g.,* Figure 5). Alternatively, one of the platelet donations may comprise greater than the number of platelets required to prepare three platelet units, but less than the number of platelets required to prepare four platelet units, and the other platelet donation may comprise greater than the number of platelets required to prepare three platelet units, but less than the number of platelets required to prepare four platelet units (see *e.g.,* Figure 4).

The present disclosure also contemplates that a pooled platelet preparation may comprise more than two platelet donations/platelet preparations (*e.g.,* a third platelet donation, a second new platelet donation). For example, the methods may further comprises collecting a third platelet donation by apheresis from a third donor, and combining the third platelet donation with a first platelet donation from a first donor and a second platelet donation from a second donors to yield a pooled platelet preparation, and separating the pooled platelet preparation into a plurality of platelet units each in an individual container, wherein the plurality of platelet units comprises a greater number of platelet units than the total number of platelet units that can be prepared from the first platelet donation, the second platelet donation and the third platelet donation without combining the first, second and third platelet donations. In some embodiments, the third platelet donation comprises greater than the number of platelets required to prepare one platelet unit, but less than the number of platelets required to prepare two platelet units. In some embodiments, the third platelet donation comprises greater than the number of platelets required to prepare two platelet units, but less than the number of platelets required to prepare three platelet units. In some embodiments, the third platelet donation comprises greater than the number of platelets required to prepare three platelet units, but less than the number of platelets required to prepare four platelet units.

In some embodiments, the method further comprises identifying a second available donor suitable for donating platelets by apheresis, collecting a second new platelet donation by apheresis from the second available donor, combining the second new platelet donation with an at least one selected platelet preparation from the inventory of apheresis-collected platelet preparations and a new platelet donation to yield the pooled platelet preparation, and separating the pooled platelet preparation into a plurality of platelet units each in an individual container, wherein the plurality of platelet units comprises a greater number of platelet units than the total number of platelet units that can be prepared from the at least one selected platelet preparation, the new platelet donation and the second new platelet donation without combining the at least one selected platelet preparation, the new platelet donation and the second new platelet donation.

In some embodiments, the method further comprises collecting a third platelet donation by apheresis from a third donor, combining the third platelet donation with an at least one selected platelet preparation from the inventory of apheresis-collected platelet preparations and a new platelet donation to yield a pooled platelet preparation, and separating the pooled platelet preparation into a plurality of platelet units each in an individual container, wherein the plurality of platelet units comprises a greater number of platelet units than the total number of platelet units that can be prepared from the at least one selected platelet preparation, the new platelet donation and the third platelet donation without combining the at least one selected platelet preparation, the new platelet donation and the third platelet donation. In some embodiments, the method further comprises subjecting the at least one selected platelet preparation, the new platelet donation, and/or the third platelet donation to a pathogen inactivation process.

In some of the aforementioned embodiments, the pooled platelet preparation comprises platelets only from donors (*e.g.,* first donor, second donor, third donor) of the same ABO blood type. In some embodiments, the pooled platelet preparation comprises platelets only from donors (*e.g.,* first donor, second donor, third donor) of the same ABO and Rh type.

Generally, when pooling two or more platelet donations and/or platelet preparations, it is preferred that the platelet donations and/or platelet preparations are derived from donations relatively close in time, such as for example within about 24 hours, within about 18 hours, within about 12 hours, or within about 6 hours of each other. Thus, in certain embodiments, the time of donation (*e.g.,* date, time, date and time) may be information of importance to be captured and/or associated with the platelet donation and/or platelet preparation. The time of donation may, in certain embodiments, refer to the time of initiating apheresis collection of platelets and/or time of completing apheresis collection of platelets. Additionally, a donation time may be, for example, a precise time (*e.g.,* to the nearest minute) or a time rounded to a time increment, such as the nearest 15 minute increment or 1 hour increment, or a time within a time range, such as 15 minute interval or time period or 1 hour interval or time period. In some of the aforementioned embodiments, platelet donations and/or platelet preparations are collected less than about (*e.g.,* within) 24 hours, less than about 22 hours, less than about 20 hours, less than about 16 hours, less than about 12 hours, less than about 10 hours, less than about 8 hours, less than about 6 hours, less than about 5 hours, less than about 4 hours, less than about 3 hours, less than about 2 hours or less than about 1 hour apart. In some embodiments, the second platelet donation is collected about the same time (*e.g.,* overlapping collection time) as the first platelet donation. In some embodiments, platelet donations and/or platelet preparations are collected at about the same time (*e.g.,* overlapping collection time). In some embodiments, platelet donations and/or platelet preparations are collected within a period (*e.g.,* the same period) of about 24 hours, about 22 hours, about 20 hours, about 16 hours, about 12 hours, about 10 hours, about 8 hours, about 6 hours, about 5 hours, about 4 hours, about 3 hours, about 2 hours or about 1 hour.

The pooled platelet preparation in any of the aforementioned embodiments may comprise, for example, at least about 5.0×10¹¹, at least about 5.5×10¹¹, at least about 6.0×10¹¹, at least about 6.5×10¹¹, at least about 7.0×10¹¹, at least about 7.5×10¹¹, at least about 8.0×10¹¹, at least about 8.5×10¹¹, at least about 9.0×10¹¹, at least about 9.5×10¹¹, at least about 10.0×10¹¹, at least about 10.5×10¹¹, at least about 11.0×10¹¹, at least about 11.5×10¹¹, at least about 12.0×10¹¹, at least about 12.5×10¹¹, at least about 13.0×10¹¹, at least about 13.5×10¹¹, at least about 14.0×10¹¹, at least about 14.5×10¹¹, or at least about 15.0×10¹¹ platelets. In some embodiments, the pooled platelet preparation may comprise, for example, less than about 16.0×10¹¹, less than about 15.0×10¹¹, less than about 14.0×10¹¹, less than about 13.0×10¹¹, or less than about 12.0×10¹¹ platelets. In some embodiments, the pooled platelet preparation may comprise, for example, about 5.0×10¹¹, about 5.5×10¹¹, about 6.0×10¹¹, about 6.5×10¹¹, about 7.0×10¹¹, about 7.5×10¹¹, about 8.0×10¹¹, about 8.5×10¹¹, about 9.0×10¹¹, about 9.5×10¹¹, about 10.0×10¹¹, about 10.5×10¹¹, about 11.0×10¹¹, about 11.5×10¹¹, about 12.0×10¹¹, about 12.5×10¹¹, about 13.0×10¹¹, about 13.5×10¹¹, about 14.0×10¹¹, about 14.5×10¹¹, or about 15.0×10¹¹ platelets Platelet Processing and Processing Sets and Kits

Platelet processing as described in the present disclosure may involve the use of blood product container or blood product bag systems, which are well known in the art. In general, such systems may include more than one plastic container, typically plastic bags, where the bags are integrally connected with plastic tubing. Some of the containers described herein include such plastic bags as are known in the storage and handling of blood products, including platelet products. Blood bags typically can be designed to hold various volumes of fluid, including, but not limited to, volumes ranging from 50 mL to 2 liters, for example having up to a 350 mL capacity, 450 mL capacity, 500 mL capacity, 1 liter capacity, up to a 1.5 liter capacity, or up to a 2 liter capacity. It is understood that when a method refers to a bag, it includes any such plastic bags used in blood product handling. Where such bags are referred to as "pooling bag", "mixing bag", "removal bag", "product bag", "storage bag", or "illumination bag", it is understood that these bags are typical blood product handling bags, or are similar to such bags in nature. Plastic bags suitable for use according to the present disclosure include for example, those comprising PL2410, as well as other suitable plastics known in the art. Plastic bag materials include polyvinyl chloride, polyolefins, ethylene vinyl acetate, ethylene vinyl acetate blended with other plastics, and the like.

As described herein, where tubing is described as connecting *e*.*g*. two bags, such as for pooling and/or of a processing set, it is understood that the tubing may be joined at some point therebetween by another component of the connection between the two bags. For example, a removal bag connected to a product bag by a tubing includes wherein the tubing comprises a filter between the two bags, i.e. the tubing is divided by a filter such that fluid flows from one bag to the other through the tubing and filter. In one example, tubing connecting a removal bag and a product bag can include a filter to remove any loose particles from fluid flowing from the removal device to the product bag, *i.e.* the tubing is divided by, or interrupted by the filter between the bags. Such filters are designed to remove any small particles that may come off of the removal device, while allowing platelets to pass through the filter. The tubing between bags allows for fluid to flow from one bag to another, which can be blocked to prevent the flow until necessary, e.g. as part of the processing the fluid in one bag may be prevented from flowing to the next bag until required for the next step in a process. As such an openable seal, such as a clamp, plug, valve or the like is included in or on the tubing connecting the bags, where the clamp, plug, valve or the like can be selectively opened as required, for example to transfer the fluid from one bag to the next. In certain embodiments, the tubing between bags comprises a breakable seal, such as a breakable valve, whereupon breaking the breakable seal allows for the blood product solution to flow between the bags through the tubing. It is understood that the breakable seal is contained within the connection between containers, such that sterility of the system is maintained. It is also understood that a tubing comprising a filter, or a breakable seal, includes where the tubing may be interrupted by the filter or the seal, for example the tubing runs from one bag and is connected to the filter or seal (an incoming portion of the tubing), and the tubing continues from another portion of the filter or seal to another bag (an outgoing portion of the tubing). In such a configuration, fluid flows from the first bag, through the incoming portion of the tubing, through the filter or seal, and through the outgoing portion of the tubing and into the other bag.

Different bags within a blood product bag system can be used for different steps of a process (see *e.g*., Figures 7-8). For example, a system of bags to be used for the pathogen inactivation of a preparation of platelets can include a container with pathogen inactivating compound contained within, a bag for receiving the unit of platelets (*e*.*g*., platelet donation) and a pathogen inactivating compound (*e*.*g*. an illumination bag), a bag for the illumination of the unit of platelets when the pathogen inactivation method includes illumination (*e*.*g*., an illumination bag, and typically the same bag to receive the unit of platelets and pathogen inactivating compound), a bag for the removal of pathogen inactivating compounds and/or by-products thereof from the treated unit of platelets (*e*.*g*., referred to as a removal bag, compound adsorption device, CAD), and one or more bags for containing the final platelet product, *i.e.* the pathogen inactivated platelet unit (*e*.*g*., therapeutic dosage unit) that has the concentration of the inactivating compound and/or by-products thereof reduced to below a desired concentration, which is ready for use or can be stored for later use (*e*.*g*., referred to as a product bag, storage bag). Each bag in the system is typically made up of a plastic material. For example, the container for containing a solution of pathogen inactivating compound can be made of a suitable plastic such as PL2411 (Baxter Healthcare), or other plastics such as polyvinyl chloride, polyolefins, ethylene vinyl acetate, ethylene vinyl acetate blended with other plastics, and the like. This container is also overwrapped with a material that is impermeable to light of a wavelength that will activate the photoactive pathogen inactivation compound (for example suitable plastic such as PL2420, Baxter Healthcare). The illumination bag for a photoactivated pathogen inactivating compound requires a clear, durable thermoplastic material that is translucent to light of the selected wavelength. Suitable plastics that are translucent to light in the UVA wavelength range include polyvinyl chloride, polyolefins, ethylene vinyl acetate, ethylene vinyl acetate blended with other plastics, or other blends of thermoplastic polymers. Such suitable plastics include PL2410 (Baxter Healthcare) and PL732 (Baxter Healthcare). Similar materials may be used to make the removal bag and the product bag. The product bags include, for example, those made of PL2410. Suitable bag materials are discussed, for example, in PCT publication number WO 2003078023, and US patent 7025877, the disclosures of which are hereby incorporated by reference as it relates to such bag materials and related materials. In all cases, the materials used in preparing the processing set have to be sterilizable by known methods such as steam and gamma or electron beam radiation used to ensure sterility of the processing set. While these are exemplary materials for making the bags, the methods described herein are applicable to processes using any suitable bag material as would be readily available to one skilled in the art, and can also be used with containers other than bags. The bags used for illumination, removal, and storage are also designed to allow for gases such as oxygen and carbon dioxide to go into and out of the blood bag, so that the platelets therein have adequate oxygen supply and carbon dioxide levels during the processing and storage.

Examples of processing sets are illustrated in **Figures 7** **&** **8**. Exemplary processing set 700 is shown in **Figure 7**. In some embodiments, set 700 includes optional bag 702 containing donor platelets. Processing set 700 includes container 704 (*e*.*g*., a first container) that contains a pathogen inactivation compound (PIC, *e.g.,* a psoralen) and is sterile connected to optional container (*e*.*g*., bag) 702 (*e*.*g*., an additional container) and connected to container (*e*.*g*., bag) 706 to allow for exposure of donor platelets to the PIC and sterile transfer of the donor platelets to container 706 for photochemical inactivation (*e*.*g*., a second container). Processing set 700 further includes CAD (*e*.*g*., CAD container, CAD bag) 708 connected to container 706 via sterile tubing 720, which allows transfer of the donor platelets after photochemical inactivation to the CAD. CAD 708 contains two wafers, 710 and 712, each of which provides for removal or reducing the concentration of pathogen inactivating compound. Advantageously, this allows for pathogen inactivation of a larger number or volume of donor platelets. For example, 710 and 712 can include adsorbent particles contained in a mesh pouch and/or matrix (*e.g.,* as described *infra*) that bind and/or otherwise adsorb the pathogen inactivating compound. Processing set 700 further includes sterile tubing 722, three-way lead 724, and three additional sterile tubings (*e*.*g*., tubing 726) that connect CAD 708 to platelet unit containers (*e*.*g*., bags) 714, 716, and 718 (*e*.*g*., three third containers). Advantageously, this configuration allows for the production of an increased number of platelet units (*e*.*g*., in bags 714, 716, and 718) from the donor platelets (*e*.*g*., if donor platelets 702 are from an apheresis donation).

An alternative configuration for processing set 700 is shown in **Figure 8**. Instead of being sterile connected to bag 702 containing donor platelets, container 704 is sterile connected to two containers, 730a and 730b (*e*.*g*., first and second additional containers), containing donor platelets (*e*.*g*., first donor platelets, second donor platelets). 730a and 730b are sterile connected to each other (*e*.*g*., for pooling) via sterile tubing 732. Advantageously, this configuration provides for a pooled platelet preparation that can be subjected to pathogen inactivation and subsequently divided into an increased number of individual platelet units (*e*.*g*., 714, 716, and 718).

### Pathogen inactivation

Blood products, including platelet-containing blood products, may contain pathogens, or may be contaminated with pathogens during processing. As such, it is desirable to subject such blood products to a pathogen inactivation process in order to reduce the risk of transfusiontransmitted diseases. Various processes and methods have been assessed to mitigate the risk of transfusion-associated disease transmission in platelet-containing blood products. Aside from screening and detection of pathogens and subsequent elimination of contaminated blood products, processes that incorporate treatments to inactivate pathogens (*i*.*e*., pathogen inactivation) that may be present are available. Ideally, such a process results in the inactivation of a broad range of pathogens such as viruses, bacteria and parasites that may be present in the blood product. In certain preferred embodiments, the methods of pathogen inactivation require addition of an amount of pathogen inactivating compound to a preparation of platelets (*e*.*g*., treating the platelet preparation). For example, pathogen inactivation may involve the addition of a low molecular weight compound that inactivates various pathogens, where a preferred method involves the addition of a photosensitizer that, when activated by illumination using light of suitable wavelengths, will inactivate a variety of pathogens that may be present. Two preferred methods that are commercially available include the addition of amotosalen or riboflavin to the platelets, with subsequent illumination with UV light. Other methods include illumination with UV light without addition of a photosensitizer, as well as illumination with other photoactive compounds, including psoralen derivatives other than amotosalen, isoalloxazines other than riboflavin, alloxazines, dyes such as phthalocyanines, phenothiazine dyes (e.g. methylene blue, azure B, azure C, thionine, toluidine blue), porphyrin derivatives (e.g. dihematoporphyrin ether, hematoporphyrin derivatives, benzoporphyrin derivatives, alkylsubstituted sapphyrin), and merocyanine 540 (Prodouz et al., Blood Cells 1992, 18(1):101-14; Sofer, Gail, BioPharm, August 2002). Other pathogen inactivation systems include, for example, those described in PCT publication numbers WO 2012071135; WO 2012018484; WO 2003090794; WO 2003049784; WO 1998018908; WO 1998030327; WO 1996008965; WO 1996039815; WO 1996039820; WO 1996040857; WO 1993000005; US patent application number US 20050202395; and US patent numbers 8296071 and 6548242, the disclosures of which are hereby incorporated by reference as they relate to pathogen inactivation in blood products. In some embodiments, the pathogen inactivating compound is a photoactive pathogen inactivating compound selected from the group consisting of a psoralen, an isoalloxazine, an alloxazine, a phthalocyanine, a phenothiazine, a porphyrin, and merocyanine 540. In some embodiments, the pathogen inactivating compound is a psoralen. In some embodiments, the pathogen inactivating compound is amotosalen. Where addition of a compound to the platelets is used for pathogen inactivation, whether the method requires illumination or not, in some instances it is desirable to remove any residual pathogen inactivation compound or by-product thereof.

Methods for pathogen inactivation and removal of pathogen inactivating compound as described herein are applicable to any platelet preparations, whether the platelet preparations comprise individual platelet donations (*e*.*g*., apheresis collected platelets) or pooled platelet preparations. These processes typically provide a platelet preparation that is either in about 85% to 100% plasma or has some amount of platelet additive solution added, typically in the range of 50 to 95% platelet additive solution, with the rest of the volume effectively being plasma, *i.e.* plasma in the range of about 5 to 50%. It is understood that a solution of pathogen inactivating compound can be added during the processing to inactivate pathogens, since pathogen inactivating compound is not typically combined in solid form, but is dissolved in a solution (for example, amotosalen is the HCl salt dissolved in a saline solution). As such, in some instances, when a platelet preparation is designated as about 100% plasma, it is understood that this means no additional platelet additive solution is included in the platelet unit. If such a preparation of platelets in about 100% plasma is treated for pathogen inactivation, some volume of the solution of pathogen inactivating compound will be included in the final product, as well as some volume of anticoagulant used in collecting the blood for isolation of platelets. While the plasma has been diluted partially with whatever amount of anticoagulant and solution that is used to contain the pathogen inactivating compound, the resulting platelet preparation including pathogen inactivation compound may be referred to as comprising about 100% plasma, or may be referred to as about 85 to 100% plasma (typically less than about 5 to 15% of the volume will comprise the solution used to deliver the pathogen inactivating compound). Platelet preparations can also be prepared with some amount of platelet additive solution, which may, for example, be added after concentrating the platelets, removing a portion of the plasma from the supernatant, and adding the desired amount of platelet additive solution to the platelet preparation. The platelet additive is added to provide the desired percentage of platelet additive solution. Such a preparation of platelets is typically adjusted so the plasma content is about 5 to 50%, with the remainder of the solution being platelet additive solution, i.e. 50 to 95% platelet additive solution. When amounts of plasma and platelet additive solutions are described, it is understood that as with platelet preparations described as being in about 100% plasma, some volume of solution containing a pathogen inactivating compound may be included in the unit of platelets containing a pathogen inactivating compound. While the solution has been diluted partially with whatever amount of solution is used to contain the pathogen inactivating compound, it is understood that, for example, a platelet preparation designated as comprising 35% plasma and 65% platelet additive solution may refer to relative amounts of plasma and platelet additive solution prior to the addition of a solution containing pathogen inactivating compound.

Some pathogen inactivation methods may require the use of a removal device, *i.e.* a device for reducing the concentration of pathogen inactivating compound, such as a small organic compound, *e*.*g*. platelet inactivating compound, and by-products thereof in a preparation of platelets, while substantially maintaining a desired biological activity of the platelets. In some embodiments, the removal device is referred to as a compound adsorption device (CAD), and may comprise a container (*e*.*g*., CAD container, CAD bag) containing one or more materials, such as for example, adsorbent particles, and which is suitable for also containing a preparation of platelets from which the concentration of pathogen inactivating compound and by-products thereof are to be reduced. Such a removal device is generally intended to be used in a batch mode, *i.e.* the device is placed in contact with the platelets, and continued contact with the removal device, *e*.*g*. with shaking to allow essentially the entirety of the solution of platelets to come into contact with the removal device over time of contact, results in reducing the levels of pathogen inactivating compound. Such batch devices entail the use of an adsorbent particle that binds the pathogen inactivation compound, and can be used by either adding adsorbent particles directly to the platelet container (*e*.*g*., bag) following illumination or transferring the platelets to a bag containing the adsorbent particles following illumination and the platelets are then agitated for a specified period of time with the platelet preparations contacting the removal device. While free adsorbent particles may be used as a removal device, such particles may be contained within a mesh pouch, such as a polyester or nylon mesh pouch, which allows for contact of the platelet solution with the adsorbent particles while containing the particles within the pouch. Alternatively, the adsorbent particles may be immobilized within a matrix, where the immobilized matrix can reside directly in the blood bag used for batch removal, or may be similarly contained within a mesh pouch. In some instances, the removal device comprises porous adsorbent particles in an amount sufficient to reduce the pathogen inactivating compound to below a desired concentration, wherein the adsorbent particles have an affinity for the pathogen inactivating compound, where it is understood such adsorbent particle can be selected to best adsorb the compound or compounds to be removed, with minimal effect on components that should not be removed or damaged by contact with the adsorbent particle. A variety of adsorbent particles are known, including generally particles made from any natural or synthetic material capable of interacting with compounds to be removed, including particulates made of natural materials such as activated carbon, silica, diatomaceous earth, and cellulose, and synthetic materials such as hydrophobic resins, hydrophilic resins or ion exchange resins. Such synthetic resins include, for example, carbonaceous materials, polystyrene, polyacrylic, polyacrylic ester, cation exchange resin, and polystyrene-divinylbenzene. Detailed description of such removal devices suitable for use in the methods as described herein can be found in PCT publication numbers WO 1996040857, WO 1998030327, WO 1999034914, and WO 2003078023, the disclosures of which are hereby incorporated by reference with respect to the discussion of such removal devices and the adsorbent particles and other materials used to prepare such devices. Exemplary adsorbent particles include, but are not limited to, Amberlite (Rohm and Haas) XAD-2, XAD-4, XAD-7, XAD-16, XAD-18, XAD-1180, XAD-1600, XAD-2000, XAD-2010; Amberchrom (Toso Haas) CG-71m, CG-71c, CG-161m, CG161c; Diaion Sepabeads (Mitsubishi Chemicals) HP20, SP206, SP207, SP850, HP2MG, HP20SS, SP20MS; Dowex (Dow Chemical) XLTS-40285, XLTS-40323, XLTS-43493 (also referred to as Optipore V493 (dry form) or Optipore L493 (hydrated form)), Optipore V503, Optipore SD-2; Hypersol Macronet (Purolite) MN-100, MN-102, MN-150, MN-152, MN-170, MN-200, MN-202, MN-250, MN-252, MN-270, MN-300, MN-400, MN-500, MN-502, Purosorb (Purolite) PAD 350, PAD 400, PAD 428, PAD 500, PAD 550, PAD 600, PAD 700, PAD 900, and PAD 950. The material used to form the immobilized matrix comprises a low melting polymer, such as nylon, polyester, polyethylene, polyamide, polyolefin, polyvinyl alcohol, ethylene vinyl acetate, or polysulfone. In one example, the adsorbent particles immobilized in a matrix are in the form of a sintered medium. While it is understood that the methods and devices described herein encompass removal devices as are known in the art, such methods and devices may be exemplified using the removal device of an amotosalen inactivated platelet product as is commercially available. Such a removal device comprises Hypersol Macronet MN-200 adsorbent contained within a sintered matrix, where the sintered matrix comprises PL2410 plastic as a binder. In one instance, the removal device comprises Hypersol Macronet MN-200 adsorbent in a sintered matrix comprising PL2410, wherein the Hypersol Macronet MN-200 is in an amount of about 5-50 grams, about 5-10 grams, about 10-15 grams, about 15-20 grams, about, 20-25 grams, about 25-30 grams, about 30-35 grams, about 35-40 grams, about 40-45 grams or about 45-50 grams dry weight equivalent.

As various resins may require different processing when used to make the removal devices useful in the methods and devices as described herein, comparison of amounts of adsorbent resins described herein, unless otherwise indicated, are comparison of the dry weight of the resin. For example, the resins are dried to < 5% water prior to processing, and the equivalent of the dry weight of adsorbent is used in comparing amounts of resin in use. For example, Hypersol Macronet MN-200 is processed to stabilize the adsorbent, or what is typically referred to as wetting the adsorbent, so as to be directly usable upon contact with a platelet unit. Such a wetted sample may include, for example, about 50% glycerol or other suitable wetting agent. In some embodiments, the adsorbent resin is a polystyrene-divinylbenzene resin. In some embodiments, the polystyrene-divinylbenzene resin is Hypersol Macronet MN-200. In some embodiments, the adsorbent is contained within a sintered matrix, wherein the sintered matrix comprises PL2410 binder. In some embodiments, Hypersol Macronet MN-200 adsorbent is contained within a sintered matrix to provide a removal device.

### Platelet Units

The platelet units (*e*.*g*., each platelet unit) in a plurality of platelet units resulting from the methods of the invention comprise a therapeutic dose (*e*.*g*., therapeutic dosage unit) of platelets suitable for infusion into a human subject (*e*.*g*., a subject in need of a platelet infusion). In some embodiments, the therapeutic dose comprises a minimum number (*e*.*g*., at least a minimum number) of platelets as defined by criteria (*e*.*g*., acceptance criteria) of a governmental agency, regulatory agency, institution and/or accrediting organization (*e*.*g*., governmental agency, regulatory agency, institution and/or accrediting organization for donated blood products (*e*.*g*., donated platelets)). The platelet units may be prepared in the country of the governmental agency, regulatory agency, institution and/or accrediting organization defining the criteria of a therapeutic dose of platelets. In some embodiments, at least about 75%, at least about 80%, at least about 85%, at least about 90%, at least about 95%, at least about 98%, at least about 99% or more of the platelet units in the plurality of platelet units comprise the minimum number of platelets of a therapeutic dose. In some embodiments, each of the platelet units in the plurality of platelet units comprises the minimum number of platelets of a therapeutic dose. In some embodiments, the minimum number of platelets in a therapeutic dose is at least about 2.0×10¹¹ platelets, at least about 2.2×10¹¹ platelets, at least about 2.4×10¹¹ platelets, at least about 2.5×10¹¹ platelets, at least about 2.6×10¹¹ platelets, at least about 2.7×10¹¹ platelets, at least about 2.8×10¹¹ platelets, at least about 2.9×10¹¹ platelets or at least about 3.0×10¹¹ platelets. In some embodiments, the platelet units in a plurality of platelet units comprise at least about 2.0×10¹¹ platelets, at least about 2.2×10¹¹ platelets, at least about 2.4×10¹¹ platelets, at least about 2.5×10¹¹ platelets, at least about 2.6×10¹¹ platelets, at least about 2.7×10¹¹ platelets, at least about 2.8×10¹¹ platelets, at least about 2.9×10¹¹ platelets or at least about 3.0×10¹¹ or more platelets. In some embodiments, each of the platelet units in a plurality of platelet units comprise at least about 2.0×10¹¹ platelets, at least about 2.2×10¹¹ platelets, at least about 2.4×10¹¹ platelets, at least about 2.5×10¹¹ platelets, at least about 2.6×10¹¹ platelets, at least about 2.7×10¹¹ platelets, at least about 2.8×10¹¹ platelets, at least about 2.9×10¹¹ platelets or at least about 3.0×10¹¹ or more platelets. In some embodiments, at least about 75% of the platelet units in a plurality of platelet units comprise at least about 2.0×10¹¹ platelets, at least about 2.2×10¹¹ platelets, at least about 2.4×10¹¹ platelets, at least about 2.5×10¹¹ platelets, at least about 2.6×10¹¹ platelets, at least about 2.7×10¹¹ platelets, at least about 2.8×10¹¹ platelets, at least about 2.9×10¹¹ platelets or at least about 3.0×10¹¹ or more platelets. In some embodiments, at least about 80% of the platelet units in the plurality of platelet units comprise at least about 2.0×10¹¹ platelets, at least about 2.2×10¹¹ platelets, at least about 2.4×10¹¹ platelets, at least about 2.5×10¹¹ platelets, at least about 2.6×10¹¹ platelets, at least about 2.7×10¹¹ platelets, at least about 2.8×10¹¹ platelets, at least about 2.9×10¹¹ platelets or at least about 3.0×10¹¹ or more platelets. In some embodiments, at least about 85% of the platelet units in the plurality of platelet units comprise at least about 2.0×10¹¹ platelets, at least about 2.2×10¹¹ platelets, at least about 2.4×10¹¹ platelets, at least about 2.5×10¹¹ platelets, at least about 2.6×10¹¹ platelets, at least about 2.7×10¹¹ platelets, at least about 2.8×10¹¹ platelets, at least about 2.9×10¹¹ platelets or at least about 3.0×10¹¹ or more platelets. In some embodiments, at least about 90% of the platelet units in the plurality of platelet units comprise at least about 2.0×10¹¹ platelets, at least about 2.2×10¹¹ platelets, at least about 2.4×10¹¹ platelets, at least about 2.5×10¹¹ platelets, at least about 2.6×10¹¹ platelets, at least about 2.7×10¹¹ platelets, at least about 2.8×10¹¹ platelets, at least about 2.9×10¹¹ platelets or at least about 3.0×10¹¹ or more platelets. In some embodiments, at least about 95% of the platelet units in the plurality of platelet units comprise at least about 2.0×10¹¹ platelets, at least about 2.2×10¹¹ platelets, at least about 2.4×10¹¹ platelets, at least about 2.5×10¹¹ platelets, at least about 2.6×10¹¹ platelets, at least about 2.7×10¹¹ platelets, at least about 2.8×10¹¹ platelets, at least about 2.9×10¹¹ platelets or at least about 3.0×10¹¹ or more platelets. In some embodiments, at least about 98% of the platelet units in the plurality of platelet units comprise at least about 2.0×10¹¹ platelets, at least about 2.2×10¹¹ platelets, at least about 2.4×10¹¹ platelets, at least about 2.5×10¹¹ platelets, at least about 2.6×10¹¹ platelets, at least about 2.7×10¹¹ platelets, at least about 2.8×10¹¹ platelets, at least about 2.9×10¹¹ platelets or at least about 3.0×10¹¹ or more platelets. In some embodiments, at least about 99% of the platelet units in the plurality of platelet units comprise at least about 2.0×10¹¹ platelets, at least about 2.2×10¹¹ platelets, at least about 2.4×10¹¹ platelets, at least about 2.5×10¹¹ platelets, at least about 2.6×10¹¹ platelets, at least about 2.7×10¹¹ platelets, at least about 2.8×10¹¹ platelets, at least about 2.9×10¹¹ platelets or at least about 3.0×10¹¹ or more platelets.

It will also be understood by those skilled in the art that changes in the form and details of the implementations described herein may be made without departing from the scope of this disclosure. In addition, although various advantages, aspects, and objects have been described with reference to various implementations, the scope of this disclosure should not be limited by reference to such advantages, aspects, and objects. Rather, the scope of this disclosure should be determined with reference to the appended claims.

The invention is illustrated further by the following examples.

### EXAMPLES

### Example 1: Apheresis collection of platelets

Donor(s) suitable for apheresis donation of platelet are identified and platelets are collected using an approved device with apheresis collection procedures known in the art. More specifically, in one example platelet donations were collected by apheresis using an AMICUS^{™} system (Fenwal, Inc., Lake Zurich, IL). Gender, weight, height, hematocrit and platelet pre-count were determined for available donors and the donors were targeted for a single, double or triple apheresis collections to prepare 1, 2 or 3 platelet units, each comprising at least the number of platelets per unit according to applicable FDA regulatory standards. The apheresis collections factored in potential losses (*e*.*g*., processing losses) and split ranges for the blood center were >3.1×10¹¹ platelets for a single, >6.2×10¹¹ platelets for a double, and >9.6×10¹¹ platelets for a triple. The corresponding ranges for such splits also may be represented, for example, as >3.1×10¹¹ to <6.2×10¹¹ platelets for a single, >6.2×10¹¹ to <9.6×10¹¹ platelets for a double, and >9.6×10¹¹ to <12.8×10¹¹ platelets for a triple.

Table 1 illustrates donor characterization and targeted platelet collections (target platelet yield) in order to prepare the indicated number of platelet products for each of twelve donors. Preparation of apheresis platelet units by standard methods as the targeted singles, doubles or triples resulted in 23 platelet units from the above twelve donors.

**Table 1. Apheresis platelet collections**

| Donor | Gender | Weight (lbs) | Height (in.) | Pre-count | Target Yield | Products |
|---|---|---|---|---|---|---|
| 1 | M | 185 | 75" | 375 | 10.96 | 3 |
| 2 | M | 167 | 71" | 257 | 7.20 | 2 |
| 3 | M | 170 | 70" | 246 | 6.38 | 2 |
| 4 | F | 202 | 66" | 433 | 10.50 | 3 |
| 5 | F | 165 | 63" | 309 | 7.80 | 2 |
| 6 | M | 210 | 70" | 309 | 8.75 | 2 |
| 7 | M | 175 | 72" | 205 | 5.21 | 1 |
| 8 | F | 165 | 64" | 441 | 7.00 | 2 |
| 9 | F | 135 | 67" | 220 | 4.50 | 1 |
| 10 | M | 212 | 73" | 234 | 8.00 | 2 |
| 11 | M | 140 | 67" | 190 | 4.86 | 1 |
| 12 | M | 155 | 66" | 362 | 9.15 | 2 |

Among these twelve donors, platelet collections for donors #6, 7, 11 and 12 may be categorized as extra-large collections, in that each comprises excess platelet numbers, in this case greater than 50% platelet above the platelet split number, but less than the platelet number to achieve the next higher split level to prepare an additional platelet unit product (*e*.*g*., based on platelet units of at least 3×10¹¹). For example, donor #6 with 8.75×10¹¹ platelets, exceeded the >6.2×10¹¹ yield for a double collection but is less than the 9.6×10¹¹ platelets for a triple collection (*e*.*g*., an extra-large double). Similarly, donor #11 with 4.86×10¹¹ platelets, exceeded the >3.1×10¹¹ yield for a single collection but is less than the >6.2×10¹¹ yield for a double collection (*e*.*g*., an extra-large single).

### Example 2: Preparing increased number of platelet units

The methods provided herein set forth a method of preparing a plurality of platelet units comprising a greater number of platelet units than the total number of platelet units that can be prepared from the same apheresis platelet donations without using the disclosed methods. More specifically, in one example, an increased number of platelet units are prepared from the twelve donors of Example 1 and Table 1. Following collection, the following pairs of platelet donations are combined:
Donors #7 and 11, each comprising greater than the number of platelets required to prepare one platelet unit, but less than the number of platelets required to prepare two platelet units.
Donors #6 and 12, each comprising greater than the number of platelets required to prepare two platelet units, but less than the number of platelets required to prepare three platelet units.

Following the combining of the platelet donations pairs, the pooled platelet preparations are separated into three platelet units (in the case of #7 with #11), and five platelet units (in the case of #6 and #12). Together with the units (e.g., non-pooled units) from the remaining eight donors from Table 1, these methods provide for an increased number of apheresis platelet units, with 25 total platelet units prepared, as compared to 23 platelet units that would result from convention apheresis collection and preparation methods (*e*.*g*., based on platelet units of at least 3×10¹¹). Thus, an increased platelet unit production of almost 9% based on the present disclosure, without additional donors or platelet donations.

Similarly, alternative combinations of the platelet donations may be combined to achieve and increased number of total platelet units prepared. For example, following collection, the following pairs of platelet donations are combined:
Donors #6 and 7, with #6 comprising greater than the number of platelets required to prepare two platelet units, but less than the number of platelets required to prepare three platelet units, and #7 comprising greater than the number of platelets required to prepare one platelet unit, but less than the number of platelets required to prepare two platelet units.
Donors #11 and 12, with #12 comprising greater than the number of platelets required to prepare two platelet units, but less than the number of platelets required to prepare three platelet units, and #11 comprising greater than the number of platelets required to prepare one platelet unit, but less than the number of platelets required to prepare two platelet units

Following the combining of the platelet donations pairs, the pooled platelet preparations are separated into four platelet units (in the case of #6 with #7), and four platelet units (in the case of #11 and #12). Together with the units (*e*.*g*., non-pooled units) from the remaining eight donors from Table 1, these methods again provide for an increased number of apheresis platelet units, with 25 total platelet units prepared, as compared to 23 platelet units that would result from convention apheresis collection and preparation methods (*e*.*g*., based on platelet units of at least 3×10¹¹). Analysis of a significantly larger apheresis platelet donation data set provides an opportunity to further highlight the improved preparation of a plurality of platelet units, with a significantly greater number of platelet units that may be prepared according to methods of the present disclosure, as compared to currently accepted apheresis collection practices for preparing platelet units.

### Example 3: Pathogen inactivation

The methods described in Example 2 may further include a pathogen inactivation treatment step. More specifically, the apheresis collected platelets are individually subjected to a pathogen inactivation process with a pathogen inactivating compound, using the commercially available INTERCEPT^{®} Blood System for platelets (Cerus Corporation, Concord, CA), according to manufacturer instructions. Following the pathogen inactivation treatment of the platelet donations from donors #6, 7, 11 and 12, the platelet donations are combined in pairs as set forth above, and the pooled platelet preparations are separated into three platelet units. Each of the other eight platelet donations are similarly subjected to the pathogen inactivation process, providing a plurality of pathogen inactivated platelet units. Alternatively, the pooled platelet preparation may be subjected to a pathogen inactivation process with a pathogen inactivating compound, using the commercially available INTERCEPT^{®} Blood System for platelets, together with a processing set, such as depicted in Figure 7 or Figure 8.

In another example, pools of two apheresis platelet donations (n=6) were prepared to contain between approximately 10.2×10¹¹ to 11.8×10¹¹ platelets in approximately 623 to 648 mL of 35% plasma/65% PAS-3 platelet additive solution. The pooled apheresis platelets were subjected to photochemical pathogen-inactivation with amotosalen and UVA, using a processing set as provided in the present disclosure (see *e.g.,* Figures 7 and 8) to generate three platelet units of at least 3×10¹¹ platelets from each of the two input apheresis platelet donations. The units were evaluated on Day 7 post-donation for product dose and volume retention, pH, and in vitro platelet quality (*e*.*g*., function) parameters correlating with platelet survival and recovery in vivo (*e.g..,* pH, normalized lactate, ATP, morphology score, ESC and HSR), and found to meet EDQM requirements and to be within the ranges for platelet units from single donors and pathogen-inactivated with the commercially available INTERCEPT^{®} Blood System for platelets.

### Example 4: Preparing further increased number of platelet units

The platelet preparation methods disclosed herein may be further improved by targeting increased apheresis platelet collections from available donors. For example, a maximum platelet yield is determined for the above twelve donors based on the previously described donor characteristics. In addition to the prior target yield and targeted products reproduced below from Table 1, the maximum yield platelet number is also illustrated in the following Table 2, as well as the additional targeted maximum product numbers that may be achieved by collecting at the maximum amount of platelets.

**Table 2.**

| Donor | Gender | Weight | Height | Pre-count | Target Yield | Targeted Products | Max Yield | Max Products |
|---|---|---|---|---|---|---|---|---|
| 1 | M | 185 | 75" | 375 | 10.96 | 3 | 11.74 | 3 |
| 2 | M | 167 | 71" | 257 | 7.20 | 2 | 9.39 | 2 |
| 3 | M | 170 | 70" | 246 | 6.38 | 2 | 8.16 | 2 |
| 4 | F | 202 | 66" | 433 | 10.50 | 3 | 11.52 | 3 |
| 5 | F | 165 | 63" | 309 | 7.80 | 2 | 9.09 | 2 |
| 6 | M | 210 | 70" | 309 | 8.75 | 2 | 11.56 | 3 |
| 7 | M | 175 | 72" | 205 | 5.21 | 1 | 7.21 | 2 |
| 8 | F | 165 | 64" | 441 | 7.00 | 2 | 9.90 | 3 |
| 9 | F | 135 | 67" | 220 | 4.50 | 1 | 6.05 | 1 |
| 10 | M | 212 | 73" | 234 | 8.00 | 2 | 9.49 | 2 |
| 11 | M | 140 | 67" | 190 | 4.86 | 1 | 5.81 | 1 |
| 12 | M | 155 | 66" | 362 | 9.15 | 2 | 9.83 | 3 |

As shown in Table 2, four additional platelet units may be prepared by increasing the apheresis collections to the maximum amount shown, increasing the total to 27 platelet units, as compared to 23 units with previous collection targets. Additionally, as provided in the present disclosure a plurality of even more platelet units may be prepared from the same twelve donors, based on the maximum collections and the methods previously described in Example 2. More specifically, following collection for maximum yield, the following pairs of platelet donations are combined:
Donors #9 and 11, each comprising greater than the number of platelets required to prepare one platelet unit, but less than the number of platelets required to prepare two platelet units.
Donors #2 and 5, each comprising greater than the number of platelets required to prepare two platelet units, but less than the number of platelets required to prepare three platelet units.

Following the combining of the platelet donations pairs, the pooled platelet preparations are separated into three platelet units (in the case of #9 with #11), and five platelet units (in the case of #2 and #5). Together with units (*e*.*g*., unpooled units) from the remaining eight donors, these methods provide for an increased number of apheresis platelet units, with 29 total platelet units prepared, as compared to 23 platelet units from conventional apheresis collection and preparation methods or 27 platelet units from convention apheresis collection at the maximum yield (*e*.*g*., based on platelet units of at least 3×10¹¹). Analysis of a significantly larger apheresis platelet donation data set provides an opportunity to further highlight the improved preparation of a plurality of platelet units, with a significantly greater number of platelet units that may be prepared according to methods of the present disclosure, as compared to currently accepted apheresis collection practices for preparing platelet units.

As described in Example 3, these platelet donations may be subjected to a pathogen inactivation process prior to combining, or alternatively after combining by subjecting the pooled platelet preparation to the pathogen inactivation process.

### Example 5: Preparing platelet units from inventory and new donations

The present disclosure also provides further improved methods for preparing a plurality of platelet units suitable for infusion. More specifically, in one embodiment, an inventory log of an inventory of apheresis-collected platelet preparations from at least a first donor is maintained. The following Table 3 illustrates a model inventory log of an inventory of such platelet preparations, and includes for each platelet preparation a unique identifier, a donor ABO blood type, a donation time and a quantity of platelets (×10¹¹) in the preparation.

**Table 3. Apheresis platelet inventory**

| Identifier | ABO | Date | Time | Pre-count | Platelets | Products |
|---|---|---|---|---|---|---|
| 15072347 | B | 23July2015 | 16:33 | 375 | 10.96 | 3 |
| 15072348 | O | 23July2015 | 16:42 | 257 | 7.20 | 2 |
| 15072349 | O | 23July2015 | 16:55 | 246 | 6.38 | 2 |
| 15072201 | A | 22July2015 | 10:42 | 433 | 10.50 | 3 |
| 15072202 | AB | 22July2015 | 10:58 | 309 | 7.80 | 2 |
| 15072203 | A | 22July2015 | 11:26 | 309 | 8.75 | 2 |
| 15072204 | B | 22July2015 | 11:32 | 205 | 5.21 | 1 |
| 15072205 | O | 22July2015 | 11:48 | 441 | 7.00 | 2 |
| 15072206 | AB | 22July2015 | 12:14 | 220 | 4.50 | 1 |
| 15072207 | A | 22July2015 | 12:22 | 234 | 8.00 | 2 |
| 15072208 | A | 22July2015 | 12:59 | 190 | 4.86 | 1 |
| 15072209 | O | 22July2015 | 13:16 | 362 | 9.15 | 2 |

An available donor of blood type A suitable for donating platelets by apheresis is identified and a platelet preparation in the inventory (#15072208, an extra-large single) is selected from the inventory log for combining with platelets collected from the available donor. A new platelet donation with target yield of 5.10×10¹¹ platelets (an extra-large single) is collected by apheresis from the available donor and combined with the selected platelet preparation from the inventory of apheresis-collected platelet preparations (*e*.*g*., within 6 hours) to yield a pooled platelet preparation comprising about 9.86×10¹¹ platelets. The pooled platelet preparation is separated into three platelet units, which is a greater number of platelet units than the two platelet units that can be prepared from selected platelet preparation and the new platelet donation without combining them as provided by the present disclosure. Similarly additional new platelet donations may be collected and combined with other platelet preparations, for example a new extra-large single platelet donation of blood type A to combine with inventory platelet preparation 15072203 and yield a pooled platelet preparation comprising sufficient platelets to generate 4 platelet units, a new extra-large double platelet donation of blood type B to combine with inventory platelet preparation 15072204 and yield a pooled platelet preparation comprising sufficient platelets to generate 4 platelet units, and a new extra-large double platelet donation of blood type O to combine with inventory platelet preparation 15072209 yield a pooled platelet preparation comprising sufficient platelets to generate 5 platelet units, to further increase the plurality of platelet units that may be obtained. For example, in the above scenario illustrated in this example, the 23 possible units from inventory may be combined with the four new platelet donations which would otherwise provide 6 new platelet units by conventional methods, to instead prepare 33 total platelet units (*e*.*g*., at least 3×10¹¹ per unit) based on the methods of the present disclosure. A pathogen inactivation process, such as described in Example 3 (*e*.*g*., photochemical pathogen-inactivation with amotosalen and UVA, and processing set as provided Figures 7 and 8), may optionally be performed prior to combining, or alternatively after combining by subjecting the pooled platelet preparation to the pathogen inactivation process.

The use of the terms "a" and "an" and "the" and similar referents (especially in the context of the following claims) are to be construed to cover both the singular and the plural, unless otherwise indicated herein or clearly contradicted by context. The terms "comprising," "having," "including," and "containing" are to be construed as open-ended terms (*i.e.,* meaning "including, but not limited to,") unless otherwise noted. Wherever an open-ended term is used to describe a feature or element, it is specifically contemplated that a closed-ended term can be used in place of the open-ended term without departing from the spirit and scope of the disclosure. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (*e*.*g*., "such as") provided herein, is intended merely to better illuminate the description and does not pose a limitation on the scope of the description unless otherwise claimed. No language in the specification should be construed as indicating any non-claimed element as essential to the practice of the methods, systems and compositions disclosed herein.

The invention is set out in the appended set of claims. Preferred embodiments are described herein. Variations of those preferred embodiments may become apparent to those working in the art upon reading the foregoing description. It is expected that skilled artisans will be able to employ such variations as appropriate, and the practice of the methods, systems and compositions described herein otherwise than as specifically described herein.

## Claims

1. A method of preparing a plurality of platelet units each comprising a therapeutic dose of platelets suitable for infusion into a human subject, said method comprising:
a) collecting a first platelet donation by apheresis from a first donor,
b) collecting a second platelet donation by apheresis from a second donor,
c) combining the first platelet donation and the second platelet donation to yield a pooled platelet preparation, and
d) separating the pooled platelet preparation into a plurality of platelet units each in an individual container, wherein the plurality of platelet units comprises a greater number of platelet units than the total number of platelet units that can be prepared from the first platelet donation and second platelet donation without combining the first and second platelet donations; wherein each platelet unit comprises a therapeutic dose of platelets.

2. The method of claim 1, wherein collecting the first platelet donation from the first donor comprises targeting the platelet collection to yield a first platelet donation comprising at least about 125% of the number of platelets required to prepare one platelet unit, but less than the number of platelets required to prepare two platelet units; and/or the first platelet donation comprises greater than the number of platelets required to prepare one platelet unit, but less than the number of platelets required to prepare two platelet units.

3. The method of claim 1, wherein collecting the first platelet donation from the first donor comprises targeting the platelet collection to yield a first platelet donation comprising at least about 225% of the number of platelets required to prepare one platelet unit, but less than the number of platelets required to prepare three platelet units; and/or the first platelet donation comprises greater than the number of platelets required to prepare two platelet units, but less than the number of platelets required to prepare three platelet units.

4. The method of claim 1, wherein collecting the first platelet donation from the first donor comprises targeting the platelet collection to yield a first platelet donation comprising at least about 325% of the number of platelets required to prepare one platelet unit, but less than the number of platelets required to prepare four platelet units; and/or the first platelet donation comprises greater than the number of platelets required to prepare three platelet units, but less than the number of platelets required to prepare four platelet units.

5. The method of any one of the preceding claims, wherein collecting the second platelet donation from the second donor comprises targeting the platelet collection to yield a second platelet donation comprising at least about 125% of the number of platelets required to prepare one platelet unit, but less than the number of platelets required to prepare two platelet units; and/or the second platelet donation comprises greater than the number of platelets required to prepare one platelet unit, but less than the number of platelets required to prepare two platelet units.

6. The method of any one of claims 1 to 4 wherein collecting the second platelet donation from the second donor comprises targeting the platelet collection to yield a second platelet donation comprising at least about 225% of the number of platelets required to prepare one platelet unit, but less than the number of platelets required to prepare three platelet units; and/or the second platelet donation comprises greater than the number of platelets required to prepare two platelet units, but less than the number of platelets required to prepare three platelet units.

7. The method of any one of claims 1 to 4 wherein collecting the second platelet donation from the second donor comprises targeting the platelet collection to yield a second platelet donation comprising at least about 325% of the number of platelets required to prepare one platelet unit, but less than the number of platelets required to prepare four platelet units.

8. The method of claim 2, wherein collecting the second platelet donation from the second donor comprises targeting the platelet collection to yield a second platelet donation comprising a sufficient number of platelets to prepare three platelet units after combining the first and second platelet donations and separating the resulting pooled platelet preparation into a plurality of platelet units; and/or the second platelet donation comprises a sufficient number of platelets that, when combined with the first platelet donation, yields a pooled platelet preparation comprising a sufficient number of platelets to separate into three platelet units.

9. The method of claim 3 wherein collecting the second platelet donation from the second donor comprises targeting the platelet collection to yield a second platelet donation comprising a sufficient number of platelets to prepare five platelet units after combining the first and second platelet donations and separating the resulting pooled platelet preparation into a plurality of platelet units; and/or the second platelet donation comprises a sufficient number of platelets that, when combined with the first platelet donation, yields a pooled platelet preparation comprising a sufficient number of platelets for separation into five platelet units.

10. The method of claim 4 wherein the second platelet donation comprises a sufficient number of platelets that, when combined with the first platelet donation, yields a pooled platelet preparation comprising a sufficient number of platelets to separate into seven platelet units.

11. The method of claim 5, wherein the first platelet donation comprises greater than the number of platelets required to prepare one platelet unit, but less than the number of platelets required to prepare two platelet units, and the second platelet donation comprises greater than the number of platelets required to prepare one platelet unit, but less than the number of platelets required to prepare two platelet units, optionally wherein the pooled platelet preparation comprises a sufficient number of platelets to prepare three platelet units, and/or optionally wherein the plurality of platelet units comprises three platelet units.

12. The method of claim 6 wherein the first platelet donation comprises greater than the number of platelets required to prepare two platelet units, but less than the number of platelets required to prepare three platelet units, and the second platelet donation comprises greater than the number of platelets required to prepare two platelet units, but less than the number of platelets required to prepare three platelet units, optionally wherein the pooled platelet preparation comprises a sufficient number of platelets to prepare five platelet units.

13. The method of any one of the preceding claims, further comprising collecting a third platelet donation by apheresis from a third donor, combining the third platelet donation with the first and the second platelet donations to yield a pooled platelet preparation, and separating the pooled platelet preparation into a plurality of platelet units each in an individual container, wherein the plurality of platelet units comprises a greater number of platelet units than the total number of platelet units that can be prepared from the first platelet donation, the second platelet donation and the third platelet donation without combining the first, second and third platelet donations, and/or wherein the second platelet donation is collected within 20 hours from the time the first platelet donation is collected, optionally wherein the second platelet donation is collected within 12 hours from the time the first platelet donation is collected.

14. The method of any one of the preceding claims, further comprising
(a) subjecting each of the first platelet donation and the second platelet donation to a pathogen inactivation process; or
(b) subjecting the pooled platelet preparation to a pathogen inactivation process; and optionally wherein subjecting the platelet donations or pooled platelet preparation to a pathogen inactivation process comprises treating the platelet donation or pooled platelet preparation with a pathogen inactivating compound; optionally wherein the pathogen inactivating compound is a photoactive pathogen inactivating compound selected from the group consisting of a psoralen, an isoalloxazine, an alloxazine, a phthalocyanine, a phenothiazine, a porphyrin, and merocyanine 540; and optionally wherein the photoactive pathogen inactivating compound is amotosalen.

15. The method of any one of the preceding claims, wherein the platelet units each comprise at least about 2.0×10¹¹ platelets.

16. The method of any one of claims 2 to 9, wherein targeting the platelet collection comprises one or more of determining an optimal platelet collection, providing an input to an apheresis system, and providing a donation volume to an apheresis system.

17. A method of preparing a plurality of platelet units, each unit comprising a therapeutic dose of platelets suitable for infusion into a human subject, said method comprising:
a) maintaining an inventory log of an inventory of apheresis-collected platelet preparations, the inventory log comprising for each platelet preparation a unique identifier, a donor blood type, a donation time and a quantity of platelets in the preparation,
b) identifying an available donor suitable for donating platelets by apheresis,
c) selecting from the inventory log at least one platelet preparation in the inventory from a first donor for combining with platelets collected from the available donor, wherein the first donor is different to the available donor,
d) collecting a new platelet donation by apheresis from the available donor,
e) combining the at least one selected platelet preparation from the inventory of apheresis-collected platelet preparations and the new platelet donation to yield a pooled platelet preparation, and
f) separating the pooled platelet preparation into a plurality of platelet units each in an individual container, wherein the plurality of platelet units comprises a greater number of platelet units than the total number of platelet units that can be prepared from the at least one selected platelet preparation from the inventory of apheresis-collected platelet preparations and the new platelet donation from the available donor without combining the at least one selected platelet preparation and new platelet donation, optionally wherein (g) the inventory of apheresis-collected platelet preparations comprises existing platelet preparations that have already been collected; and/or
(h) the at least one selected platelet preparation comprises greater than the number of platelets required to prepare one platelet unit, but less than the number of platelets required to prepare two platelet units; and/or
(i) the at least one selected platelet preparation comprises at least about 125% of the number of platelets required to prepare one platelet unit, but less than the number of platelets required to prepare two platelet units.

18. The method of any one of claims 17(a) to 17(g), wherein
(a) the at least one selected platelet preparation comprises greater than the number of platelets required to prepare two platelet units, but less than the number of platelets required to prepare three platelet units; or
(b) the at least one selected platelet preparation comprises greater than the number of platelets required to prepare three platelet units, but less than the number of platelets required to prepare four platelet units.

19. The method of any one of claims 17 (a) - 17(g), and 18, wherein:
(a) the at least one selected platelet preparation comprises at least about 225% of the number of platelets required to prepare one platelet unit, but less than the number of platelets required to prepare three platelet units; or
(b) the at least one selected platelet preparation comprises at least about 325% of the number of platelets required to prepare one platelet unit, but less than the number of platelets required to prepare four platelet units.

20. The method of any one of claims 17 to 19, wherein:
(a) collecting the new platelet donation comprises targeting the platelet collection to yield a donation comprising at least about 125% of the number of platelets required to prepare one platelet unit, but less than the number of platelets required to prepare two platelet units;
(b) the new platelet donation comprises greater than the number of platelets required to prepare one platelet unit, but less than the number of platelets required to prepare two platelet units;
(c) collecting the new platelet donation comprises targeting the platelet collection to yield a donation comprising at least about 225% of the number of platelets required to prepare one platelet unit, but less than the number of platelets required to prepare three platelet units;
(d) the new platelet donation comprises greater than the number of platelets required to prepare two platelet units, but less than the number of platelets required to prepare three platelet units; or
(e) the new platelet donation comprises greater than the number of platelets required to prepare three platelet units, but less than the number of platelets required to prepare four platelet units.

21. The method of claim 17 (h) or 17(i), wherein collecting the new platelet donation from the available donor comprises collecting a sufficient number of platelets to prepare three platelet units from the pooled preparation.

22. The method of claim 18 or claim 19, wherein:
(a) the method of claim 18(a) or 19 (a) applies, and collecting the new platelet donation from the available donor comprises collecting a sufficient number of platelets to prepare five platelet units from the pooled preparation; or
(b) the method of claim 18(b) or 19 (b) applies, and collecting the new platelet donation from the available donor comprises collecting a sufficient number of platelets to prepare seven platelet units from the pooled preparation.

23. The method of claim 20, wherein the method of claim 20(a) or claim 20(b) applies, and the at least one selected platelet preparation comprises greater than the number of platelets required to prepare one platelet unit, but less than the number of platelets required to prepare two platelet units, and the new platelet donation comprises greater than the number of platelets required to prepare one platelet unit, but less than the number of platelets required to prepare two platelet units, optionally wherein the plurality of platelet units comprises three platelet units.

24. The method of any one of claims 17-23, further comprising identifying a second available donor suitable for donating platelets by apheresis, collecting a second new platelet donation by apheresis from the second available donor, combining the second new platelet donation with the at least one selected platelet preparation from the inventory of apheresis-collected platelet preparations and the new platelet donation from the available donor to yield the pooled platelet preparation, and separating the pooled platelet preparation into a plurality of platelet units each in an individual container, wherein the plurality of platelet units comprises a greater number of platelet units than the total number of platelet units that can be prepared from the at least one selected platelet preparation, the new platelet donation and the second new platelet donation without combining the at least one selected platelet preparation, the new platelet donation and the second new platelet donation; and/or wherein the new platelet donation is collected within 12 hours from the time the at least one selected platelet preparation is collected.

25. The method of any one of claims 17-24, further comprising:
(a) subjecting each of the at least one selected platelet preparation and the new platelet donation to a pathogen inactivation process, optionally wherein subjecting each of the at least one selected platelet preparation and the new platelet donation to a pathogen inactivation process comprises treating each of the at least one selected platelet preparation and the new platelet donation with a pathogen inactivating compound; or
(b) subjecting the pooled platelet preparation to a pathogen inactivation process, optionally wherein subjecting the pooled platelet preparation to a pathogen inactivation process comprises treating the pooled platelet preparation with a pathogen inactivating compound, optionally wherein the pathogen inactivating compound is a photoactive pathogen inactivating compound selected from the group consisting of a psoralen, an isoalloxazine, an alloxazine, a phthalocyanine, a phenothiazine, a porphyrin, and merocyanine 540; and, optionally, wherein the photoactive pathogen inactivating compound is amotosalen.

26. The method of any one of claims 17 to 25, wherein the platelet units in the plurality of platelet units each comprise a therapeutic dose of platelets suitable for infusion into a human subject; and optionally wherein the platelet units each comprise at least about 2.0×10¹¹ platelets.

27. The method of claim 20(a) or claim 20(c), wherein targeting the platelet collection comprises one or more of determining an optimal platelet collection, providing an input to an apheresis system, and providing a donation volume to an apheresis system.

28. The method of claim 17, further comprising:
adetermining a target volume of the new platelet donation from the available donor, wherein the target volume optimizes the number of platelet units that can be prepared from a combination comprising the new platelet donation and the at least one selected platelet preparations from the inventory of apheresis-collected platelet preparations; and
b) collecting the target volume of the new platelet donation by apheresis from the available donor;
c) combining the at least one selected platelet preparations from the inventory of apheresis-collected platelet preparations and the new platelet donation from the available donor to yield a pooled platelet preparation; and
d) separating the pooled platelet preparation into a plurality of platelet units each in an individual container; wherein each of the platelet units in the plurality of platelet units comprises a therapeutic dose suitable for infusion into a human subject; and wherein the plurality of platelet units comprises a greater number of platelet units than the total number of platelet units that can be prepared from the at least one selected platelet preparations from the inventory of apheresis-collected platelet preparations and the new platelet donation from the available donor without combining the one or more selected platelet preparations and the new platelet donation.

29. The method of claim 28, wherein:
(a) the one or more selected platelet preparations comprises greater than the number of platelets required to prepare one platelet unit, but less than the number of platelets required to prepare two platelet units, optionally wherein collecting the new platelet donation from the available donor comprises collecting a sufficient number of platelets to prepare three platelet units from the pooled preparation;
(b) the one or more selected platelet preparations comprises at least about 125% of the number of platelets required to prepare one platelet unit, but less than the number of platelets require to prepare two platelet units; optionally wherein collecting the new platelet donation from the available donor comprises collecting a sufficient number of platelets to prepare three platelet units from the pooled preparation; or
(c) the one or more selected platelet preparations comprises greater than the number of platelets required to prepare two platelet units, but less than the number of platelets required to prepare three platelet units; optionally wherein collecting the new platelet donation from the available donor comprises collecting a sufficient number of platelets to prepare five platelet units from the pooled preparation.

30. The method of claim 28, wherein
(a) each of the one or more selected platelet preparations and the new platelet donation comprises greater than the number of platelets required to prepare one therapeutic dose, but less than the number of platelets required to prepare two therapeutic doses; or
(b) each of the one or more selected platelet preparations and the new platelet donation comprises greater than the number of platelets required to prepare two therapeutic dosage units of platelets, but less than the number of platelets required to prepare three therapeutic dosage units of platelets.

31. The method of any one of claims 28-30, wherein the one or more selected platelet preparations and the new platelet donation are combined within 12 hours from the time of collection; and/or wherein each platelet unit comprises at least 2.0×10¹¹ platelets; and/or the method further comprising:
(a) subjecting the pooled platelet preparation to a pathogen inactivation process; optionally wherein subjecting the pooled platelet preparation to a pathogen inactivation process comprises treating the pooled platelet preparation with a pathogen inactivating compound; or
(b) subjecting each of the one or more selected platelet preparations and the new platelet donation to a pathogen inactivation process; optionally wherein subjecting the one or more selected platelet preparations and the new platelet donation to a pathogen inactivation process comprises treating the one or more selected platelet preparations and the new platelet donation with a pathogen inactivating compound; optionally wherein the pathogen inactivating compound is a photoactive pathogen inactivating compound selected from the group consisting of a psoralen, an isoalloxazine, an alloxazine, a phthalocyanine, a phenothiazine, a porphyrin, and merocyanine 540; and optionally wherein the photoactive pathogen inactivating compound is amotosalen.

## Patentansprüche

1. Verfahren zur Herstellung einer Vielzahl von Plättcheneinheiten, die jeweils eine therapeutische Dosis von Plättchen umfasst, die zur Infusion in ein menschliches Individuum geeignet ist, wobei das Verfahren Folgendes umfasst:
a) Abnehmen einer ersten Plättchenspende durch Apherese von einem ersten Spender,
b) Abnehmen einer zweiten Plättchenspende durch Apherese von einem zweiten Spender,
c) Vereinigen der ersten Plättchenspende und der zweiten Plättchenspende, um ein gepooltes Plättchenpräparat zu ergeben, und
d) Trennen des gepoolten Plättchenpräparats in eine Vielzahl von Plättcheneinheiten in jeweils einem eigenen Behälter, wobei die Vielzahl von Plättcheneinheiten eine größere Anzahl von Plättcheneinheiten umfasst als die Gesamtanzahl von Plättcheneinheiten, die aus der ersten Plättchenspende und der zweiten Plättchenspende ohne Vereinigen der ersten und der zweiten Plättchenspende hergestellt werden kann; wobei jede Plättcheneinheit eine therapeutische Dosis von Plättchen umfasst.

2. Verfahren nach Anspruch 1, wobei das Abnehmen der ersten Plättchenspende von dem ersten Spender das Ausrichten der Plättchenabnahme umfasst, um eine erste Plättchenspende zu ergeben, die zumindest etwa 125 % der Anzahl von Plättchen, die erforderlich sind, um eine Plättcheneinheit herzustellen, aber weniger als die Anzahl von Plättchen umfasst, die erforderlich ist, um zwei Plättcheneinheiten herzustellen; und/oder wobei die erste Plättchenspende mehr als die Anzahl von Plättchen, die erforderlich ist, um eine Plättcheneinheit herzustellen, aber weniger als die Anzahl von Plättchen umfasst, die erforderlich ist, um zwei Plättcheneinheiten herzustellen.

3. Verfahren nach Anspruch 1, wobei das Abnehmen der ersten Plättchenspende von dem ersten Spender das Ausrichten der Plättchenabnahme umfasst, um eine erste Plättchenspende zu ergeben, die zumindest etwa 225 % der Anzahl von Plättchen, die erforderlich sind, um eine Plättcheneinheit herzustellen, aber weniger als die Anzahl von Plättchen umfasst, die erforderlich ist, um drei Plättcheneinheiten herzustellen; und/oder wobei die erste Plättchenspende mehr als die Anzahl von Plättchen, die erforderlich ist, um zwei Plättcheneinheit herzustellen, aber weniger als die Anzahl von Plättchen umfasst, die erforderlich ist, um drei Plättcheneinheiten herzustellen.

4. Verfahren nach Anspruch 1, wobei das Abnehmen der ersten Plättchenspende von dem ersten Spender das Ausrichten der Plättchenabnahme umfasst, um eine erste Plättchenspende zu ergeben, die zumindest etwa 325 % der Anzahl von Plättchen, die erforderlich sind, um eine Plättcheneinheit herzustellen, aber weniger als die Anzahl von Plättchen umfasst, die erforderlich ist, um vier Plättcheneinheiten herzustellen; und/oder wobei die erste Plättchenspende mehr als die Anzahl von Plättchen, die erforderlich ist, um drei Plättcheneinheit herzustellen, aber weniger als die Anzahl von Plättchen umfasst, die erforderlich ist, um vier Plättcheneinheiten herzustellen.

5. Verfahren nach einem der vorangegangenen Ansprüche, wobei das Abnehmen der zweiten Plättchenspende von dem zweiten Spender das Ausrichten der Plättchenabnahme umfasst, um eine zweite Plättchenspende zu ergeben, die zumindest etwa 125 % der Anzahl von Plättchen, die erforderlich sind, um eine Plättcheneinheit herzustellen, aber weniger als die Anzahl von Plättchen umfasst, die erforderlich ist, um zwei Plättcheneinheiten herzustellen; und/oder wobei die zweite Plättchenspende mehr als die Anzahl von Plättchen, die erforderlich ist, um eine Plättcheneinheit herzustellen, aber weniger als die Anzahl von Plättchen umfasst, die erforderlich ist, um zwei Plättcheneinheiten herzustellen.

6. Verfahren nach einem der Ansprüche 1 bis 4, wobei das Abnehmen der zweiten Plättchenspende von dem zweiten Spender das Ausrichten der Plättchenabnahme umfasst, um eine zweite Plättchenspende zu ergeben, die zumindest etwa 225 % der Anzahl von Plättchen, die erforderlich sind, um eine Plättcheneinheit herzustellen, aber weniger als die Anzahl von Plättchen umfasst, die erforderlich ist, um drei Plättcheneinheiten herzustellen; und/oder wobei die zweite Plättchenspende mehr als die Anzahl von Plättchen, die erforderlich ist, um zwei Plättcheneinheit herzustellen, aber weniger als die Anzahl von Plättchen umfasst, die erforderlich ist, um drei Plättcheneinheiten herzustellen.

7. Verfahren nach einem der Ansprüche 1 bis 4, wobei das Abnehmen der zweiten Plättchenspende von dem zweiten Spender das Ausrichten der Plättchenabnahme umfasst, um eine zweite Plättchenspende zu ergeben, die zumindest etwa 325 % der Anzahl von Plättchen, die erforderlich sind, um eine Plättcheneinheit herzustellen, aber weniger als die Anzahl von Plättchen umfasst, die erforderlich ist, um vier Plättcheneinheiten herzustellen.

8. Verfahren nach Anspruch 2, wobei das Abnehmen der zweiten Plättchenspende von dem zweiten Spender das Ausrichten der Plättchenabnahme umfasst, um eine zweite Plättchenspende zu ergeben, die eine ausreichende Anzahl von Plättchen umfasst, um drei Plättcheneinheiten nach dem Vereinigen der ersten und der zweiten Plättchenspende und nach dem Trennen des resultierenden gepoolten Plättchenpräparats in eine Vielzahl von Plättcheneinheiten herzustellen; und/oder wobei die zweite Plättchenspende eine ausreichende Anzahl von Plättchen umfasst, die, wenn sie mit der ersten Plättchenspende vereinigt wird, ein gepooltes Plättchenpräparat ergibt, das eine ausreichende Anzahl von Plättchen umfasst, um in drei Plättcheneinheiten aufgetrennt zu werden.

9. Verfahren nach Anspruch 3, wobei das Abnehmen der zweiten Plättchenspende von dem zweiten Spender das Ausrichten der Plättchenabnahme umfasst, um eine zweite Plättchenspende zu ergeben, die eine ausreichende Anzahl von Plättchen umfasst, um fünf Plättcheneinheiten nach dem Vereinigen der ersten und der zweiten Plättchenspende und nach dem Trennen des resultierenden gepoolten Plättechenpräparats in eine Vielzahl von Plättcheneinheiten herzustellen; und/oder wobei die zweite Plättchenspende eine ausreichende Anzahl von Plättchen umfasst, die, wenn sie mit der ersten Plättchenspende vereinigt wird, ein gepooltes Plättchenpräparat ergibt, das eine ausreichende Anzahl von Plättchen umfasst, um in fünf Plättcheneinheiten aufgetrennt zu werden.

10. Verfahren nach Anspruch 4, wobei die zweite Plättchenspende eine ausreichende Anzahl von Plättchen umfasst, die, wenn sie mit der ersten Plättchenspende vereinigt wird, ein gepooltes Plättchenpräparat ergibt, das eine ausreichende Anzahl von Plättchen umfasst, um in sieben Plättcheneinheiten aufgetrennt zu werden.

11. Verfahren nach Anspruch 5, wobei die erste Plättchenspende mehr als die Anzahl von Plättchen, die erforderlich ist, um eine Plättcheneinheit herzustellen, aber weniger als die Anzahl von Plättchen umfasst, die erforderlich ist, um zwei Plättcheneinheiten herzustellen, und wobei die zweite Plättchenspende mehr als die Anzahl von Plättchen, die erforderlich ist, um eine Plättcheneinheit herzustellen, aber weniger als die Anzahl von Plättchen umfasst, die erforderlich ist, um zwei Plättcheneinheiten herzustellen, wobei das gepoolte Plättchenpräparat gegebenenfalls eine ausreichende Anzahl von Plättchen umfasst, um drei Plättcheneinheiten herzustellen, und/oder wobei die Vielzahl von Plättcheneinheiten gegebenenfalls drei Plättcheneinheiten umfasst.

12. Verfahren nach Anspruch 6, wobei die erste Plättchenspende mehr als die Anzahl von Plättchen, die erforderlich ist, um zwei Plättcheneinheiten herzustellen, aber weniger als die Anzahl von Plättchen umfasst, die erforderlich ist, um drei Plättcheneinheiten herzustellen, und wobei die zweite Plättchenspende mehr als die Anzahl von Plättchen, die erforderlich ist, um zwei Plättcheneinheiten herzustellen, aber weniger als die Anzahl von Plättchen umfasst, die erforderlich ist, um drei Plättcheneinheiten herzustellen, wobei das gepoolte Plättchenpräparat gegebenenfalls eine ausreichende Anzahl von Plättchen umfasst, um fünf Plättcheneinheiten herzustellen.

13. Verfahren nach einem der vorangegangenen Ansprüche, das weiters das Abnehmen einer dritten Plättchenspende durch Apherese von einem dritten Spender, das Vereinigen der dritten Plättchenspende mit der ersten und der zweiten Plättchenspende zum Ergeben eines gepoolten Plättchenpräparats und das Auftrennen des gepoolten Plättchenpräparats in eine Vielzahl von Plättcheneinheiten jeweils in einen eigenen Behälter umfasst, wobei die Vielzahl von Plättcheneinheiten eine größere Anzahl von Plättcheneinheiten als die Gesamtanzahl von Plättcheneinheiten, die aus der ersten Plättchenspende, der zweiten Plättchenspende und der dritten Plättchenspende ohne Vereinigen der ersten, der zweiten und der dritten Plättchenspende hergestellt werden können, umfasst, und/oder wobei die zweite Plättchenspende innerhalb von 20 h ab dem Zeitpunkt abgenommen wird, zu dem die erste Plättchenspende abgenommen wird, wobei die zweite Plättchenspende gegebenenfalls innerhalb von 12 h ab dem Zeitpunkt abgenommen wird, zu dem die erste Plättchenspende abgenommen wird.

14. Verfahren nach einem der vorangegangenen Ansprüche, das weiters Folgendes umfasst:
(a) Unterziehen jeder aus der ersten Plättchenspende und der zweiten Plättchenspende einem Pathogeninaktivierungsverfahren; oder
(b) Unterziehen des gepoolten Plättchenpräparats einem Pathogeninaktivierungsverfahren; und
Wobei das Unterziehen der Plättchenspenden oder des gepoolten Plättchenpräparats einem Pathogeninaktivierungsverfahren gegebenenfalls das Versetzen der Plättchenspende oder des gepoolten Plättchenpräparats mit einer Pathogeninaktivierungsverbindung umfasst; wobei die Pathogeninaktivierungsverbindung gegebenenfalls eine photoaktive Pathogeninaktivierungsverbindung ist, die aus der aus einem Psoralen, einem Isoalloxazin, einem Alloxazin, einem Phthalocyanin, einem Phenothiazin, einem Porphyrin und Merocyanin 540 bestehenden Gruppe ausgewählt ist; und wobei die photoaktive Pathogeninaktivierungsverbindung gegebenenfalls Amotosalen ist.

15. Verfahren nach einem der vorangegangenen Ansprüche, wobei die Plättcheneinheiten jeweils zumindest etwa 2,0 × 10¹¹ Plättchen umfassen.

16. Verfahren nach einem der Ansprüche 2 bis 9, wobei das Ausrichten der Plättchenabnahme eines oder mehrere aus dem Bestimmen einer optimalen Plättchenabnahme, dem Bereitstellen eines Eingangs in ein Apherese-System und dem Bereitstellen eines Spendenvolumens an einem Apherese-System umfasst.

17. Verfahren zur Herstellung einer Vielzahl von Plättcheneinheiten, wobei jede Einheit eine therapeutische Dosis von Plättchen umfasst, die zur Infusion in ein menschliches Individuum geeignet sind, wobei das Verfahren Folgendes umfasst:
a) Führen eines Inventarprotokolls eines Inventars von durch Apherese abgenommenen Plättchenpräparaten, wobei das Inventarprotokoll für jedes Plättchenpräparat einen eindeutigen Identifikator, eine Spenderblutgruppe, einen Spendezeitpunkt und eine Menge von Plättchen in dem Präparat umfasst,
b) Identifizieren eines verfügbaren Spenders, der zum Spenden von Plättchen durch Apherese geeignet ist,
c) Auswählen aus dem Inventarprotokoll zumindest eines Plättchenpräparats in dem Inventar von einem ersten Spender zum Vereinigen mit Plättchen, die von dem verfügbaren Spender abgenommen wurden, wobei der erste Spender ein anderer ist als der verfügbare Spender,
d) Abnehmen einer neuen Plättchenspende durch Apherese von dem verfügbaren Spender,
e) Vereinigen des zumindest einen ausgewählten Plättchenpräparats aus dem Inventar von durch Apherese abgenommenen Plättchenpräparaten und der neuen Plättchenspende, um ein gepooltes Plättchenpräparat zu erhalten, und
f) Auftrennen des gepoolten Plättchenpräparats in eine Vielzahl von Plättcheneinheiten jeweils in einem eigenen Behälter, wobei die Vielzahl von Plättcheneinheiten eine größere Anzahl von Plättcheneinheiten als die Gesamtanzahl von Plättcheneinheiten umfasst, die aus dem zumindest einen ausgewählten Plättchenpräparat aus dem Inventar von durch Apherese abgenommenen Plättchenpräparaten und der neuen Plättchenspende von dem verfügbaren Spender ohne Vereinigen des zumindest einen ausgewählten Plättchenpräparats und der neuen Plättchenspende hergestellt werden können, wobei gegebenenfalls:
g) das Inventar von durch Apherese abgenommenen Plättchenpräparaten bereits existierende Plättchenpräparate umfasst, die bereits abgenommen wurden; und/oder
h) das zumindest eine ausgewählte Plättchenpräparat mehr als die Anzahl von Plättchen, die erforderlich ist, um eine Plättcheneinheit herzustellen, aber weniger als die Anzahl von Plättchen umfasst, die erforderlich ist, um zwei Plättcheneinheiten herzustellen; und/oder
i) das zumindest eine ausgewählte Plättchenpräparat zumindest etwa 125 % der Anzahl von Plättchen, die erforderlich ist, um eine Plättcheneinheit herzustellen, aber weniger als die Anzahl von Plättchen umfasst, die erforderlich ist, um zwei Plättcheneinheiten herzustellen.

18. Verfahren nach einem der Ansprüche 17(a) bis 17(g), wobei:
(a) das zumindest eine ausgewählte Plättchenpräparat mehr als die Anzahl von Plättchen, die erforderlich ist, um zwei Plättcheneinheiten herzustellen, aber weniger als die Anzahl von Plättchen umfasst, die erforderlich ist, um drei Plättcheneinheiten herzustellen; oder
(b) das zumindest eine ausgewählte Plättchenpräparat mehr als die Anzahl von Plättchen, die erforderlich ist, um drei Plättcheneinheiten herzustellen, aber weniger als die Anzahl von Plättchen umfasst, die erforderlich ist, um vier Plättcheneinheiten herzustellen.

19. Verfahren nach einem der Ansprüche 17(a) bis 17(g) und 18, wobei:
(a) das zumindest eine ausgewählte Plättchenpräparat zumindest etwa 225 % der Anzahl von Plättchen, die erforderlich ist, um eine Plättcheneinheit herzustellen, aber weniger als die Anzahl von Plättchen umfasst, die erforderlich ist, um drei Plättcheneinheiten herzustellen; oder
(b) das zumindest eine ausgewählte Plättchenpräparat zumindest etwa 325 % der Anzahl von Plättchen, die erforderlich ist, um ein Plättcheneinheiten herzustellen, aber weniger als die Anzahl von Plättchen umfasst, die erforderlich ist, um vier Plättcheneinheiten herzustellen.

20. Verfahren nach einem der Ansprüche 17 bis 19, wobei:
(a) das Abnehmen der neuen Plättchenspende das Ausrichten der Plättchenabnahme zum Erhalten einer Spende umfasst, die zumindest etwa 125 % der Anzahl von Plättchen, die erforderlich ist, um eine Plättcheneinheit herzustellen, aber weniger als die Anzahl von Plättchen umfasst, die erforderlich ist, um zwei Plättcheneinheiten herzustellen;
(b) die neue Plättchenspende mehr als die Anzahl von Plättchen, die erforderlich ist, um eine Plättcheneinheit herzustellen, aber weniger als die Anzahl von Plättchen umfasst, die erforderlich ist, um zwei Plättcheneinheiten herzustellen;
(c) das Abnehmen der neuen Plättchenspende das Ausrichten der Plättchenabnahme zum Erhalten einer Spende umfasst, die zumindest etwa 225 % der Anzahl von Plättchen, die erforderlich ist, um eine Plättcheneinheit herzustellen, aber weniger als die Anzahl von Plättchen umfasst, die erforderlich ist, um drei Plättcheneinheiten herzustellen;
(d) die neue Plättchenspende mehr als die Anzahl von Plättchen, die erforderlich ist, um zwei Plättcheneinheiten herzustellen, aber weniger als die Anzahl von Plättchen umfasst, die erforderlich ist, um drei Plättcheneinheiten herzustellen; oder
(e) die neue Plättchenspende mehr als die Anzahl von Plättchen, die erforderlich ist, um drei Plättcheneinheiten herzustellen, aber weniger als die Anzahl von Plättchen umfasst, die erforderlich ist, um vier Plättcheneinheiten herzustellen.

21. Verfahren nach Anspruch 17(h) oder 17(i), wobei das Abnehmen der neuen Plättchenspende von dem verfügbaren Spender das Abnehmen einer ausreichenden Anzahl von Plättchen umfasst, um drei Plättcheneinheiten aus dem gepoolten Präparat herzustellen.

22. Verfahren nach Anspruch 18 oder Anspruch 19, wobei:
(a) ein Verfahren nach Anspruch 18(a) oder 19(a) anwendbar ist und das Abnehmen der neuen Plättchenspende von dem verfügbaren Spender das Abnehmen einer ausreichenden Anzahl von Plättchen umfasst, um fünf Plättcheneinheiten aus dem gepoolten Präparat herzustellen; oder
(b) ein Verfahren nach Anspruch 18(b) oder 19(b) anwendbar ist und das Abnehmen der neuen Plättchenspende von dem verfügbaren Spender das Abnehmen einer ausreichenden Anzahl von Plättchen umfasst, um sieben Plättcheneinheiten aus dem gepoolten Präparat herzustellen.

23. Verfahren nach Anspruch 20, wobei ein Verfahren nach Anspruch 20(a) oder Anspruch 20(b) anwendbar ist und das zumindest eine ausgewählte Plättchenpräparat mehr als die Anzahl von Plättchen, die erforderlich ist, um eine Plättcheneinheit herzustellen, aber weniger als die Anzahl von Plättchen umfasst, die erforderlich ist, um zwei Plättcheneinheiten herzustellen, und wobei die neue Plättchenspende mehr als die Anzahl von Plättchen, die erforderlich ist, um eine Plättcheneinheit herzustellen, aber weniger als die Anzahl von Plättchen umfasst, die erforderlich ist, um zwei Plättcheneinheiten herzustellen, wobei die Vielzahl von Plättcheneinheiten gegebenenfalls drei Plättcheneinheiten umfasst.

24. Verfahren nach einem der Ansprüche 17 bis 23, das weiters das Identifizieren eines zweiten Spenders, der zum Spenden von Plättchen durch Apherese geeignet ist, das Abnehmen einer zweiten neuen Plättchenspende durch Apherese von dem zweiten verfügbaren Spender, das Vereinigen der zweiten neuen Plättchenspende mit dem zumindest einen ausgewählten Plättchenpräparat aus dem Inventar von durch Apherese abgenommenen Plättchenpräparaten und der neuen Plättchenspende von dem verfügbaren Spender, um das gepoolte Plättchenpräparat zu erhalten, und das Auftrennen des gepoolten Plättchenpräparats in eine Vielzahl von Plättcheneinheiten jeweils in einem eigenen Behälter umfasst, wobei die Vielzahl von Plättcheneinheiten eine größere Anzahl von Plättcheneinheiten umfasst als die Gesamtanzahl von Plättcheneinheiten, die aus dem zumindest einen ausgewählten Plättchenpräparat, der neuen Plättchenspende und der zweiten neuen Plättchenspende ohne Vereinigen des zumindest einen ausgewählten Plättchenpräparats, der neuen Plättchenspende und der zweiten neuen Plättchenspende hergestellt werden kann; und/oder wobei die neue Plättchenspende innerhalb von 12 h ab dem Zeitpunkt abgenommen wird, zu dem das zumindest eine ausgewählte Plättchenpräparat abgenommen wurde.

25. Verfahren nach einem der Ansprüche 17 bis 24, das weiters Folgendes umfasst:
(a) Unterziehen jedes einzelnen aus dem zumindest einen ausgewählten Plättchenpräparat und der neuen Plättchenspende einem Pathogeninaktivierungsverfahren, wobei das Unterziehen jedes einzelnen aus dem zumindest einen ausgewählten Plättchenpräparat und der neuen Plättchenspende einem Pathogeninaktivierungsverfahren gegebenenfalls das Versetzen jeder einzelnen aus dem zumindest einen ausgewählten Plättchenpräparat und der neuen Plättchenspende mit einer Pathogeninaktivierungsverbindung umfasst; oder
(b) Unterziehen des gepoolten Plättchenpräparats einem Pathogeninaktivierungsverfahren; wobei das Unterziehen des gepoolten Plättchenpräparats einem Pathogeninaktivierungsverfahren gegebenenfalls das Versetzen des gepoolten Plättchenpräparats mit einer Pathogeninaktivierungsverbindung umfasst, wobei die Pathogeninaktivierungsverbindung gegebenenfalls eine photoaktive Pathogeninaktivierungsverbindung ist, die aus der aus einem Psoralen, einem Isoalloxazin, einem Alloxazin, einem Phthalocyanin, einem Phenothiazin, einem Porphyrin und Merocyanin 540 bestehenden Gruppe ausgewählt ist; und wobei die photoaktive Pathogeninaktivierungsverbindung gegebenenfalls Amotosalen ist.

26. Verfahren nach einem der Ansprüche 17 bis 25, wobei die Plättcheneinheiten in der Vielzahl von Plättcheneinheiten jeweils eine therapeutische Dosis von Plättchen umfassen, die zur Infusion in ein menschliches Individuum geeignet ist; und wobei die Plättcheneinheiten gegebenenfalls jeweils zumindest etwa 2,0 × 10¹¹ Plättchen umfassen.

27. Verfahren nach Anspruch 20(a) oder Anspruch 20(c), wobei das Ausrichten der Plättchenabnahme eines oder mehrere aus dem Bestimmen einer optimalen Plättchenabnahme, dem Bereitstellen eines Eingangs in ein Apherese-System und dem Bereitstellen eines Spendervolumens an einem Apherese-System umfasst.

28. Verfahren nach Anspruch 17, das weiters Folgendes umfasst:
a) Bestimmen eines Zielvolumens der neuen Plättchenspende von dem verfügbaren Spender, wobei das Zielvolumen die Anzahl von Plättcheneinheiten optimiert, die aus einer Vereinigung, umfassend die neue Plättchenspende und das zumindest eine ausgewählte Plättchenpräparat aus dem Inventar von durch Apherese abgenommenen Plättchenpräparaten, hergestellt werden können; und
b) Abnehmen des Zielvolumens der neuen Plättchenspende durch Apherese von dem verfügbaren Spender;
c) Vereinigen des zumindest einen ausgewählten Plättchenpräparats aus dem Inventar von durch Apherese abgenommenen Plättchenpräparaten und der neuen Plättchenspender von dem verfügbaren Spender, um ein gepooltes Plättchenpräparat zu erhalten; und
d) Auftrennen des gepoolten Plättchenpräparats in eine Vielzahl von Plättcheneinheiten jeweils in einem eigenen Behälter; wobei jede der Plättcheneinheiten in der Vielzahl von Plättcheneinheiten eine therapeutische Dosis umfasst, die zur Infusion in ein menschliches Individuum geeignet ist; und wobei die Vielzahl von Plättcheneinheiten eine größere Anzahl von Plättcheneinheiten als die Gesamtanzahl von Plättcheneinheiten umfasst, die aus dem zumindest einen ausgewählten Plättchenpräparat aus dem Inventar von durch Apherese abgenommenen Plättchenpräparaten und der neuen Plättchenspende von dem verfügbaren Spender ohne Vereinigen des einen oder der mehreren ausgewählten Plättchenpräparate und der neuen Plättchenspende hergestellt werden kann.

29. Verfahren nach Anspruch 28, wobei:
(a) das eine oder die mehreren ausgewählten Plättchenpräparate mehr als die Anzahl von Plättchen, die erforderlich ist, um eine Plättcheneinheit herzustellen, aber weniger als die Anzahl von Plättchen umfasst, die erforderlich ist, um zwei Plättcheneinheiten herzustellen, wobei das Abnehmen der neuen Plättchenspende von dem verfügbaren Spender gegebenenfalls das Abnehmen einer ausreichenden Menge von Plättchen umfasst, um drei Plättcheneinheiten aus dem gepoolten Präparat herzustellen;
(b) das eine oder die mehreren Plättchenpräparate zumindest etwa 125 % der Anzahl von Plättchen, die erforderlich ist, um eine Plättcheneinheit herzustellen, aber weniger als die Anzahl von Plättchen umfasst, die erforderlich ist, um zwei Plättcheneinheiten herzustellen; wobei das Abnehmen der neuen Plättchenspende von dem verfügbaren Spender gegebenenfalls das Abnehmen einer ausreichenden Anzahl von Plättchen umfasst, um drei Plättcheneinheiten aus dem gepoolten Präparat herzustellen; oder
(c) das eine oder die mehreren Plättchenpräparate mehr als die Anzahl von Plättchen, die erforderlich ist, um zwei Plättcheneinheiten herzustellen, aber weniger als die Anzahl von Plättchen umfasst, die erforderlich ist, um drei Plättcheneinheiten herzustellen; wobei das Abnehmen der neuen Plättchenspende von dem verfügbaren Spender gegebenenfalls das Abnehmen einer ausreichenden Anzahl von Plättchen umfasst, um fünf Plättcheneinheiten aus dem gepoolten Präparat herzustellen.

30. Verfahren nach Anspruch 28, wobei:
(a) jedes aus dem einen oder den mehreren ausgewählten Plättchenpräparaten und der neuen Plättchenspende mehr als die Anzahl von Plättchen, die erforderlich ist, um eine therapeutische Dosis herzustellen, aber weniger als die Anzahl von Plättchen umfasst, die erforderlich ist, um zwei therapeutische Dosen herzustellen; oder
(b) jedes aus dem einen oder den mehreren ausgewählten Plättchenpräparaten und der neuen Plättchenspende mehr als die Anzahl von Plättchen, die erforderlich ist, um zwei therapeutische Dosierungseinheiten von Plättchen herzustellen, aber weniger als die Anzahl von Plättchen umfasst, die erforderlich ist, um drei therapeutische Dosierungseinheiten von Plättchen herzustellen.

31. Verfahren nach einem der Ansprüche 28 bis 30, wobei das eine oder die mehreren ausgewählten Plättchenpräparate und die neue Plättchenspende innerhalb von 12 h ab dem Zeitpunkt des Abnehmens vereinigt werden; und/oder wobei jede Plättcheneinheit zumindest 2,0 × 10¹¹ Plättchen umfasst; und/oder wobei das Verfahren weiters Folgendes umfasst:
(a) Unterziehen des gepoolten Plättchenpräparats einem Pathogeninaktivierungsverfahren, wobei das Unterziehen des gepoolten Plättchenpräparats einem Pathogeninaktivierungsverfahren gegebenenfalls das Versetzen des gepoolten Plättchenpräparats mit einer Pathogeninaktivierungsverbindung umfasst; oder
(b) Unterziehen jedes einzelnen aus dem einen oder den mehreren ausgewählten Plättchenpräparaten und der neuen Plättchenspende einem Pathogeninaktivierungsverfahren; wobei das Unterziehen jeder einzelnen aus dem einen oder den mehreren ausgewählten Plättchenpräparaten und der neuen Plättchenspende einem Pathogeninaktivierungsverfahren gegebenenfalls das Versetzen des einen oder der mehreren ausgewählten Plättchenpräparate und der neuen Plättchenspende mit einer Pathogeninaktivierungsverbindung umfasst; wobei die Pathogeninaktivierungsverbindung gegebenenfalls eine photoaktive Pathogeninaktivierungsverbindung ist, die aus der aus einem Psoralen, einem Isoalloxazin, einem Alloxazin, einem Phthalocyanin, einem Phenothiazin, einem Porphyrin und Merocyanin 540 bestehenden Gruppe ausgewählt ist; und wobei die photoaktive Pathogeninaktivierungsverbindung gegebenenfalls Amotosalen ist.

## Revendications

1. Procédé de préparation d'une pluralité d'unités de plaquettes comprenant chacune une dose thérapeutique de plaquettes appropriée pour une perfusion chez un sujet humain, ledit procédé comprenant :
a) la collecte d'un premier don de plaquettes par aphérèse à partir d'un premier donneur,
b) la collecte d'un deuxième don de plaquettes par aphérèse à partir d'un deuxième donneur,
c) la combinaison du premier don de plaquettes et du deuxième don de plaquettes afin de produire une préparation de plaquettes mélangées, et
d) la séparation de la préparation de plaquettes mélangées en une pluralité d'unités de plaquettes, chacune dans un récipient individuel, où la pluralité d'unités de plaquettes comprend un plus grand nombre d'unités de plaquettes que le nombre total d'unités de plaquettes qui peut être préparé à partir du premier don de plaquettes et du deuxième don de plaquettes sans combiner les premier et deuxième dons de plaquettes ; où chaque unité de plaquettes comprend une dose thérapeutique de plaquettes.

2. Procédé selon la revendication 1, dans lequel la collecte du premier don de plaquettes à partir du premier donneur comprend le ciblage de la collecte de plaquettes pour produire un premier don de plaquettes comprenant au moins environ 125 % du nombre de plaquettes requis pour préparer une unité de plaquettes, mais un moins grand nombre de plaquettes que celui requis pour préparer deux unités de plaquettes ; et/ou le premier don de plaquettes comprend un plus grand nombre de plaquettes que celui requis pour préparer une unité de plaquettes, mais un moins grand nombre de plaquettes que celui requis pour préparer deux unités de plaquettes.

3. Procédé selon la revendication 1, dans lequel la collecte du premier don de plaquettes à partir du premier donneur comprend le ciblage de la collecte de plaquettes pour produire un premier don de plaquettes comprenant au moins environ 225 % du nombre de plaquettes requis pour préparer une unité de plaquettes, mais un moins grand nombre de plaquettes que celui requis pour préparer trois unités de plaquettes ; et/ou le premier don de plaquettes comprend un plus grand nombre de plaquettes que celui requis pour préparer deux unités de plaquettes, mais un moins grand nombre de plaquettes que celui requis pour préparer trois unités de plaquettes.

4. Procédé selon la revendication 1, dans lequel la collecte du premier don de plaquettes à partir du premier donneur comprend le ciblage de la collecte de plaquettes pour produire un premier don de plaquettes comprenant au moins environ 325 % du nombre de plaquettes requis pour préparer une unité de plaquettes, mais un moins grand nombre de plaquettes que celui requis pour préparer quatre unités de plaquettes ; et/ou le premier don de plaquettes comprend un plus grand nombre de plaquettes que celui requis pour préparer trois unités de plaquettes, mais un moins grand nombre de plaquettes que celui requis pour préparer quatre unités de plaquettes.

5. Procédé selon l'une quelconque des revendications précédentes, dans lequel la collecte du deuxième don de plaquettes à partir du deuxième donneur comprend le ciblage de la collecte de plaquettes pour produire un deuxième don de plaquettes comprenant au moins environ 125 % du nombre de plaquettes requis pour préparer une unité de plaquettes, mais un moins grand nombre de plaquettes que celui requis pour préparer deux unités de plaquettes ; et/ou le deuxième don de plaquettes comprend un plus grand nombre de plaquettes que celui requis pour préparer une unité de plaquettes, mais un moins grand nombre de plaquettes que celui requis pour préparer deux unités de plaquettes.

6. Procédé selon l'une quelconque des revendications 1 à 4, dans lequel la collecte du deuxième don de plaquettes à partir du deuxième donneur comprend le ciblage de la collecte de plaquettes pour produire un deuxième don de plaquettes comprenant au moins environ 225 % du nombre de plaquettes requis pour préparer une unité de plaquettes, mais un moins grand nombre de plaquettes que celui requis pour préparer trois unités de plaquettes ; et/ou le deuxième don de plaquettes comprend un plus grand nombre de plaquettes que celui requis pour préparer deux unités de plaquettes, mais un moins grand nombre de plaquettes que celui requis pour préparer trois unités de plaquettes.

7. Procédé selon l'une quelconque des revendications 1 à 4, dans lequel la collecte du deuxième don de plaquettes à partir du deuxième donneur comprend le ciblage de la collecte de plaquettes pour produire un deuxième don de plaquettes comprenant au moins environ 325 % du nombre de plaquettes requis pour préparer une unité de plaquettes, mais un moins grand nombre de plaquettes que celui requis pour préparer quatre unités de plaquettes.

8. Procédé selon la revendication 2, dans lequel la collecte du deuxième don de plaquettes à partir du deuxième donneur comprend le ciblage de la collecte de plaquettes pour produire un deuxième don de plaquettes comprenant un nombre de plaquettes suffisant pour préparer trois unités de plaquettes après la combinaison des premier et deuxième dons de plaquettes et la séparation de la préparation de plaquettes mélangées résultante en une pluralité d'unités de plaquettes ; et/ou le deuxième don de plaquettes comprend un nombre de plaquettes suffisant qui, une fois combiné avec le premier don de plaquettes, produit une préparation de plaquettes mélangées comprenant un nombre de plaquettes suffisant pour être séparé en trois unités de plaquettes.

9. Procédé selon la revendication 3, dans lequel la collecte du deuxième don de plaquettes à partir du deuxième donneur comprend le ciblage de la collecte de plaquettes pour produire un deuxième don de plaquettes comprenant un nombre de plaquettes suffisant pour préparer cinq unités de plaquettes après la combinaison des premier et deuxième dons de plaquettes et la séparation de la préparation de plaquettes mélangées résultante en une pluralité d'unités de plaquettes ; et/ou le deuxième don de plaquettes comprend un nombre de plaquettes suffisant qui, une fois combiné avec le premier don de plaquettes, produit une préparation de plaquettes mélangées comprenant un nombre de plaquettes suffisant pour être séparé en cinq unités de plaquettes.

10. Procédé selon la revendication 4, dans lequel le deuxième don de plaquettes comprend un nombre de plaquettes suffisant qui, une fois combiné avec le premier don de plaquettes, produit une préparation de plaquettes mélangées comprenant un nombre de plaquettes suffisant pour être séparé en sept unités de plaquettes.

11. Procédé selon la revendication 5, dans lequel le premier don de plaquettes comprend un plus grand nombre de plaquettes que celui requis pour préparer une unité de plaquettes, mais un moins grand nombre de plaquettes que celui requis pour préparer deux unités de plaquettes, et le deuxième don de plaquettes comprend un plus grand nombre de plaquettes que celui requis pour préparer une unité de plaquettes, mais un moins grand nombre de plaquettes que celui requis pour préparer deux unités de plaquettes, facultativement dans lequel la préparation de plaquettes mélangées comprend un nombre de plaquettes suffisant pour préparer trois unités de plaquettes, et/ou facultativement dans lequel la pluralité d'unités de plaquettes comprend trois unités de plaquettes.

12. Procédé selon la revendication 6, dans lequel le premier don de plaquettes comprend un plus grand nombre de plaquettes que celui requis pour préparer deux unités de plaquettes, mais un moins grand nombre de plaquettes que celui requis pour préparer trois unités de plaquettes, et le deuxième don de plaquettes comprend un plus grand nombre de plaquettes que celui requis pour préparer deux unités de plaquettes, mais un moins grand nombre de plaquettes que celui requis pour préparer trois unités de plaquettes, facultativement dans lequel la préparation de plaquettes mélangées comprend un nombre de plaquettes suffisant pour préparer cinq unités de plaquettes.

13. Procédé selon l'une quelconque des revendications précédentes, comprenant en outre la collecte d'un troisième don de plaquettes par aphérèse à partir d'un troisième donneur, la combinaison du troisième don de plaquettes avec les premier et deuxième dons de plaquettes afin de produire une préparation de plaquettes mélangées, et la séparation de la préparation de plaquettes mélangées en une pluralité d'unités de plaquettes, chacune dans un récipient individuel, dans lequel la pluralité d'unités de plaquettes comprend un plus grand nombre d'unités de plaquettes que le nombre total d'unités de plaquettes qui peut être préparé à partir du premier don de plaquettes, du deuxième don de plaquettes et du troisième don de plaquettes sans combiner les premier, deuxième et troisième dons de plaquettes, et/ou dans lequel le deuxième don de plaquettes est collecté dans les 20 heures suivant le moment où le premier don de plaquettes est collecté, facultativement dans lequel le deuxième don de plaquettes est collecté dans les 12 heures suivant le moment où le premier don de plaquettes est collecté.

14. Procédé selon l'une quelconque des revendications précédentes, comprenant en outre
(a) le fait de soumettre chacun du premier don de plaquettes et du deuxième don de plaquettes à un processus d'inactivation des agents pathogènes ; ou
(b) le fait de soumettre la préparation de plaquettes mélangées à un processus d'inactivation des agents pathogènes ; et
facultativement dans lequel le fait de soumettre les dons de plaquettes ou la préparation de plaquettes mélangées à un processus d'inactivation des agents pathogènes comprend le traitement du don de plaquettes ou de la préparation de plaquettes mélangées avec un composé inactivant les agents pathogènes ; facultativement dans lequel le composé inactivant les agents pathogènes est un composé photoactif inactivant les agents pathogènes sélectionné dans le groupe consistant en un psoralène, une isoalloxazine, une alloxazine, une phtalocyanine, une phénothiazine, une porphyrine, et la mérocyanine 540 ; et facultativement dans lequel le composé photoactif inactivant les agents pathogènes est l'amotosalène.

15. Procédé selon l'une quelconque des revendications précédentes, dans lequel les unités de plaquettes comprennent chacune au moins environ 2,0 × 10¹¹ plaquettes.

16. Procédé selon l'une quelconque des revendications 2 à 9, dans lequel le ciblage de la collecte de plaquettes comprend un ou plusieurs paramètres parmi le fait de déterminer une collecte de plaquettes optimale, fournir une entrée à un système d'aphérèse, et fournir un volume de don à un système d'aphérèse.

17. Procédé de préparation d'une pluralité d'unités de plaquettes, chaque unité comprenant une dose thérapeutique de plaquettes appropriée pour une perfusion chez un sujet humain, ledit procédé comprenant :
a) le maintien d'un journal d'inventaire d'un inventaire de préparations de plaquettes collectées par aphérèse, le journal d'inventaire comprenant, pour chaque préparation de plaquettes, un identifiant unique, un groupe sanguin de donneur, une indication temporelle de don et une quantité de plaquettes dans la préparation,
b) l'identification d'un donneur disponible approprié pour donner des plaquettes par aphérèse,
c) la sélection, à partir du journal d'inventaire, d'au moins une préparation de plaquettes de l'inventaire issue d'un premier donneur afin de la combiner avec des plaquettes collectées chez le donneur disponible, où le premier donneur est différent du donneur disponible,
d) la collecte d'un nouveau don de plaquettes par aphérèse à partir du donneur disponible,
e) la combinaison de la au moins une préparation de plaquettes sélectionnée dans l'inventaire de préparations de plaquettes collectées par aphérèse et du nouveau don de plaquettes afin de produire une préparation de plaquettes mélangées, et
f) la séparation de la préparation de plaquettes mélangées en une pluralité d'unités de plaquettes, chacune dans un récipient individuel, où la pluralité d'unités de plaquettes comprend un plus grand nombre d'unités de plaquettes que le nombre total d'unités de plaquettes qui peut être préparé à partir de la au moins une préparation de plaquettes sélectionnée dans l'inventaire de préparations de plaquettes collectées par aphérèse et du nouveau don de plaquettes du donneur disponible sans combiner la au moins une préparation de plaquettes sélectionnée et le nouveau don de plaquettes, facultativement dans lequel
(g) l'inventaire de préparations de plaquettes collectées par aphérèse comprend des préparations de plaquettes existantes qui ont déjà été collectées ; et/ou
(h) la au moins une préparation de plaquettes sélectionnée comprend un plus grand nombre de plaquettes que celui requis pour préparer une unité de plaquettes, mais un moins grand nombre de plaquettes que celui requis pour préparer deux unités de plaquettes ; et/ou
(i) la au moins une préparation de plaquettes sélectionnée comprend au moins environ 125 % du nombre de plaquettes requis pour préparer une unité de plaquettes, mais un moins grand nombre de plaquettes que celui requis pour préparer deux unités de plaquettes.

18. Procédé selon l'une quelconque des revendications 17(a) à 17(g), dans lequel
(a) la au moins une préparation de plaquettes sélectionnée comprend un plus grand nombre de plaquettes que celui requis pour préparer deux unités de plaquettes, mais un moins grand nombre de plaquettes que celui requis pour préparer trois unités de plaquettes ; ou
(b) la au moins une préparation de plaquettes sélectionnée comprend un plus grand nombre de plaquettes que celui requis pour préparer trois unités de plaquettes, mais un moins grand nombre de plaquettes que celui requis pour préparer quatre unités de plaquettes.

19. Procédé selon l'une quelconque des revendications 17(a) - 17(g), et 18, dans lequel :
(a) la au moins une préparation de plaquettes sélectionnée comprend au moins environ 225 % du nombre de plaquettes requis pour préparer une unité de plaquettes, mais un moins grand nombre de plaquettes que celui requis pour préparer trois unités de plaquettes ; ou
(b) la au moins une préparation de plaquettes sélectionnée comprend au moins environ 325 % du nombre de plaquettes requis pour préparer une unité de plaquettes, mais un moins grand nombre de plaquettes que celui requis pour préparer quatre unités de plaquettes.

20. Procédé selon l'une quelconque des revendications 17 à 19, dans lequel :
(a) la collecte du nouveau don de plaquettes comprend le ciblage de la collecte de plaquettes pour produire un don comprenant au moins environ 125 % du nombre de plaquettes requis pour préparer une unité de plaquettes, mais un moins grand nombre de plaquettes que celui requis pour préparer deux unités de plaquettes ;
(b) le nouveau don de plaquettes comprend un plus grand nombre de plaquettes que celui requis pour préparer une unité de plaquettes, mais un moins grand nombre de plaquettes que celui requis pour préparer deux unités de plaquettes ;
(c) la collecte du nouveau don de plaquettes comprend le ciblage de la collecte de plaquettes pour produire un don comprenant au moins environ 225 % du nombre de plaquettes requis pour préparer une unité de plaquettes, mais un moins grand nombre de plaquettes que celui requis pour préparer trois unités de plaquettes ;
(d) le nouveau don de plaquettes comprend un plus grand nombre de plaquettes que celui requis pour préparer deux unités de plaquettes, mais un moins grand nombre de plaquettes que celui requis pour préparer trois unités de plaquettes ; ou
(e) le nouveau don de plaquettes comprend un plus grand nombre de plaquettes que celui requis pour préparer trois unités de plaquettes, mais un moins grand nombre de plaquettes que celui requis pour préparer quatre unités de plaquettes.

21. Procédé selon la revendication 17(h) ou 17(i), dans lequel la collecte du nouveau don de plaquettes à partir du donneur disponible comprend la collecte d'un nombre de plaquettes suffisant pour préparer trois unités de plaquettes à partir de la préparation mélangée.

22. Procédé selon la revendication 18 ou la revendication 19, dans lequel :
(a) le procédé selon la revendication 18(a) ou 19(a) s'applique, et la collecte du nouveau don de plaquettes à partir du donneur disponible comprend la collecte d'un nombre de plaquettes suffisant pour préparer cinq unités de plaquettes à partir de la préparation mélangée ; ou
(b) le procédé selon la revendication 18(b) ou 19(b) s'applique, et la collecte du nouveau don de plaquettes à partir du donneur disponible comprend la collecte d'un nombre de plaquettes suffisant pour préparer sept unités de plaquettes à partir de la préparation mélangée.

23. Procédé selon la revendication 20, dans lequel le procédé selon la revendication 20(a) ou la revendication 20(b) s'applique, et la au moins une préparation de plaquettes sélectionnée comprend un plus grand nombre de plaquettes que celui requis pour préparer une unité de plaquettes, mais un moins grand nombre de plaquettes que celui requis pour préparer deux unités de plaquettes, et le nouveau don de plaquettes comprend un plus grand nombre de plaquettes que celui requis pour préparer une unité de plaquettes, mais un moins grand nombre de plaquettes que celui requis pour préparer deux unités de plaquettes, facultativement dans lequel la pluralité d'unités de plaquettes comprend trois unités de plaquettes.

24. Procédé selon l'une quelconque des revendications 17 à 23, comprenant en outre l'identification d'un deuxième donneur disponible pour donner des plaquettes par aphérèse, la collecte d'un deuxième nouveau don de plaquettes par aphérèse à partir du deuxième donneur disponible, la combinaison du deuxième nouveau don de plaquettes avec la au moins une préparation de plaquettes sélectionnée dans l'inventaire de préparations de plaquettes collectées par aphérèse et le nouveau don de plaquettes issu du donneur disponible afin de produire la préparation de plaquettes mélangées, et la séparation de la préparation de plaquettes mélangées en une pluralité d'unités de plaquettes, chacune dans un récipient individuel, où la pluralité d'unités de plaquettes comprend un plus grand nombre d'unités de plaquettes que le nombre total d'unités de plaquettes qui peut être préparé à partir de la au moins une préparation de plaquettes sélectionnée, du nouveau don de plaquettes et du deuxième nouveau don de plaquettes sans combiner la au moins une préparation de plaquettes sélectionnée, le nouveau don de plaquettes et le deuxième nouveau don de plaquettes ; et/ou dans lequel le nouveau don de plaquettes est collecté dans les 12 heures suivant le moment où la au moins une préparation de plaquettes sélectionnée est collectée.

25. Procédé selon l'une quelconque des revendications 17 à 24, comprenant en outre :
(a) le fait de soumettre chacun de la au moins une préparation de plaquettes sélectionnée et du nouveau don de plaquettes à un processus d'inactivation des agents pathogènes, facultativement dans lequel le fait de soumettre chacun de la au moins une préparation de plaquettes sélectionnée et du nouveau don de plaquettes à un processus d'inactivation des agents pathogènes comprend le traitement de chacun de la au moins une préparation de plaquettes sélectionnée et du nouveau don de plaquettes avec un composé inactivant les agents pathogènes ; ou
(b) le fait de soumettre la préparation de plaquettes mélangées à un processus d'inactivation des agents pathogènes, facultativement dans lequel le fait de soumettre la préparation de plaquettes mélangées à un processus d'inactivation des agents pathogènes comprend le traitement de la préparation de plaquettes mélangées avec un composé inactivant les agents pathogènes, facultativement dans lequel le composé inactivant les agents pathogènes est un composé photoactif inactivant les agents pathogènes sélectionné dans le groupe consistant en un psoralène, une isoalloxazine, une alloxazine, une phtalocyanine, une phénothiazine, une porphyrine, et la mérocyanine 540 ; et, facultativement, dans lequel le composé photoactif inactivant les agents pathogènes est l'amotosalène.

26. Procédé selon l'une quelconque des revendications 17 à 25, dans lequel les unités de plaquettes dans la pluralité d'unités de plaquettes comprennent chacune une dose thérapeutique de plaquettes appropriée pour une perfusion chez un sujet humain ; et facultativement dans lequel les unités de plaquettes comprennent chacune au moins environ 2,0 × 10¹¹ plaquettes.

27. Procédé selon la revendication 20(a) ou la revendication 20(c), dans lequel le ciblage de la collecte de plaquettes comprend un ou plusieurs paramètres parmi le fait de déterminer une collecte de plaquettes optimale, fournir une entrée à un système d'aphérèse, et fournir un volume de don à un système d'aphérèse.

28. Procédé selon la revendication 17, comprenant en outre :
a) la détermination d'un volume cible du nouveau don de plaquettes issu du donneur disponible, dans lequel le volume cible optimise le nombre d'unités de plaquettes qui peut être préparé à partir d'une combinaison comprenant le nouveau don de plaquettes et la au moins une préparation de plaquettes sélectionnée dans l'inventaire de préparations de plaquettes collectées par aphérèse ; et
b) la collecte du volume cible du nouveau don de plaquettes par aphérèse à partir du donneur disponible ;
c) la combinaison de la au moins une préparation de plaquettes sélectionnée dans l'inventaire de préparations de plaquettes collectées par aphérèse et du nouveau don de plaquettes issu du donneur disponible afin de produire une préparation de plaquettes mélangées ; et
d) la séparation de la préparation de plaquettes mélangées en une pluralité d'unités de plaquettes, chacune dans un récipient individuel ; dans lequel chacune des unités de plaquettes dans la pluralité d'unités de plaquettes comprend une dose thérapeutique appropriée pour une perfusion chez un sujet humain ; et dans lequel la pluralité d'unités de plaquettes comprend un plus grand nombre d'unités de plaquettes que le nombre total d'unités de plaquettes qui peut être préparé à partir de la au moins une préparation de plaquettes sélectionnée dans l'inventaire de préparations de plaquettes collectées par aphérèse et du nouveau don de plaquettes issu du donneur disponible sans combiner la une ou plusieurs préparations de plaquettes sélectionnées et le nouveau don de plaquettes.

29. Procédé selon la revendication 28, dans lequel :
(a) la une ou plusieurs préparations de plaquettes sélectionnées comprend/comprennent un plus grand nombre de plaquettes que celui requis pour préparer une unité de plaquettes, mais un moins grand nombre de plaquettes que celui requis pour préparer deux unités de plaquettes, facultativement dans lequel la collecte du nouveau don de plaquettes à partir du donneur disponible comprend la collecte d'un nombre de plaquettes suffisant pour préparer trois unités de plaquettes à partir de la préparation mélangée ;
(b) la une ou plusieurs préparations de plaquettes sélectionnées comprend/comprennent au moins environ 125 % du nombre de plaquettes requis pour préparer une unité de plaquettes, mais un moins grand nombre de plaquettes que celui requis pour préparer deux unités de plaquettes ; facultativement dans lequel la collecte du nouveau don de plaquettes à partir du donneur disponible comprend la collecte d'un nombre de plaquettes suffisant pour préparer trois unités de plaquettes à partir de la préparation mélangée ; ou
(c) la une ou plusieurs préparations de plaquettes sélectionnées comprend/comprennent un plus grand nombre de plaquettes que celui requis pour préparer deux unités de plaquettes, mais un moins grand nombre de plaquettes que celui requis pour préparer trois unités de plaquettes ; facultativement dans lequel la collecte du nouveau don de plaquettes à partir du donneur disponible comprend la collecte d'un nombre de plaquettes suffisant pour préparer cinq unités de plaquettes à partir de la préparation mélangée.

30. Procédé selon la revendication 28, dans lequel
(a) chacun de la une ou plusieurs préparations de plaquettes sélectionnées et du nouveau don de plaquettes comprend un plus grand nombre de plaquettes que celui requis pour préparer une dose thérapeutique, mais un moins grand nombre de plaquettes que celui requis pour préparer deux doses thérapeutiques ; ou
(b) chacun de la une ou plusieurs préparations de plaquettes sélectionnées et du nouveau don de plaquettes comprend un plus grand nombre de plaquettes que celui requis pour préparer deux doses thérapeutiques d'unités de plaquettes, mais un moins grand nombre de plaquettes que celui requis pour préparer trois doses thérapeutiques d'unités de plaquettes.

31. Procédé selon l'une quelconque des revendications 28 à 30, dans lequel la une ou plusieurs préparations de plaquettes sélectionnées et le nouveau don de plaquettes sont combinés dans les 12 heures suivant le moment de collecte ; et/ou dans lequel chaque unité de plaquettes comprend au moins 2,0 × 10¹¹ plaquettes ; et/ou le procédé comprend en outre :
(a) le fait de soumettre la préparation de plaquettes mélangées à un processus d'inactivation des agents pathogènes ; facultativement dans lequel le fait de soumettre la préparation de plaquettes mélangées à un processus d'inactivation des agents pathogènes comprend le traitement de la préparation de plaquettes mélangées avec un composé inactivant les agents pathogènes ; ou
(b) le fait de soumettre chacun de la une ou plusieurs préparations de plaquettes sélectionnées et du nouveau don de plaquettes à un processus d'inactivation des agents pathogènes ; facultativement dans lequel le fait de soumettre la une ou plusieurs préparations de plaquettes sélectionnées et le nouveau don de plaquettes à un processus d'inactivation des agents pathogènes comprend le traitement de la une ou plusieurs préparations de plaquettes sélectionnées et du nouveau don de plaquettes avec un composé inactivant les agents pathogènes ; facultativement dans lequel le composé inactivant les agents pathogènes est un composé photoactif inactivant les agents pathogènes sélectionné dans le groupe consistant en un psoralène, une isoalloxazine, une alloxazine, une phtalocyanine, une phénothiazine, une porphyrine, et la mérocyanine 540 ; et facultativement dans lequel le composé photoactif inactivant les agents pathogènes est l'amotosalène.
